# DEMANDE DE BREVET EUROPEEN

(11) **EP 2 335 702 A1**
(43) Date de publication de la demande: **22.06.2011**
(21) Numéro de dépôt: 08020152.8
(22) Date de dépôt: 24.06.2004
(51) Int. Cl.: A61K 31/425, A61K 31/42, A61P 35/00, C07D 277/66, C07D 277/64, C07D 417/04, C07D 263/56, C07D 417/12, C07D 413/12, C07D 413/04, C07D 519/00

(54) **Produit comprenant au moins un inhibiteur de phosphatase Cdc25 en association avec au moins un autre agent anti-cancéreux**

(30) Priorité: 25.06.2003 FR 0307649
(62) Demande divisionnaire de: 04767442.9
(71) Demandeur: IPSEN PHARMA, 92100 Boulogne-Billancourt (FR)
(72) Inventeur: Prevost, Grégoire, 92160 Antony (FR); Brezak Pannetier, Marie-Christine, 92160 Antony (FR)
(74) Mandataire: Audonnet, Nathalie

(57) **Abrégé**

L'invention a pour objet un produit comprenant au moins un inhibiteur de phosphatase Cdc25 en association avec au moins un autre agent anti-cancéreux pour une utilisation thérapeutique simultané, séparée ou étalée dans le temps dans le traitement du cancer et où l'agent anti-cancéreux associé à l'inhibiteur de phosphatase Cdc25 est un inhibiteur des kinases dépendantes des cyclines (CDKs).

## Description

La présente invention a pour objet un produit comprenant au moins un inhibiteur de phosphatase Cdc25 en association avec au moins un autre agent anti-cancéreux pour une utilisation thérapeutique simultanée, séparée ou étalée dans le temps dans le traitement du cancer.

Le contrôle de la transition entre les différentes phases du cycle cellulaire durant la mitose ou de 1a méiose est assurée par un ensemble de protéines dont les activités enzymatiques sont associées à des états différents de phosphorylation. Ces états sont contrôlés par deux grandes classes d'enzymes : les kinases et les phosphatases.

La synchronisation des différentes phases du cycle cellulaire permet ainsi la réorganisation de l'architecture cellulaire à chaque cycle dans l'ensemble du monde vivant (microorganismes, levure, vertébrés, plantes). Parmi les kinases, les kinases dépendantes des cyclines (CDKs) jouent un rôle majeur dans ce contrôle du cycle cellulaire. L'activité enzymatique de ces différentes CDKs est contrôlée par deux autres familles d'enzymes qui travaillent en opposition (Jessus et Ozon, Prog. Cell Cycle Res. (1995), 1, 215-228). La première regroupe des kinases telles que Weel et Mikl qui désactivent les CDKs en phosphorylant certains acides aminés (Den Haese et coll., Mol. Biol. Cell (1995), 6, 371-385). La seconde regroupe des phosphatases telle que Cdc25 qui activent les CDKs en déphosphorylant des résidus tyrosine et thréonine de CDKs (Gould et coll., Science (1990), 250, 1573-1576).

Les phosphatases sont classifiées en 3 groupes : les sérines/thréonines phosphatases (PPases), les tyrosines phosphatases (PTPases) et les phosphatases à double spécificité (DSPases). Ces phosphatases jouent un rôle important dans la régulation de nombreuses fonctions cellulaires.

En ce qui concerne les phosphatases Cdc25 humaines, 3 gènes (Cdc25-A, Cdc25-B et Cdc25-C) codent pour les protéines Cdc25. De plus, des variants issus de splicing alternatif des gènes Cdc25 ont été identifiés (cf. par exemple Baldin et coll., Oncogene (1997), 14 , 2485-2495).

Le rôle des phosphatases Cdc25 dans l'oncogénèse est maintenant mieux connu et les mécanismes d'action de ces phosphatases sont illustrés en particulier dans les références suivantes : Galaktionov et coll., Science (1995), 269, 1575-1577 ; Galaktionov et coll., Nature (1996), 382, 511-517 ; et Mailand et coll., Science (2000), 288, 1425-1429.

En particulier, la surexpression des différentes formes de Cdc25 est maintenant reportée dans de nombreuses séries de tumeurs humaines :
- Cancer du sein : cf. Cangi et coll., *Résumé 2984, AACR meeting San Francisco,* 2000) ;
- Lymphomes : cf. Hernandez et coll., Int. J. Cancer (2000), 89, 148-152 et Hemandez et coll., Cancer Res. (1998), 58, 1762-1767 ;
- Cancers du cou et de la tête : cf. Gasparotto et coll., Cancer Res. (1997), 57, 2366-2368.

Par ailleurs, le groupe de E. Sausville rapporte une corrélation inverse entre le niveau d'expression de Cdc25-B dans un panel de 60 lignées et leurs sensibilités aux inhibiteurs de CDK, suggérant que la présence de Cdc25 puisse apporter une résistance à certains agents antitumoraux et plus particulièrement aux inhibiteurs de CDK (Hose et coll., Proceedings of AACR, Abstract 3571, San Francisco, 2000).

Parmi d'autres cibles, on recherche donc à présent des composés capables d'inhiber les phosphatases Cdc25 afin de les utiliser notamment comme agents anti-cancéreux.

L'invention concerne un produit comprenant au moins un inhibiteur de phosphatase Cdc25 en association avec au moins un autre agent anti-cancéreux pour une utilisation thérapeutique simultanée, séparée ou étalée dans le temps dans le traitement du cancer.

De préférence, l'invention concernera un produit comprenant un inhibiteur de phosphatase Cdc25 en association avec un autre agent anti-cancéreux pour une utilisation thérapeutique simultanée, séparée ou étalée dans le temps dans le traitement du cancer.

Par utilisation thérapeutique simultanée, on entend dans la présente demande une administration de plusieurs principes actifs par la même voie et au même moment. Par utilisation séparée, on entend notamment une administration de plusieurs principes actifs sensiblement au même moment par des voies différentes. Par utilisation thérapeutique étalée dans le temps, on entend une administration de plusieurs principes actifs à des moments différents et notamment un mode d'administration selon lequel l'ensemble de l'administration de l'un des principes actifs est effectué avant que l'administration de l'autre ou des autres ne commence. On peut ainsi administrer l'un des principes actifs pendant plusieurs mois avant d'administrer l'autre ou les autres principes actifs. Il n'y a pas de traitement simultané dans ce cas.

Selon l'invention, l'agent anti-cancéreux associé à l'inhibiteur de phosphatase Cdc25 sera de préférence tel qu'il agisse selon une autre voie que les phosphatases Cdc25. En particulier, ledit agent anti-cancéreux associé aura une concentration inhibitrice CI₅₀ d'au moins 50 µM par rapport aux phosphatases Cdc25 ou alors aura une autre actrivité avec une dose CI₅₀ au moins 10 fois plus faible que celle par rapport aux phosphatases Cdc25. De préférence, l'association réalisée selon l'invention sera telle qu'elle présente une synergie.

Selon l'invention, l'inhibiteur de phosphatase Cdc25 sera de préférence choisi parmi les dérivés de benzothiazole-4,7-diones et de benzooxazole-4,7-diones répondant à 1a formule générale **(I)** définie ci-après.

Un certain nombre de dérivés de benzothiazole-4,7-diones et de benzooxazole-4,7-diones est déjà connu.

En particulier, le brevet GB 1 534 275 concerne des herbicides dont le principe actif est un composé répondant à l'une des formules générales 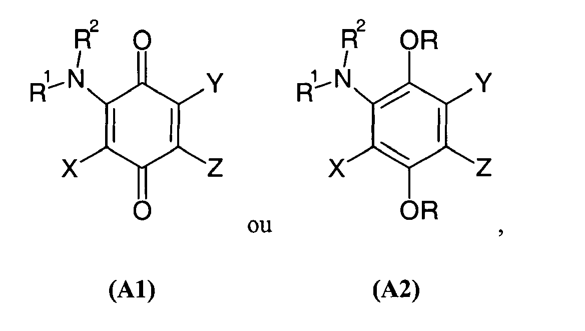 dans lesquelles :
R¹ représente notamment un atome d'hydrogène ou un radical alkyle ou cycloalkyle ;
R² représente notamment un atome d'hydrogène, un radical alkyle ou cycloalkyle ;
X représente notamment un atome halogène ou un radical alkoxy ;
Y et Z peuvent notamment représenter ensemble avec les atomes de carbone qui les portent un cycle thiazole éventuellement substitué par un radical alkyle ; et
R représente notamment un radical alkyle.

Par ailleurs, la demande de brevet PCT WO 99/32115 décrit les composés de formule générale **(A3)** 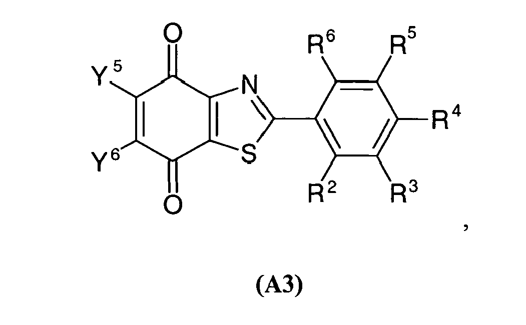 dans laquelle :
les substituants R²-R⁶ sont choisis parmi le groupe constitué d'un atome d'hydrogène,
de substituants donneurs d'électrons, de substituants attracteurs d'électrons et de substituants modulateurs d'électrons ;
et Y⁵ et Y⁵ sont notamment choisis parmi le groupe constitué d'un atome d'hydrogène, de substituants donneurs d'électrons, de substituants attracteurs d'électrons et de substituants modulateurs d'électrons.

Dans la demande de brevet PCT WO 99/32115, le terme « substituant donneur d'électrons » fait référence à un groupe fonctionnel ayant tendance à donner de la densité électronique ; sont cités les substituants alkyle, alkényle et alkynyle. Toujours dans cette demande de brevet, « substituant attracteur d'électrons » fait référence à un groupe fonctionnel ayant tendance à attraire de la densité électronique ; sont cités les substituants cyano, acyle, carbonyle, fluoro, nitro, sulfonyle et trihalométhyle. Enfin, un « substituant modulateur d'électrons » est défini dans cette demande comme un groupe fonctionnel ayant tendance à moduler la densité électronique, lequel peut à la fois se attirer et donner des électrons et est donc tel qu'il puisse stabiliser un intermédiaire cationique dans une réaction de substitution électrophile aromatique ; est cité un groupe fonctionnel incluant, par exemple, des substituants amino (par exemple -NH₂, alkylamino ou dialkylamino), hydroxy, alkoxy ou aryle, des substituants hétérocycliques, des atomes halogènes, etc.

Les composés de formule générale **(A3)** sont présentés comme des modulateurs des récepteurs de la ryanodine qui peuvent être utilisés en tant que pesticides ou en tant qu'agents thérapeutiques, par exemple dans le traitement de la défaillance cardiaque congestive, des maux de tête migraineux, de l'hypertension, de la maladie de Parkinson ou de la maladie d'Alzheimer ou dans la prévention de l'avortement prématuré.

Enfin, les dérivés de benzooxazole-4,7-diones de formule générale **(A4)** 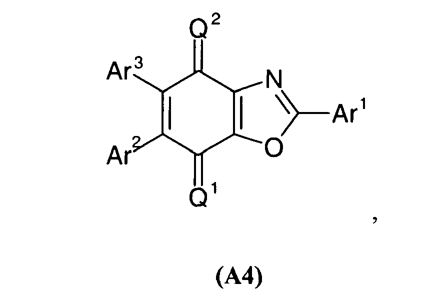 dans laquelle :
Ar¹ représente un radical aryle éventuellement substitué,
chacun de Ar² et Ar³ représente un atome d'hydrogène ou un radical aryle éventuellement substitué, et
chacun de Q¹ et Q² représente notamment O,
sont décrits en tant que constituants actifs de couches photosensibles de photorécepteurs.
Dans la demande de brevet PCT/FR02/04544, la demanderesse a décrit les composés répondant à la formule générale **(I)** 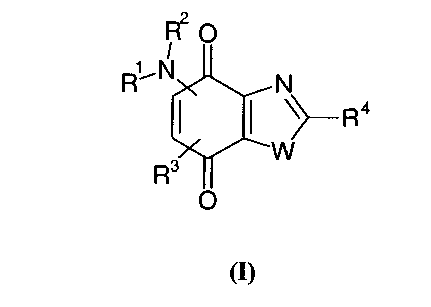
   dans laquelle :
   R¹ représente un atome d'hydrogène ou un radical alkyle, alkoxyalkyle, alkylthioalkyle, cycloalkyle, -(CH₂)-X-Y, -(CH₂)-Z-NR⁵R⁶ ou un radical -CHR³⁵R³⁶ dans lequel R³⁵ et
   R³⁶ forment ensemble avec l'atome de carbone qui les porte un radical indanyle ou tétralinyle, ou encore R³⁵ et R³⁶ forment ensemble avec l'atome de carbone qui les porte un hétérocycle saturé comptant de 5 à 7 chaînons et de 1 à 2 hétéroatomes choisis parmi O, N et S, les atomes d'azote dudit hétérocycle étant éventuellement substitués par des radicaux choisis parmi les radicaux alkyle et le radical benzyle,
   R¹ pouvant aussi, lorsque W représente O, représenter en outre un radical aryle carbocyclique éventuellement substitué de 1 à 3 fois par des substituants choisis indépendamment parmi un atome halogène et un radical alkyle, haloalkyle ou alkoxy, X représentant une liaison ou un radical alkylène linéaire ou ramifié comptant de 1 à 5 atomes de carbone,
   Y représentant un système cyclique carboné saturé comptant de 1 à 3 cycles condensés choisis indépendamment parmi des cycles de 3 à 7 chaînons, ou bien Y représentant un hétérocycle saturé comptant de 1 à 2 hétéroatomes choisis indépendamment parmi O, N et S et attaché au radical X par un chaînon N ou CH, ledit hétérocycle saturé comptant par ailleurs de 2 à 6 chaînons supplémentaires choisis indépendamment parmi -CHR⁷-, -CO-, -NR⁸-, -O- et -S-, R⁷ représentant un atome d'hydrogène ou un radical alkyle et R⁸ représentant un atome d'hydrogène ou un radical alkyle ou aralkyle, ou encore Y représentant un radical aryle carbocyclique ou hétérocyclique éventuellement substitué de 1 à 3 fois par des substituants choisis indépendamment parmi le groupe constitué par un atome halogène, un radical alkyle, un radical haloalkyle, un radical alkoxy, un radical haloalkoxy, un radical hydroxy, un radical nitro, un radical cyano, le radical phényle, un radical SO₂NHR⁹ et un radical NR¹⁰R¹¹, R⁹ représentant un atome d'hydrogène ou un radical alkyle ou phényle, et R¹⁰ et R¹¹ représentant indépendamment des radicaux alkyle,
   Z représentant une liaison ou un radical alkylène linéaire ou ramifié comptant de 1 à 5 atomes de carbone,
   R⁵ et R⁶ étant choisis indépendamment parmi un atome d'hydrogène, un radical alkyle, aralkyle ou -(CH₂)ₙ-OH dans lequel n représente un entier de 1 à 6,
      ou R⁵ représentant un radical alkoxycarbonyle, haloalkoxycarbonyle ou aralkoxycarbonyle et R⁶ représentant un atome d'hydrogène ou un radical méthyle,
      ou encore R⁵ et R⁶ formant ensemble avec l'atome d'azote un hétérocycle de 4 à 7 chaînons comportant de 1 à 2 hétéroatomes, les chaînons nécessaires pour compléter l'hétérocycle étant choisis indépendamment parmi les radicaux -CR¹²R¹³-, -O-, -S- et -NR¹⁴-, R¹² et R¹³ représentant indépendamment à chaque fois qu'ils interviennent un atome d'hydrogène ou un radical alkyle, et R¹⁴ représentant un atome d'hydrogène ou un radical alkyle ou aralkyle, ou encore R¹⁴ représentant un radical phényle éventuellement substitué de 1 à 3 fois par des substituants choisis indépendamment parmi un atome halogène et un radical alkyle ou alkoxy,
   R² représentant un atome d'hydrogène ou un radical alkyle ou aralkyle ;
      ou encore R¹ et R² formant ensemble avec l'atome d'azote un hétérocycle de 4 à 8 chaînons comportant de 1 à 2 hétéroatomes, les chaînons nécessaires pour compléter l'hétérocycle étant choisis indépendamment parmi les radicaux -CR¹⁵R¹⁶-, -O-, -S- et -NR¹⁷-, R¹⁵ et R¹⁶ représentant indépendamment à chaque fois qu'ils interviennent un atome d'hydrogène ou un radical alkyle, et R¹⁷ représentant un atome d'hydrogène ou un radical alkyle ou aralkyle ;
   R³ représente un atome d'hydrogène, un atome halogène, ou un radical alkyle, haloalkyle, alkoxy ou alkylthio ;
   R⁴ représente un radical alkyle, cycloalkyle, cycloalkylalkyle, cyano, amino, -CH₂-COOR¹⁸, -CH₂-CO-NR¹⁹R²⁰ ou -CH₂-NR²¹R²², ou bien R⁴ représente un radical aryle carbocyclique ou hétérocyclique éventuellement substitué de 1 à 4 fois par des substituants choisis indépendamment parmi un atome halogène et un radical alkyle, haloalkyle, alkoxy, haloalkoxy ou NR³⁷R³⁸, ou encore R⁴ représente un radical phényle possédant deux substituants qui forment ensemble un radical méthylènedioxy ou éthylènedioxy,
   R¹⁸ représentant un atome d'hydrogène ou un radical alkyle,
   R¹⁹ représentant un atome d'hydrogène, un radical alkyle ou un radical aralkyle dont le groupe aryle est éventuellement substitué de 1 à 3 fois par des substituants choisis indépendamment parmi le groupe constitué par un atome halogène, un radical alkyle, un radical haloalkyle, un radical alkoxy, un radical haloalkoxy, un radical hydroxy, un radical nitro, un radical cyano, le radical phényle, un radical SO₂NHR²³ et un radical NR²⁴R²⁵, R²³ représentant un atome d'hydrogène ou un radical alkyle ou phényle, et R²⁴ et R²⁵ représentant indépendamment des radicaux alkyle,
   R²⁰ représentant un atome d'hydrogène ou un radical alkyle,
      ou encore R¹⁹ et R²⁰ formant ensemble avec l'atome d'azote un hétérocycle de 4 à 7 chaînons comportant de 1 à 2 hétéroatomes, les chaînons nécessaires pour compléter l'hétérocycle étant choisis indépendamment parmi les radicaux -CR²⁶R²⁷-, -O-, -S- et -NR²⁸-, R²⁶ et R²⁷ représentant indépendamment à chaque fois qu'ils interviennent un atome d'hydrogène ou un radical alkyle, et R²⁸ représentant un atome d'hydrogène ou un radical alkyle ou aralkyle, ou encore R²⁸ représentant un radical phényle éventuellement substitué de 1 à 3 fois par des substituants choisis indépendamment parmi un atome halogène et un radical alkyle ou alkoxy,
   R²¹ représentant un atome d'hydrogène, un radical alkyle ou un radical aralkyle dont le groupe aryle est éventuellement substitué de 1 à 3 fois par des substituants choisis indépendamment parmi le groupe constitué par un atome halogène, un radical alkyle, un radical haloalkyle, un radical alkoxy, un radical haloalkoxy, un radical hydroxy, un radical nitro, un radical cyano, le radical phényle, un radical SO₂NHR²⁹ et un radical NR³⁰R³¹, R²⁹ représentant un atome d'hydrogène ou un radical alkyle ou phényle, et R³⁰ et R³¹ représentant indépendamment des radicaux alkyle,
   R²² représentant un atome d'hydrogène ou un radical alkyle, ou encore R²¹ et R²² formant ensemble avec l'atome d'azote un hétérocycle de 4 à 7 chaînons comportant de 1 à 2 hétéroatomes, les chaînons nécessaires pour compléter l'hétérocycle étant choisis indépendamment parmi les radicaux -CR³²R³³-, -O-, -S- et -NR³⁴-, R³² et R³³ représentant indépendamment à chaque fois qu'ils interviennent un atome d'hydrogène ou un radical alkyle, et R³⁴ représentant un atome d'hydrogène, un radical alkyle ou aralkyle, ou encore R³⁴ représentant un radical phényle éventuellement substitué de 1 à 3 fois par des substituants choisis indépendamment parmi un atome halogène et un radical alkyle ou alkoxy,
   R³⁷ et R³⁸ étant choisis indépendamment parmi un atome d'hydrogène et un radical alkyle ou R³⁷ et R³⁸ formant ensemble avec l'atome d'azote un hétérocycle de 4 à 7 chaînons comportant de 1 à 2 hétéroatomes, les chaînons nécessaires pour compléter l'hétérocycle étant choisis indépendamment parmi les radicaux -CR³⁹R⁴⁰-, -O-, -S- et -NR⁴¹-, R³⁹ et R⁴⁰ représentant indépendamment à chaque fois qu'ils interviennent un atome d'hydrogène ou un radical alkyle, et R⁴¹ représentant un atome d'hydrogène ou un radical alkyle ; et
   W représente O ou S ;
      ou les sels pharmaceutiquement acceptables de composés de formule générale **(I)** définie ci-dessus
      en tant qu'inhibiteurs de phosphatases Cdc25, et en particulier des inhibiteurs de la phosphatase Cdc25-C, et/ou de la phosphatase CD 45. Lesdits composés peuvent donc être utilisés pour préparer un médicament destiné à inhiber les phosphatases Cdc25, et en particulier la phosphatase Cdc25-C, et/ou la phosphatase CD 45.

Par alkyle, lorsqu'il n'est pas donné plus de précision, on entend un radical alkyle linéaire ou ramifié comptant de 1 à 12 atomes de carbone, de préférence de 1 à 10 atomes de carbone et plus préférentiellement 1 à 8 atomes de carbone (et notamment de 1 à 6 atomes de carbone). Par alkyle inférieur, lorsqu'il n'est pas donné plus de précision, on entend un radical alkyle linéaire ou ramifié comptant de 1 à 6 atomes de carbone. Par cycloalkyle, lorsqu'il n'est pas donné plus de précision, on entend un radical cycloalkyle comptant de 3 à 7 atomes de carbone. Par aryle carbocyclique ou hétérocyclique, on entend un système carbocyclique ou hétérocyclique de 1 à 3 cycles condensés comprenant au moins un cycle aromatique, un système étant dit hétérocyclique lorsque l'un au moins des cycles qui le composent comporte un hétéroatome (O, N ou S) ; lorsqu'un radical aryle carbocyclique ou hétérocyclique est dit substitué sans qu'il soit donné plus de précision, on entend que ledit radical aryle carbocyclique ou hétérocyclique est substitué de 1 à 3 fois, et de préférence de 1 à 2 fois par des radicaux différents d'un atome d'hydrogène qui, s'ils ne sont pas précisés, sont choisis parmi un atome halogène et les radicaux alkyle ou alkoxy ; par ailleurs, lorsqu'il n'est pas donné plus de précision, on entend par aryle un aryle carbocyclique exclusivement. Par haloalkyle, on entend un radical alkyle dont au moins l'un des atomes d'hydrogène (et éventuellement tous) est remplacé par un atome halogène.

Par radicaux cycloalkylalkyle, alkoxy, haloalkyle, haloalkoxy et aralkyle, on entend respectivement les radicaux cycloalkylalkyle, alkoxy, haloalkyle, haloalkoxy et aralkyle dont les radicaux alkyle, cycloalkyle et aryle ont les significations indiquées précédemment.

Lorsqu'il est indiqué qu'un radical est éventuellement substitué de 1 à 3 fois, il est de préférence éventuellement substitué de 1 à 2 fois et plus préférentiellement éventuellement substitué une fois.

Par alkyle linéaire ou ramifié ayant de 1 à 6 atomes de carbone, on entend en particulier les radicaux méthyle, éthyle, propyle, isopropyle, butyle, isobutyle, sec-butyle et tert-butyle, pentyle, néopentyle, isopentyle, hexyle, isohexyle. Par haloalkyle, on entend notamment le radical trifluorométhyle. Par haloalkoxy, on entend notamment le radical trifluorométhoxy. Par aryle carbocyclique, on entend en particulier les radicaux phényle et naphtyle. Par aralkyle, on entend en particulier les radicaux phénylalkyle, et notamment le radical benzyle. Par système cyclique carboné saturé comptant de 1 à 3 cycles condensés choisis indépendamment parmi des cycles de 3 à 7 chaînons, on entend en particulier les radicaux cyclopropyle, cyclobutyle, cyclohexyle et adamantyle. Par aryle hétérocyclique ou hétéroaryle, on entend en particulier les radicaux thiényle, furannyle, pyrrolyle, imidazolyle, thiazolyle, oxazolyle et pyridyle. Enfin, par halogène, on entend les atomes de fluor, de chlore, de brome ou d'iode.

Par sel pharmaceutiquement acceptable, on entend notamment des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt selection for basic drugs", Int. J. Pharm. (1986), 33, 201-217.

Dans certains cas, les composés de formule générale **(I)** peuvent comporter des atomes de carbone asymétriques. Par conséquent, les composés selon la présente invention ont deux formes énantiomères possibles, c'est-à-dire les configurations "R" et "S". La présente invention inclut les deux formes énantiomères et toutes combinaisons de ces formes, y compris les mélanges racémiques "RS". Dans un souci de simplicité, lorsqu'aucune configuration spécifique n'est indiquée dans les formules de structure, il faut comprendre que les deux formes énantiomères et leurs mélanges sont représentés.

Les produits selon la présente invention comprenant un composé de formule générale **(I)** présentent aussi de façon générale quatre variantes :
- selon une première variante, les composés de formule générale **(I)** qui répondent
   aussi à la sous-formule générale **(I)₁** 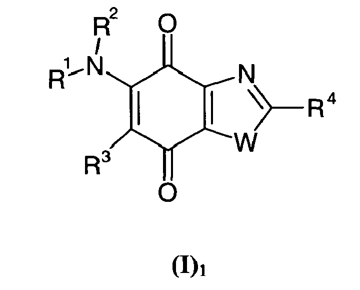 dans laquelle W représente S et R¹, R², R³ et R⁴ ont la même signification que dans la formule générale **(I)**, ou leurs sels pharmaceutiquement acceptables, seront les composés de formule générale **(I)** inclus dans le produit de l'invention;
- selon une deuxième variante, les composés de formule générale **(I)** qui répondent
   aussi à 1a sous-formule générale **(I)₂** 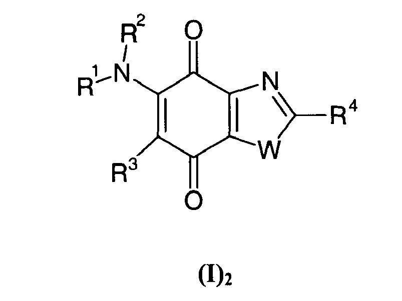 dans laquelle W représente O et R¹, R², R³ et R⁴ ont la même signification que dans la formule générale **(I)**, ou leurs sels pharmaceutiquement acceptables, seront les composés de formule générale **(I)** inclus dans le produit de l'invention ;
- selon une troisième variante, les composés de formule générale **(I)** qui répondent aussi à la sous-formule générale **(I)**₃ 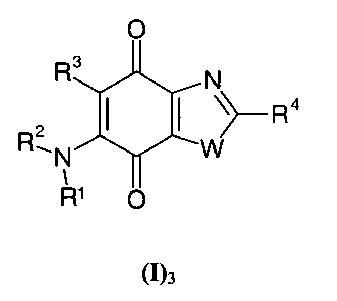 dans laquelle W représente S et R¹, R², R³ et R⁴ ont la même signification que dans la formule générale **(I)**, ou leurs sels pharmaceutiquement acceptables, seront les composés de formule générale **(I)** inclus dans le produit de l'invention ; et
- selon une quatrième variante, les composés de formule générale **(I)** qui répondent
   aussi à la sous-formule générale **(I)₄** 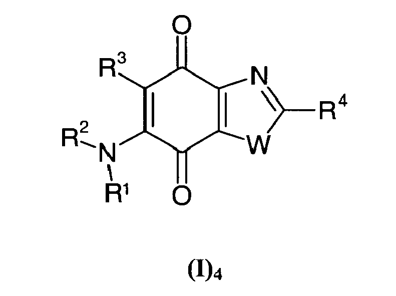 dans laquelle W représente O et R¹, R², R³ et R⁴ ont la même signification que dans la formule générale **(I)**, ou leurs sels pharmaceutiquement acceptables, seront les composés de formule générale **(I)** inclus dans le produit de l'invention.

L'invention concerne donc notamment les produits précédemment mentionnés comprenant au moins un composé choisi parmi les composés de formule générale **(I)₁** ou **(I)₂**, ou de leurs sels pharmaceutiquement acceptables. De même, l'invention concerne les produits précédemment mentionnés comprenant au moins un composé choisi parmi les composés de formule générale **(I)₃** ou **(I)₄,** ou leurs sels pharmaceutiquement acceptables.

De préférence, les composés de formule générale **(I), (I)₁, (I)₂, (I)₃** ou **(I)₄** inclus dans un produit selon l'invention posséderont au moins l'une des caractéristiques suivantes :
- R¹ représentant un radical alkyle, cycloalkyle, alkoxyalkyle, -(CH₂)-X-Y, -(CH₂)-Z-NR⁵R⁶ ou-CHR³⁵R³⁶;
- R² représentant un atome d'hydrogène ou le radical méthyle, éthyle ou benzyle ;
- R¹ et R² formant ensemble avec l'atome d'azote un hétérocycle de 4 à 8 chaînons (de préférence de 5 à 7 chaînons, et notamment de 6 chaînons) comportant de 1 à 2 hétéroatomes (et de préférence 2 hétéroatomes), les chaînons nécessaires pour compléter l'hétérocycle étant choisis indépendamment parmi les radicaux -CH₂-, -O- et -NR¹⁷ (et de préférence parmi les radicaux -CH₂- et -NR¹⁷-), R¹⁷ représentant un radical méthyle ou benzyle ;
- R³ représentant un atome d'hydrogène, un atome halogène ou un radical alkyle, alkoxy ou alkylthio ;
- R⁴ représentant un radical alkyle, -CH₂-COOR¹⁸ ou -CH₂-CO-NR¹⁹R²⁰ ou -CH₂-NR²¹R²² ou encore un radical aryle carbocyclique ou hétérocyclique éventuellement substitué de 1 à 4 fois (et notamment de 1 à 3 fois) par des substituants choisis indépendamment parmi un atome halogène et un radical alkyle, haloalkyle, alkoxy ou NR³⁷R³⁸.

D'une façon générale, on préférera, pour un produit selon l'invention, les composés de formule générale **(I)** dans lesquels W représente un atome de soufre. Une autre alternative intéressante pour un produit selon l'invention consistera néanmoins à y inclure des composés de formule générale **(I)** dans lesquels W représente un atome d'oxygène.

Par ailleurs, le radical X représentera de préférence une liaison ou un radical alkylène linéaire comptant de 1 à 5 atomes de carbone. De préférence aussi, le radical Y représentera un système cyclique carboné saturé comptant de 1 à 3 cycles condensés choisis indépendamment parmi des cycles de 3 à 7 chaînons, ou Y représentera un radical aryle carbocyclique éventuellement substitué (de préférence éventuellement substitué par 1 à 3 radicaux choisis parmi un atome halogène et un radical alkyle, haloalkyle, alkoxy, haloalkoxy, SO₂NHR⁹ ou NR¹⁰R¹¹, et plus préférentiellement éventuellement substitué par 1 à 3 radicaux choisis parmi un atome halogène et un radical alkyle, alkoxy, SO₂NHR⁹ ou NR¹⁰R¹¹) ou encore Y représentera un radical aryle hétérocyclique éventuellement substitué, ledit radical aryle hétérocyclique étant de préférence choisi parmi les radicaux aryle à 5 chaînons (et notamment parmi les radicaux imidazolyle, thiényle ou pyridinyle) et de préférence éventuellement substitué par 1 à 3 radicaux choisis parmi un atome halogène et un radical alkyle, haloalkyle, alkoxy, haloalkoxy, SO₂NHR⁹ ou NR¹⁰R¹¹, et plus préférentiellement éventuellement substitué par 1 à 3 radicaux choisis parmi un atome halogène et un radical alkyle, alkoxy, SO₂NHR⁹ ou NR¹⁰R¹¹; R⁹ représentera de préférence un atome d'hydrogène et R¹⁰ et R¹¹ représenteront de préférence des radicaux choisis indépendamment parmi les radicaux alkyle. Le radical Z représentera de préférence un radical alkylène comptant de 1 à 5 atomes de carbone, et en particulier un radical -(CH₂)ₚ- dans lequel p représente un entier de 1 à 3 (p étant de préférence égal à 1 ou 2 et plus préférentiellement égal à 1). De préférence également, R⁵ et R⁶ seront choisis indépendamment parmi un atome d'hydrogène et un radical alkyle, ou encore R⁵ et R⁶ formeront ensemble avec l'atome d'azote qui les porte un hétérocycle de 4 à 7 chaînons comportant de 1 à 2 hétéroatomes, ledit hétérocycle étant alors de préférence l'un des radicaux azétidinyle, pyrrolidinyle, pipéridinyle, pipérazinyle, homopipérazinyle, morpholinyle et thiomorpholinyle éventuellement substitués par 1 à 3 radicaux alkyle (et de préférence par 1 à 3 radicaux méthyle) ; de façon encore plus préférentielle, R⁵ et R⁶ seront choisis indépendamment parmi des radicaux alkyle ou alkoxycarbonyle (et en particulier R⁵ et R⁶ seront chacun un radical méthyle ou *tert*-butoxycarbonyle) ou R⁵ et R⁶ formeront ensemble avec l'atome d'azote qui les porte un hétérocycle de 4 à 7 chaînons comportant de 1 à 2 hétéroatomes, ledit hétérocycle étant alors de préférence l'un des radicaux azétidinyle, pyrrolidinyle, pipéridinyle, pipérazinyle, homopipérazinyle, morpholinyle et thiomorpholinyle éventuellement substitués par 1 à 3 radicaux alkyle (et de préférence par 1 à 3 radicaux méthyle). R¹⁸ représentera de préférence un atome d'hydrogène ou le radical méthyle ou éthyle.

De plus, les radicaux R⁷, R¹², R¹³, R¹⁵, R¹⁶, R²⁶, R²⁷, R³⁹ et R⁴⁰ seront de préférence choisis indépendamment parmi un atome d'hydrogène et un radical méthyle et les radicaux R⁸, R¹⁴, R¹⁷, R²⁸ et R⁴¹ seront de préférence choisis indépendamment parmi un atome d'hydrogène et un radical méthyle ou benzyle.

En outre, en ce qui concerne R¹⁹ et R²⁰, on préférera les cas dans lesquels R¹⁹ représente un atome d'hydrogène, un radical alkyle ou un radical benzyle et R²⁰ représente un atome d'hydrogène ou le radical méthyle, ainsi que ceux dans lesquels R¹⁹ et R²⁰ forment ensemble avec l'atome d'azote qui les porte un hétérocycle de 4 à 7 chaînons comportant de 1 à 2 hétéroatomes, ledit hétérocycle étant alors de préférence l'un des radicaux azétidinyle, pyrrolidinyle, pipéridinyle, pipérazinyle, homopipérazinyle, morpholinyle et thiomorpholinyle éventuellement substitués par 1 à 3 radicaux alkyle (et de préférence éventuellement substitués par 1 à 3 radicaux méthyle).

Par ailleurs, en ce qui concerne R²¹ et R²², on préférera les cas dans lesquels R²¹ représente un atome d'hydrogène, un radical alkyle ou un radical benzyle et R²² représente un atome d'hydrogène ou le radical méthyle, ainsi que ceux dans lesquels R²¹ et R²² forment ensemble avec l'atome d'azote qui les porte un hétérocycle de 4 à 7 chaînons comportant de 1 à 2 hétéroatomes, ledit hétérocycle étant alors de préférence l'un des radicaux azétidinyle, pyrrolidinyle, pipéridinyle, pipérazinyle, homopipérazinyle, morpholinyle et thiomorpholinyle éventuellement substitués. En ce qui concerne les radicaux R³², R³³ et R³⁴ correspondants, ceux-ci seront de préférence tels que R³² et R³³ soient choisis indépendamment parmi un atome d'hydrogène et un radical alkyle et de préférence parmi un atome d'hydrogène et un radical méthyle (R³² et R³³ représentant encore plus préférentiellement tous deux des atomes d'hydrogène) et que R³⁴ représente un atome d'hydrogène, un radical alkyle ou un radical phényle éventuellement substitué de 1 à 3 fois par des substituants choisis indépendamment parmi un atome halogène et un radical alkyle ou alkoxy (R³⁴ représentant de façon encore plus préférentielle un atome d'hydrogène ou un radical méthyle ou phényle).

De plus, en ce qui concerne R³⁵ et R³⁶, on préférera les cas dans lesquels R³⁵ et R³⁶ forment ensemble avec l'atome de carbone qui les porte un radical indanyle ou R³⁵ et R³⁶ forment ensemble avec l'atome de carbone qui les porte un hétérocycle saturé comptant de 5 à 7 chaînons et de 1 à 2 hétéroatomes choisis parmi O, N et S, les atomes d'azote dudit hétérocycle étant éventuellement substitués par des radicaux choisis parmi les radicaux alkyle et le radical benzyle.

Par ailleurs, en ce qui concerne R³⁷ et R³⁸, on préférera les cas dans lesquels R³⁷ et R³⁸ représentent indépendamment des radicaux choisis parmi les radicaux alkyle.

Enfin, lorsque R⁴ est un radical aryle carbocyclique ou hétérocyclique éventuellement substitué de 1 à 4 fois, l'on préfère qu'il soit choisi dans le groupe consistant en des radicaux aryle carbocycliques et hétérocycliques éventuellement substitués de 1 à 3 fois par des substituants choisis indépendamment parmi un atome halogène et un radical alkyle, haloalkyle, alkoxy, haloalkoxy ou NR³⁷R³⁸ (et en particulier de 1 à 3 fois par des substituants choisis indépendamment parmi un atome halogène et un radical alkyle, haloalkyle, alkoxy ou haloalkoxy) et le radical 2,3,4,5-tétrafluorophényle. Plus préférentiellement, lorsque R⁴ est un radical aryle carbocyclique ou hétérocyclique éventuellement substitué de 1 à 4 fois, R⁴ sera choisi dans le groupe consistant en des radicaux aryle carbocycliques et hétérocycliques éventuellement substitués de 1 à 2 fois par des substituants choisis indépendamment un atome halogène, un radical alkyle, haloalkyle, alkoxy, haloalkoxy ou NR³⁷R³⁸ (et en particulier 1 à 2 fois par des substituants choisis indépendamment parmi un atome halogène et un radical alkyle, haloalkyl, alkoxy ou haloalkoxy), un radical 3,4,5-trihalophényle et le radical 2,3,4,5-tétrafluorophényle.

Plus préférentiellement, les composés de formule générale **(I), (I)₁**, **(I)₂, (I)₃** ou **(I)₄** inclus dans un produit selon l'invention posséderont au moins l'une des caractéristiques suivantes :
- R¹ représentant un radical alkyle, cycloalkyle, ou -(CH₂)-Z-NR⁵R⁶ ;
- R² représentant un atome d'hydrogène ou le radical méthyle ;
- R³ représentant un atome d'hydrogène, un atome halogène ou le radical méthoxy ;
- R⁴ représentant un radical alkyle, -CH₂-NR²¹R²², ou encore un radical aryle carbocyclique ou hétérocyclique éventuellement substitué de 1 à 4 fois (et notamment de 1 à 3 fois) par des substituants choisis indépendamment parmi un atome halogène et un radical alkyle, ou NR³⁷R³⁸.

Encore plus préférentiellement, les composés de formule générale **(I), (I)₁**, **(I)₂**, **(I)₃** ou **(I)₄** inclus dans un produit selon l'invention posséderont au moins l'une des caractéristiques suivantes :
- R¹ représentant un radical-(CH₂)-Z-NR⁵R⁶ ;
- R² représentant un atome d'hydrogène ;
- R³ représentant un atome d'hydrogène ou un atome halogène (ledit atome halogène étant de préférence un atome de chlore ou de brome) ;
- R⁴ représentant un radical alkyle ou encore un radical phényle, pyridyle, thiényle ou furannyle éventuellement substitué par 1 à 4 (de préférence 1 à 3) atomes halogènes ou par un radical NR³⁷R³⁸.

De façon encore plus particulièrement préférée, les composés de formule générale **(I), (I)₁, (I)₂, (I)₃** ou **(I)₄** inclus dans un produit selon l'invention posséderont au moins l'une des caractéristiques suivantes :
- R³ représentant un atome d'hydrogène ou un atome de chlore (et plus préférentiellement un atome d'hydrogène) ;
- R⁴ représentant un radical alkyle ou encore un radical phényle, pyridyle, thiényle furannyle éventuellement substitué par 1 à 4 (de préférence 1 à 3) atomes halogènes (et en particulier R⁴ représentant un radical alkyle, et de préférence un radical alkyle comptant de 1 à 4 atomes de carbone, et plus préférentiellement encore un radical méthyle ou éthyle).

Selon une variante particulière de l'invention, W représente O. Dans ce cas particulier, l'on préférera que R¹ représente un radical aryle, et en particulier un radical phényle, éventuellement substitué de 1 à 3 fois par des substituants choisis indépendamment parmi un atome halogène et un radical alkyle, haloalkyle ou alkoxy. Plus préférentiellement, toujours lorsque W représente O, l'on préférera que R¹ représente un radical phényle éventuellement substitué par un atome halogène (ledit atome halogène étant de préférence un atome de fluor).

Selon un aspect particulier de l'invention, R⁴ représentera un radical phényle ou un radical aryle hétérocyclique de 5 à 6 chaînons éventuellement substitué de 1 à 4 fois (et de préférence de 1 à 3 fois) par des substituants choisis parmi le groupe consistant en des atomes halogènes, le radical trifluorométhyle et le radical trifluorométhoxy (et de préférence choisis parmi le groupe consistant en des atomes halogènes et le radical trifluorométhyle). En particulier, ledit aryle hétérocyclique de 5 à 6 chaînons éventuellement substitué sera un cycle pyridine, thiophène, furanne ou pyrrole éventuellement substitué.

Un autre aspect particulier de cette invention concerne l'utilisation de composés de formule générale **(I)** dans laquelle W représente S, R³ représente un atome d'hydrogène, le substituant -NR¹R² (les préférences indiquées précédemment pour R¹ et R² restant applicables) est attaché à la position 5 du cycle benzothiazoledione et R⁴ est choisi parmi les radicaux alkyle, cycloalkylalkyl, -CH₂-COOR¹⁸, -CH₂-CO-NR¹⁹R²⁰ et -CH₂-NR²¹R²² (R⁴ étant de préférence alkyle ou cycloalkylalkyle et plus préférentiellement alkyle selon cet aspect particulier de l'invention).

Pour un produit selon l'invention, les composés de formule générale **(I)** décrits (le cas échéant sous forme de sels ou de mélanges) dans les exemples 1 à 138 de composés de formule générale **(I),** ou les sels pharmaceutiquement acceptables de tels composés, seront particulièrement préférés. Parmi les composés des exemples 1 à 138 de composés de formule générale **(I)** et leurs sels pharmaceutiquement acceptables, les composés des exemples 1 à 14, 18 à 39, 48 à 52, 55, 57, 58 et 60 à 138 présenteront d'une manière générale plus d'intérêt pour être inclus dans un produit selon cette invention.

De plus, les composés de formule générale **(I)** décrits (le cas échéant sous forme de sels ou de mélanges) dans les exemples 2 à 5, 16, 19 à 26, 32, 34, 38 à 40, 43 à 47, 55 à 58, 60 à 77, 79 à 98 et 101 à 115 de composés de formule générale **(I)**, ou les sels pharmaceutiquement acceptables de tels composés, seront encore plus particulièrement préférés pour être inclus dans un produit selon l'invention.

En outre, les composés de formule générale **(I)** décrits (le cas échéant sous forme de sels ou de mélanges) dans les exemples 2, 19, 20, 23, 24, 34, 57, 60, 62, 63, 67 à 77, 80 à 92, 94, 96 à 98, 103, 104, 106 et 110 à 113 de composés de formule générale **(I),** ou les sels pharmaceutiquement acceptables de tels composés, seront tout particulièrement préférés pour être inclus dans un produit selon l'invention:

De façon particulièrement préférée, les produits selon l'invention comprenant un composé de formule générale **(I)** incluront un composé choisi parmi les composés suivants :
- 5-{[2-(diméthylamino)éthyl]amino}-2-méthyl-1,3-benzothiazole-4,7-dione ;
- 2-méthyl-5-[(2-pyrrolidin-1-yléthyl)amino]-1,3-benzothiazole-4,7-dione ;
- 2-méthyl-5-[(2-pipéridin-1-yléthyl)amino]-1,3-benzothiazole-4,7-dione ;
- 2-(2-chloro-6-fluorophényl)-5-{[2-(diméthylamino)éthyl]amino}-1,3-benzothiazole-4,7-dione;
   et les sels pharmaceutiquement acceptables de ces derniers.

Selon un aspect encore plus préféré de l'invention, les produits selon l'invention comprenant un composé de formule générale **(I)** incluront la 5-{[2-(diméthylamino)éthyl]amino}-2-méthyl-1,3-benzothiazole-4,7-dione ou un de ses sels pharmaceutiquement acceptables.

Alternativement, l'inhibiteur de phosphatase Cdc25 pourra être un composé de formule générale **(II)** 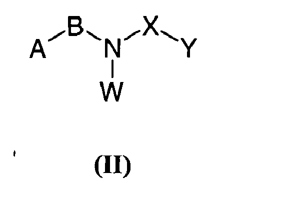 dans laquelle :
A représente un radical **(A1)** 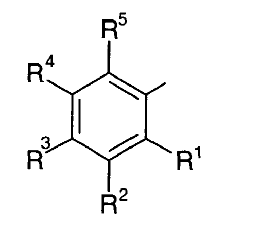
**(A1)**
   dans lequel deux des groupes R¹, R², R³, R⁴ et R⁵ représentent des atomes d'hydrogène et les trois autres sont choisis indépendamment parmi un atome d'hydrogène, un atome halogène et un radical alkyle, hydroxy, alkoxy, alkylcarbonyloxy, alkylthio ou NR⁶R⁷, étant entendu en outre que :
   - ou bien R¹ et l'un de R² et R⁴ sont choisis indépendamment parmi un radical hydroxy, alkylcarbonyloxy et NR⁶R⁷
   - ou bien R² et l'un de R³ et R⁵ sont choisis indépendamment parmi un radical hydroxy, alkylcarbonyloxy et NR⁶R⁷
   - ou bien R⁴ et l'un de R³ et R⁵ sont choisis indépendamment parmi un radical hydroxy, alkylcarbonyloxy et NR⁶R⁷
   - ou encore l'un de R¹, R³ et R⁵ est choisi parmi un radical hydroxy, alkylcarbonyloxy et NR⁶R⁷, et le reste B-N(W)-X-Y est attaché au radical A par un atome d'azote,
      R⁶ et R⁷ représentant, indépendamment à chaque fois qu'ils interviennent, un atome d'hydrogène ou un radical alkyle ou R⁶ et R⁷ formant ensemble avec l'atome d'azote un hétérocycle de 4 à 7 chaînons comportant de 1 à 2 hétéroatomes, les chaînons nécessaires pour compléter l'hétérocycle étant choisis indépendamment parmi les radicaux -CR⁸R⁹-, -O-, -S- et -NR¹⁰-, R⁸ et R⁹ représentant indépendamment à chaque fois qu'ils interviennent un atome d'hydrogène ou un radical alkyle, alkoxy, benzyloxycarbonylamino ou dialkylamino, et R¹⁰ représentant indépendamment à chaque fois qu'il intervient un atome d'hydrogène ou un radical alkyle,
      ou encore A représente un radical **(A2)** 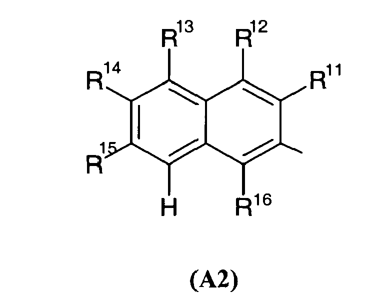 dans lequel :
   - ou bien R¹¹ et l'un de R¹³, R¹⁴ et R¹⁵ représentent des radicaux hydroxy tandis que les autres radicaux parmi R¹³, R¹⁴ et R¹⁵ ainsi que R¹⁶ représentent des atomes d'hydrogène,
   - ou bien R¹² et R¹⁶ représentent des radicaux hydroxy tandis que R¹¹, R¹³, R¹⁴ et R¹⁵ représentent des atomes d'hydrogène ;
      B représente un radical -CO-, -NH-CO-(CH₂)ₙ- ou -(CH₂)ₚ-, n étant un entier de 0 à 3 et p étant un entier de 0 à 1 ;
      W représente un atome d'hydrogène ou un radical alkyle ;
      X représente un radical -(CH₂)_{q}-, -(CH₂)_{q}-NH- ou -CO-(CH₂)ᵣ-, q étant un entier de 1 à 6 et r un entier de 0 à 6 ;
      ou encore l'ensemble B-N(W)-X-Y est tel qu'il représente le radical 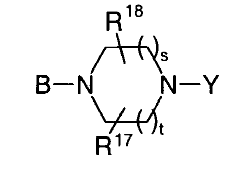 dans lequel B est tel que défini ci-dessus, t est un entier de 0 à 2, s est un entier de 0 à 1 et R¹⁷ et R¹⁸ représentent des radicaux choisis indépendamment parmi un atome d'hydrogène et un radical alkyle;
      et :
   - lorsque X représente un radical -(CH₂)_{q}- ou -CO-(CH₂)ᵣ-, alors Y représente un radical 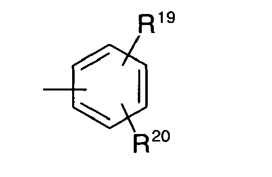 dans lequel R¹⁹ représente un atome d'hydrogène, un atome halogène, un radical nitro, alkyle, alkylthio, NR²¹R²², -SO₂-NR²³R²⁴, -NH-SO₂-R²⁵ ou -O-P(O)(OR²⁶)(OR²⁷), R²¹ et R²² représentant indépendamment un atome d'hydrogène ou un radical alkyle, R²³ et R²⁴ représentant indépendamment un atome d'hydrogène ou un radical alkyle, ou bien R²³ et R²⁴ représentant ensemble avec l'atome d'azote qui les porte un hétérocycle de 5 à 7 chaînons dont les chaînons complémentaires sont choisis indépendamment parmi -CHR²⁸-, -NR²⁹-, -O- et -S-, R²⁸ et R²⁹ représentant, indépendamment à chaque fois qu'ils interviennent, un atome d'hydrogène ou un radical alkyle, R²⁵ représentant un radical alkyle, haloalkyle ou l'un des radicaux aryle, hétéroaryle, aralkyle ou hétéroaralkyle dont le noyau aryle ou hétéroaryle est éventuellement substitué par un ou des radicaux choisis indépendamment parmi un atome halogène et des radicaux alkyle, haloalkyle, hydroxy, alkoxy ou nitro, sauf pour les éventuels atomes d'azote du noyau hétéroaryle pour lesquels les substituants éventuels sont choisis parmi des radicaux alkyle,
      R²⁶ et R²⁷ étant choisis indépendamment parmi des radicaux alkyle,
      et R²⁰ représente un atome d'hydrogène, un atome halogène ou un radical alkyle, alkoxy ou alkylthio,
      ou encore Y représente le radical **(T)** représenté ci-dessous 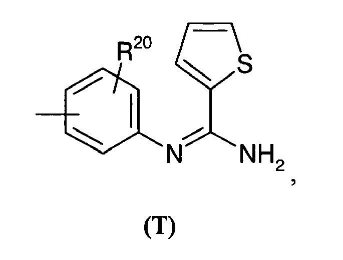 dans lequel R²⁰ représente un atome d'hydrogène ou un radical alkyle, alkoxy ou alkylthio,
   - lorsque X représente un radical -(CH₂)_{q}-NH- ou lorsque l'ensemble B-N(W)-X-Y est tel qu'il représente le radical 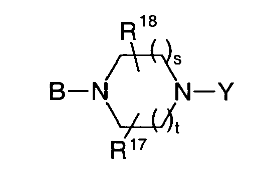 alors Y représente exclusivement un radical -SO₂-R³⁰ dans lequel R³⁰ représente un radical alkyle, haloalkyle ou l'un des radicaux aryle, hétéroaryle, aralkyle ou hétéroaralkyle dont le noyau aryle ou hétéroaryle est éventuellement substitué par un ou des radicaux choisis indépendamment parmi un atome halogène et des radicaux alkyle, haloalkyle, hydroxy, alkoxy ou nitro, sauf pour les éventuels atomes d'azote du noyau hétéroaryle pour lesquels les substituants éventuels sont choisis parmi des radicaux alkyle ;
      étant entendu en outre que lorsque l'ensemble B-N(W)-X-Y est tel qu'il représente le radical 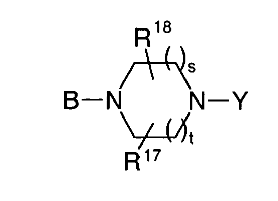 alors B représente exclusivement un radical -CO- ou -(CH₂)- ;
      ou un sel pharmaceutiquement acceptable d'un tel composé.

De préférence, les composés de formule générale **(II)** seront choisis parmi les composés suivants :
- 4-(diméthylamino)-2-méthoxy-6-({méthyl[2-(4-nitrophényl)éthyl]amino}méthyl)-phénol ;
- 4-(diméthylamino)-2-({méthyl[2-(4-nitrophényl)éthyl]amino}méthyl)phénol ;
- 2,7-dihydroxy-N-{2-[4-[(2-thiényl(imino)méthyl)amino]phényl]éthyl}-2-napthalènecarboxamide ;
- 3-[(3- {[amino(2-thiényl)méthylidène]amino} -benzyl)amino]-N-[4-(diméthylamino) phényl]propanamide ;
- 4-(4-aminophényl)-N-[4-(4-méthyl-1-pipérazinyl)phényl]butanamide ;
- 4-(diméthylamino)-2-méthoxy-6-({[2-(4-nitrophényl)éthyl]amino}méthyl)phénol;
- 4-(diméthylamino)-2-({[2-(4-nitrophényl)éthyl]amino}méthyl)phénol;
- 2-(diméthylamino)-6-méthoxy-4-({méthyl[2-(4-nitrophényl)éthyl]amino}méthyl) phénol ;
- 2-({méthyl[2-(4-nitrophényl)éthyl]amino}méthyl)-1,4-benzènediol ;
- acétate de 4-(diméthylamino)-2-méthoxy-6-({méthyl[2-(4-nitrophényl)éthyl]amino} méthyl)phényle ;
- 3,7-dihydroxy-N-[2-(4-nitrophényl)éthyl]-2-naphthamide;
- N-[4-(diméthylamino)benzyl]-3,7-dihydroxy-2-naphthamide;
- 4-{2-[(3,7-dihydroxy-2-naphthoyl)amino]éthyl}phénylphosphate de diéthyle ;
- N-{2-[4-(aminosulfonyl)phényl]éthyl}-3,7-dihydroxy-2-naphthamide ;
- 3,7-dihydroxy-*N*-[2-(4-aminophényl)éthyl]-2-naphthamide ;
- 3,7-dihydroxy-*N*-(2-{4-[(méthylsulfonyl)amino]phényl}éthyl)-2-naphthamide ;
- *N*-(2-{4-[(butylsulfonyl)amino]phényl}éthyl)-3,7-dihydroxy-2-naphthamide ;
- 3,7-dihydroxy-*N*-[2-(4-([(4-méthylphényl)sulfonyl]amino)phényl)éthyl]-2-naphthamide ;
- 3,7-dihydroxy-*N*-(2-{4-[(1-naphthylsulfonyl)amino]phénylléthyl)-2-naphthamide
- 3,7-dihydroxy-*N*-{2-[4-({[2-(trifluorométhyl)phényl]sulfonyl}amino)phényl]éthyl}-2-naphthamide ;
- *N-*(2*-*{4-[(benzylsulfonyl)amino]phényl}éthyl)-3,7-dihydroxy-2-naphthamide;
- 3,7-dihydroxy-*N*-{2-[4-({[3-(trifluorométhyl)phényl]sulfonyl}amino)phényl]éthyl}-2-naphthamide ;
- 3,7-dihydroxy-*N*-[2-(4-{[(4-nitrophényl)sulfonyl]amino1phényl)éthyl]-2-naphthamide ;
- 3,7-dihydroxy-*N*-{2-[4-({[4-(trifluorométhyl)phényl]sulfonyl}amino) phényl]éthyl}-2-naphthamide ;
- 3,7-dihydroxy-*N*-(2-{4-[(thién-2-ylsulfonyl)amino]phényl}éthyl)-2-naphthamide ;
- 3,7-dihydroxy-*N-*[2-(4-{[(4-méthoxyphényl)sulfonyl]amino}phényl)éthyl]-2-naphthamide ;
- 3,7-dihydroxy-*N*-[2-(4-{[(1-méthyl-1H-imidazol-4-yl)sulfonyl]amino}phényl)éthyl]-2-naphthamide ;
- *N*-[2-(4-{[(4-fluorophényl)sulfonyl]amino}phényl)éthyl]-3,7-dihydroxy-2-naphthamide ;
- 3,7-dihydroxy-*N*-{3-[(4-méthyl-1-pipéridinyl)sulfonyl]benzyl}-2-naphthamide ;
- 5-(4-{[(1*E*)-amino(2-thiényl)méthylidène]amino}phényl)-N-[2-(diméthylamino) phényl]pentanamide ;
- 3-({4-[(4-méthylphényl)sulfonyl]pipérazin-1-yl}carbonyl)naphthalène-2,6-diol ;
- 3-{[4-(méthylsulfonyl)pipérazin-1-yl]carbonyl}naphthalène-2,6-diol ;
- 3-{[4-(butylsulfonyl)pipérazin-1-yl]carbonyl}naphthalène-2,6-diol ;
   et les sels pharmaceutiquement acceptables de ces derniers.

L'inhibiteur de phosphatase Cdc25 inclus dans un produit selon l'invention pourra par ailleurs aussi être la ménadione (aussi connue en tant que vitamine K3) ou un de ses analogues comme par exemple la 2-(2-mercaptoéthanol)-3-méthyl-1,4-naphthoquinone (décrite dans Markovits et coll., Life Sci. (2003), 72(24), 2769-84).

L'agent anti-cancéreux associé à l'inhibiteur de phosphatase Cdc25 pourra être choisi parmi des agents anti-cancéreux aussi variés que :
- des analogues de bases de l'ADN comme le 5-fluorouracyle ;
- des inhibiteurs de topoisomérases de type I et/ou II comme par exemple la camptothécine et ses analogues, la doxorubicine ou l'amsacrine ;
- des composés interagissant avec le fuseau cellulaire comme par exemple le paclitaxel (Taxol^{®}) ou le docétaxel (tapotere^{®}) ;
- des composés agissant sur le cytosquelette comme la vinblastine ;
- des inhibiteurs de la transduction du signal passant par les protéines G hétérotrimériques ;
- des inhibiteurs de prényltransférases, et en particulier les inhibiteurs de farnésyltransférases ;
- des inhibiteurs des kinases dépendantes des cyclines (CDKs) ;
- des agents alkylants comme le cisplatine ;
- des antagonistes de l'acide folique comme le méthotrexate ; ou
- des inhibiteurs de la synthèse de l'ADN et de la division cellulaire comme la mitomycine C.

En ce qui concerne les analogues de la camptothécine pouvant être associés à l'inhibiteur de phosphatases Cdc25, ceux-ci pourront être des analogues comportant un cycle lactonique E à six chaînons (tels par exemple les composés décrits dans la demande de brevet PCT WO 94/11376), des analogues comportant un cycle lactonique E à sept chaînons (tels par exemple les homocamptothécines - les composés décrits dans la demande de brevet PCT WO 97/00876) ou des analogues tétracycliques ouverts (tels par exemple les composés décrits dans la demande de brevet PCT WO 99/33829). De préférence, l'analogue de la camptothécine sera choisi parmi le groupe composé du diflomotécan, de la (+)-9-chloro-5-éthyl-5-hydroxy-10-méthyl-12-(4-méthylpipéridinométhyl)-4,5,13,15-tétrahydro-1H,3H-oxépino[3',4':6,7]indolizino[1,2-c]quinoline-3,15-dione et ses sels (en particulier son chlorhydrate aussi connu sous le nom de BN-80927) ainsi que le composé connu sous le nom de code SN-38.

Par homocamptothécine, on entend dans la présente demande tout analogue de la camptothécine dans lequel le motif pentacyclique de la camptothécine naturelle a été modifié par remplacement, dans le cycle E, de l'α-hydroxylactone naturelle de la camptothécine par une β-hydroxylactone.

Selon une variante particulière de l'invention, les analogues de la camptothécine associés à l'inhibiteur de phosphatases Cdc25 seront des analogues comportant un cycle lactonique E à sept chaînons. Ceux-ci seront de préférence des homocamptothécines, et en particulier des homocamptothécines choisies parmi les composés de formule générale **(III)** 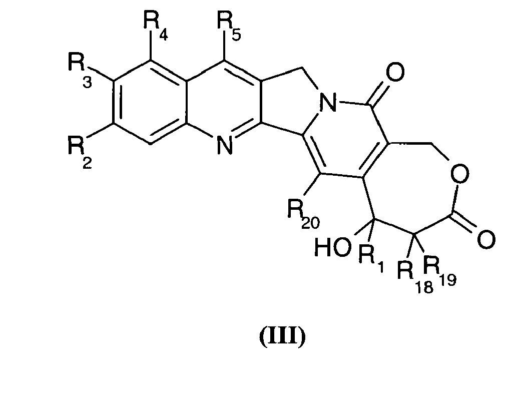 sous forme racémique, d'énantiomère ou toutes combinaisons de ces formes, dans laquelle
- R₁: représente un alkyle inférieur, un alkényle inférieur, un alkynyl inférieur, un haloalkyle inférieur, un alkoxy inférieur alkyle inférieur ou un alkylthio inférieur alkyle inférieur;
- R₂, R₃ et R₄: représentent, indépendamment, i) H, halo, halo alkyle inférieur, alkyle inférieur, alkényle inférieur, cyano, cyano alkyle inférieur, nitro, nitro alkyle inférieur, amido, amido alkyle inférieur, hydrazino, hydrazino alkyle inférieur, azido, azido alkyle inférieur, (CH₂)ₘNR₆R₇, (CH₂)ₘOR₆, (CH₂)ₘSR₆, (CH₂)ₘCO₂R₆, (CH₂)ₘNR₆C(O)R₈, (CH₂)ₘC(O)R₈, (CH₂)ₘOC(O)R₈, O(CH₂)ₘNR₆R₇, OC(O)NR₆R₇, OC(O)(CH₂)ₘCO₂R₆, ou ii) les radicaux substitués (c'est-à-dire, substitué une à quatre fois sur le groupe aryle ou l'hétérocycle) ou non substitués suivants : (CH₂)ₙ[N=X], OC(O)[N=X], (CH₂)ₘOC(O)[N=X] (dans lequel [N=X], dans cette invention, représente un groupe hétérocyclique de 4 à 7 chaînons avec l'atome d'azote N, qui est un membre du groupe hétérocyclique, et X représente les membres restants, nécessaires pour compléter le groupe hétérocyclique, sélectionnés parmi le groupe constitué de O, S, CH₂, CH, N, NR₉ et COR₁₀), aryle ou aryle alkyle inférieur, dans lesquels les substituants éventuels sont choisis parmi le groupe constitué par un alkyle inférieur, halo, nitro, amino, alkylamino inférieur, haloalkyle inférieur, hydroxy alkyle inférieur, alkoxy inférieur, et alkoxy inférieur alkyle inférieur ; ou R₂ et R₃ forment ensemble une chaîne à 3 ou 4 chaînons, dans laquelle les éléments de la chaîne sont sélectionnés parmi le groupe constitué de CH, CH₂, O, S, N ou NR₉;
- R₅: représente i) H, halo, halo alkyle inférieur, alkyle inférieur, alkoxy inférieur, alkoxy inférieur alkyle inférieur, alkylthio inférieur alkyle inférieur, cycloalkyle, cycloalkyle alkyle inférieur, cyano, cyano alkyle, alkyle inférieur sulphonyl alkyle inférieur, hydroxy alkyle inférieur, nitro, (CH₂)ₘC(O)R₈, (CH₂)ₘNR₆C(O)R₈, (CH₂)ₘNR₆R₇, (CH₂)ₘN(CH₃)(CH₂)ₙNR₆R₇, (CH₂)ₘOC(O)R₈, (CH₂)ₘOC(O)NR₆R₇, (CH₂)ₘS(O)_{q}R₁₁, (CH₂)ₘP(O)R₁₂R₁₃, (CH₂)₂P(S)R₁₂R₁₃, ou ii) l'un des radicaux substitués (c'est-à-dire une à quatre fois sur le groupe aryle ou hétéroaryle) ou non substitués suivants : (CH₂)ₙ[N=X], OC(O)[N=X], (CH₂)ₘOC(O)[N=X], aryle ou aryle alkyle inférieur, dans lesquels les substituants éventuels sont choisis parmi le groupe constitué par un alkyle inférieur, halo, nitro, amino, alkyle inférieur amino, halo alkyle
- R₆ et R₇: inférieur, hydroxy alkyle inférieur, alkoxy inférieur et alkoxy inférieur alkyle inférieur; représentent, indépendamment, i) H, un alkyle inférieur, hydroxy alkyle inférieur, alkyle inférieur amino alkyle inférieur, amino alkyle inférieur, cycloalkyle, cycloalkyle alkyle inférieur, alkényle inférieur, alkoxy inférieur alkyle inférieur, halo alkyle inférieur, ou ii) l'un des radicaux substitués (c'est-à-dire, une à quatre fois sur le groupe aryle) ou non substitués suivants : aryle ou aryle alkyle inférieur, dans lesquels les substituants éventuels sont choisis parmi le groupe constitué par un alkyle inférieur, halo, nitro, amino, alkyle inférieur amino, halo alkyle inférieur, hydroxy alkyle inférieur, alkoxy inférieur, et alkoxy inférieur alkyle inférieur;
- Rg: représente i) H, un alkyle inférieur, hydroxy alkyle inférieur, amino, alkyle inférieur amino, alkyle inférieur amino alkyle inférieur, amino alkyle inférieur, cycloalkyle, cycloalkyle alkyle inférieur, alkényle inférieur, alkoxy inférieur, alkoxy inférieur alkyle inférieur, halo alkyle inférieur, ou ii) l'un des radicaux substitués (c'est-à-dire, une à quatre fois sur le groupe aryle) ou non substitués suivants : aryle ou aryle alkyle inférieur, dans lesquels les substituants éventuels sont choisis parmi le groupe constitué par un alkyle inférieur, halo, nitro, amino, alkyle inférieur amino, halo alkyle inférieur, hydroxy alkyle inférieur, alkoxy inférieur, ou alkoxy inférieur alkyle inférieur;
- R₉: représente H, un alkyle inférieur, halo alkyle inférieur, aryle, ou aryle substitué par un ou plusieurs groupes choisis parmi le radical alkyle inférieur, halo, nitro, amino, alkyle inférieur amino, halo alkyle inférieur, hydroxy alkyle inférieur, alkoxy inférieur, ou alkoxy inférieur alkyle inférieur;
- R₁₀: représente H, un alkyle inférieur, halo alkyle inférieur, alkoxy inférieur, aryle, ou aryle substitué (c'est-à-dire, présentant un à quatre substituants sur le groupe aryle) par un ou plusieurs groupes choisis parmi le radical alkyle inférieur, halo alkyle inférieur, hydroxy alkyle inférieur, ou alkoxy inférieur alkyle inférieur;
- R₁₁: représente un alkyle inférieur, aryle, (CH₂)ₘOR₁₄, (CH₂)ₘSR₁₄, (CH₂)₂NR₁₄R₁₅ ou (CH₂)ₘ[N=X];
- R₁₂ et R₁₃: représentent, indépendamment, un alkyle inférieur, aryle, alkoxy inférieur, aryloxy ou amino;
- R₁₄ et R₁₅: représentent, indépendamment, H, un alkyle inférieur ou aryle;
- R₁₈ et R₁₉: représentent, indépendamment, H, halo, alkyl inférieur, alkoxy inférieur ou hydroxy;
- R₂₀: représente H ou halo;
- m: est un nombre entier compris entre 0 et 6;
- n: est 1 ou 2; et
- q: représente un nombre entier de 0 à 2; et [N=X] représente un groupe hétérocyclique de 4 à 7 chaînons, X représentant la chaîne nécessaire pour compléter ledit groupe hétérocyclique et sélectionnée dans le groupe constitué de O, S, CH₂, CH, N, NR₉ et COR₁₀;
et les sels pharmaceutiquement acceptables de ces derniers.

De préférence le composé de formule générale **(III)** est te1 que R₂ représente H ou halo ; R₃ représente H, un alkyle inférieur ou halo ; R₄ représente H ou halo ; R₅ représente H, un alkyle inférieur ou un groupe (CH₂)ₙ[N=X] substitué ou non substitué dans lequel le substituant éventuel est un alkyle inférieur; ou un sel pharmaceutiquement acceptable de ce dernier.

Les composés de formule générale **(III)** ou leurs sels pharmaceutiquement acceptables seront plus particulièrement choisis parmi le diflomotécan et la (+)-9-chloro-5-éthyl-5-hydroxy-10-méthyl-12-(4-méthylpipéridinométhyl)-4,5,13,15-tétrahydro-1H,3H-oxépino[3',4':6,7]indolizino[1,2-c]quinoline-3,15-dione et ses sels pharmaceutiquement acceptables (en particulier son chlorhydrate aussi connu sous le nom de BN-80927).

En ce qui concerne les inhibiteurs de la transduction du signal passant par les protéines G hétérotrimériques pouvant être associés à l'inhibiteur de phosphatases Cdc25, ceux-ci pourront être des composés de formule générale **(IV)** 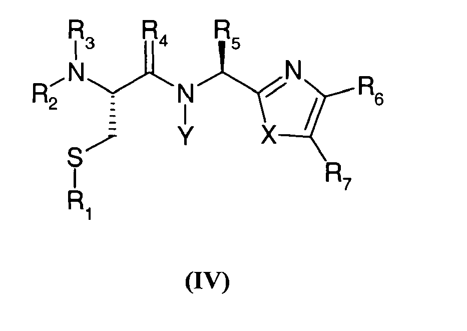 correspondant aux sous-formules **(IV_{A})** ou **(IV_{B}) :** 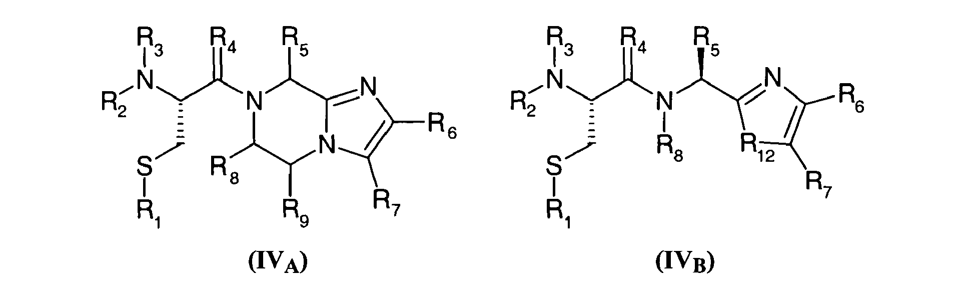 dans lesquelles :
X représente R₁₂ et Y représente R₈, ou X et Y complètent un cycle à 6 chaînons,
l'ensemble X-Y représentant le radical -CH(R₈)-CH(R₉)- ;
R₁ représente H, un radical alkyle, alkylthio ou cycloalkylthio ;
R₂ et R₃ représentent indépendamment H ou un radical alkyle ou cycloalkyle ;
R₄ représente H₂ ou O ;
R₅ représente H, ou l'un des radicaux alkyle, alkényle, alkynyle, cycloalkyle, cycloalkylalkyle, cycloalkényle, cycloalkénylalkyle, aryle, aralkyle, hétérocyclyle ou
hétérocyclylalkyle, ces radicaux pouvant éventuellement être substitués par des radicaux choisis parmi le groupe composé d'un radical alkyle, -O-R₁₀, -S(O)ₘR₁₀ (m représentant 0, 1, ou 2), -N(R₁₀)(R₁₁), -N-C(O)-R₁₀, -NH-(SO₂)-R₁₀, -CO₂-R₁₀, -C(O)-N(R₁₀)(R₁₁), et -(SO₂)-N(R₁₀)(R₁₁) ;
R₆ et R₇ représentent indépendamment H, un radical -C(O)-NH-CHR₁₃-CO₂R₁₄, ou l'un des radicaux alkyle, cycloalkyle, cycloalkylalkyle, cycloalkényle, cycloalkénylalkyle, aryle, aralkyle, hétérocyclyle ou hétérocyclylalkyle, ces radicaux pouvant éventuellement être substitués par des radicaux choisis parmi le groupe composé des radicaux OH, alkyle ou alkoxy, N(_{R1}0)(R₁₁), COOH, CON(R₁₀)(R₁₁), et halo,
   ou R₆ et R₇ forment ensemble un radical aryle ou un hétérocycle ;
R₈ et R₉ représentent indépendamment, H, ou l'un des radicaux alkyle, cycloalkyle, cycloalkylalkyle, cycloalkényle, cycloalkénylalkyle, aryle, aralkyle, hétérocyclyle ou hétérocyclylalkyle, ces radicaux pouvant éventuellement être substitués par des radicaux choisis parmi le groupe composé des radicaux OH, alkyle ou alkoxy, N(R₁₀)(R₁₁), COOH, CON(R₁₀)(R₁₁) et halo,
   ou R₈ et R₉ forment ensemble un radical aryle ou un hétérocycle ;
R₁₀ et R₁₁, représentent indépendamment H, un radical aryle ou hétérocyclyle, ou un radical alkyle, aryle, aralkyle, cycloalkyle, cycloalkylalkyle, hétérocyclyle ou hétérocyclylalkyle ;
R₁₂ représente NR₉, S, ou O ;
R₁₃ représente un radical alkyle éventuellement substitué par un radical choisi parmi les radicaux alkyle, -OR₁₀, -S(O)ₘR₁₀ (m représentant 0, 1, ou 2) et -N(R₁₀)(R₁₁);
R₁₄ représente H ou un radical alkyle ;
   ou des sels pharmaceutiquement acceptables de ces derniers.

Parmi les composés de formule générale **(IV)** et les sels pharmaceutiquement acceptables de tels composés, on préférera en particulier un composé choisi parmi la 7-(2-amino-1-oxo-3-thiopropyl)-8-(cyclohexylméthyl)-2-phényl-5,6,7,8-tétrahydroimidazo[1,2a]pyrazine et sa forme dimère, le bis-1,1'-{7-(2-amino-1-oxo-3-thiopropyl)-8-(cyclohexylméthyl)-2-phényl-5,6,7,8-tétrahydroimidazo[1,2a]pyrazine}disulfure ou la (1*R*)-1-[({(2*R*)-2-amino-3-[(8*S*)-8-(cyclohexylméthyl)-2-phényl-5,6-dihydroimidazo[1,2-a]pyrazin-7(8*H*)-yl]-3-oxopropyl}dithio)méthyl]-2-[(8*S*)-8-(cyclohexylméthyl)-2-phényl-5,6-dihydroimidazo[1,2-a]pyrazin-7(8*H*)-yl]-2-oxoéthylamine, ou un sel pharmaceutiquement acceptable de l'un de ces composés.

En ce qui concerne les inhibiteurs de farnésyltransférases, ceux-ci pourront notamment être choisis parmi le groupe composé :
- d'un composé de formule générale (V) 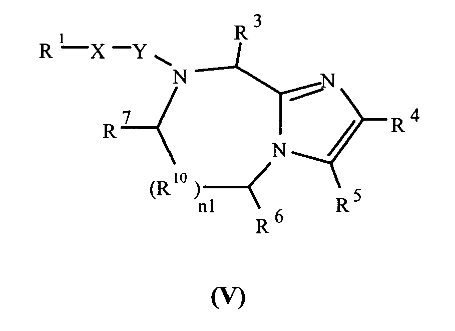 dans laquelle :
   n1 représente 0 ou 1;
   X représente, indépendamment chaque fois qu'il intervient, (CHR¹¹)ₙ₃(CH₂)ₙ₄Z(CH₂)ₙ₅;
   Z représentant O, N(R¹²), S, ou une liaison ;
   n3 représentant, indépendamment chaque fois qu'il intervient, 0 or 1;
   chacun de n4 et n5 représentant, indépendamment chaque fois qu'ils intervient, 0, 1, 2, ou 3 ;
   Y représente, indépendamment chaque fois qu'il intervient, CO, CH₂, CS, ou une liaison ;
   R¹ représente l'un des radicaux 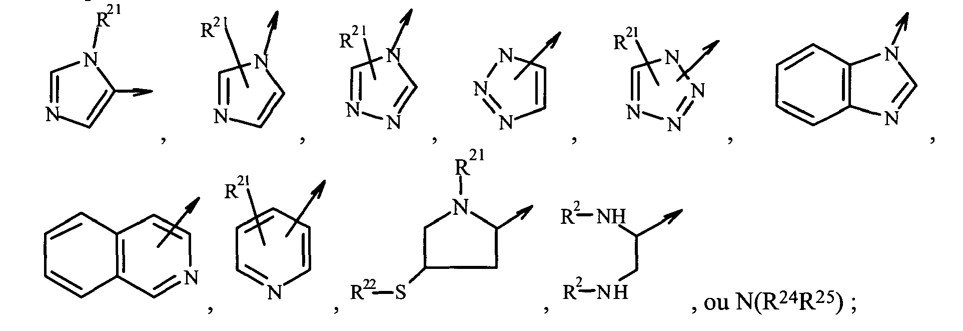 chacun de R², R¹¹, et R¹² représentant, indépendamment chaque fois qu'il intervient, H ou un radical optionnellement substitué choisi parmi le groupe consistant en un radical (C₁₋₆)alkyle et un radical aryle, ledit radical optionnellement substitué étant optionnellement substitué par au moins un radical choisi parmi les radicaux R⁸ et R³⁰, chaque substituant étant choisi indépendamment des autres ;
   R³ représente, indépendamment chaque fois qu'il intervient, H ou un radical optionnellement substitué choisi parmi le groupe consistant en les radicaux (C₁₋₆)alkyle, (C₂₋₆)alkényle, (C₂₋₆)alkynyle, (C₃₋₆)cycloalkyle, (C₃₋₆)cycloalkyl(C₁₋₆)alkyle, (C₅₋₇)cycloalkényle, (C₅₋₇)cycloalkényl(C₁₋₆)akyle, aryle, aryl(C₁₋₆)alkyle, hétérocyclyle, et hétérocyclyl(C₁₋₆)alkyle, ledit radical optionnellement substitué étant optionnellement substitué par au moins un radical choisi parmi les radicaux R³⁰, chaque substituant étant choisi indépendamment des autres ;
      chacun de R⁴ et R⁵ représente, indépendamment chaque fois qu'il intervient, H ou un radical optionnellement substitué choisi parmi le groupe consistant en les radicaux (C₁₋₆)alkyle, (C₃₋₆)cycloalkyle, aryle et hétérocyclyle, ledit radical optionnellement
      substitué étant optionnellement substitué par au moins un radical choisi parmi les radicaux R³⁰, chaque substituant étant choisi indépendamment des autres, ou R⁴ et R⁵ pris ensemble avec les atomes de carbone auxquels ils sont attachés forment ensemble un radical aryle ;
   R⁶ représente, indépendamment chaque fois qu'il intervient, H ou un radical optionnellement substitué choisi parmi le groupe consistant en les radicaux (C₁₋₆)alkyle, (C₂₋₆)alkényle, (C₃₋₆)cycloalkyle, (C₃₋₆)cycloalkyl(C₁₋₆)alkyle, (C₅₋₇)cycloalkényle, (C₅₋₇)cycloalkényl(C₁₋₆)alkyle, aryle, aryl(C₁₋₆)alkyle, hétérocyclyle et hétérocyclyl(C₁₋₆)alkyle, ledit radical optionnellement substitué étant optionnellement substitué par au moins un radical choisi parmi les radicaux OH, (C₁₋₆)alkyle, (C₁₋₆)alkoxy, -N(R⁸R⁹), -COOH, -CON(R⁸R⁹) et halo, chaque substituant étant choisi indépendamment des autres ;
   R⁷ représente, indépendamment chaque fois qu'il intervient, H, =O, =S, H ou un radical optionnellement substitué choisi parmi le groupe consistant en les radicaux (C₁₋₆)alkyle, (C₂₋₆)alkényle, (C₃₋₆)cycloalkyle, (C₃₋₆)cycloalkyl(C₁₋₆)alkyle, (C₅₋₇)cyloalkényle, (C₅₋₇)cycloalkényl(C₁₋₆)alkyle, aryle, aryl(C₁₋₆)alkyle, hétérocyclyle et hétérocyclyl(C₁₋₆)alkyle, ledit radical optionnellement substitué étant optionnellement substitué par au moins un radical choisi parmi les radicaux OH, (C₁₋₆)alkyle, (C₁₋₆)alkoxy, -N(R⁸R⁹), -COOH, -CON(R⁸R⁹) et halo, chaque substituant étant choisi indépendamment des autres ;
      chacun de R⁸ et R⁹ représentant, indépendamment chaque fois qu'il intervient, H, (C₁₋₆)alkyle, (C₂₋₆)alkényle, (C₂₋₆)alkynyle, aryle, or aryl(C₁₋₆)alkyle ;
   R¹⁰ représente C ;
      ou bien, lorsque n1 = 0, R⁶ and R⁷ peuvent être pris ensemble avec les atomes de carbone auxquels ils sont attachés pour former un radical aryle ou cyclohexyle ;
   R²¹ représente, indépendamment chaque fois qu'il intervient, H ou un radical optionnellement substitué choisi parmi le groupe consistant en les radicaux (C₁₋₆)alkyle et aryl(C₁₋₆)alkyle, ledit radical optionnellement substitué étant optionnellement substitué par au moins un radical choisi parmi les radicaux R⁸ et R³⁰ chaque substituant étant choisi indépendamment des autres ;
   R²² représente H, (C₁₋₆)alkylthio, (C₃₋₆)cycloalkylthio, R⁸-CO-, ou un substituant de formule 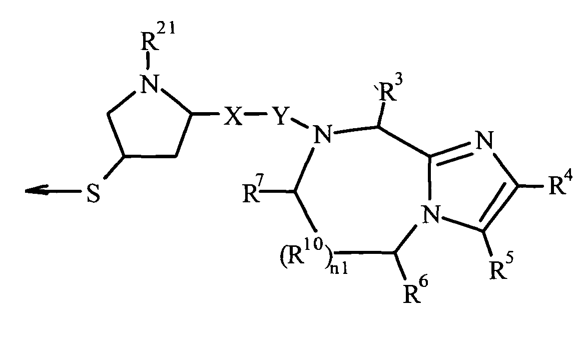 ; chacun de R²⁴ et R²⁵ représente, indépendamment chaque fois qu'il intervient, H, (C₁₋₆)alkyle ou aryl(C₁₋₆)alkyle ;
   R³⁰ représente, indépendamment chaque fois qu'il intervient, (C₁₋₆)alkyle, -O-R⁸, - S(O)ₙ₆R⁸, -S(O)ₙ₇N(R⁸R⁹), -N(R⁸R⁹), -CN, -NO₂, -CO₂R⁸, -CON(R⁸R⁹), -NCO-R⁸, ou halogène,
      chacun de n6 et n7 représentant, indépendamment chaque fois qu'il intervient, 0, 1 ou 2;
      ledit radical hétérocyclyle étant azépinyle, benzimidazolyle, benzisoxazolyle, benzofurazanyle, benzopyranyle, benzothiopyranyle, benzofuryle, benzothiazolyle, benzothiényle, benzoxazolyle, chromanyle, cinnolinyle, dihydrobenzofuryle, dihydrobenzothiényle, dihydrobenzothiopyranyle, dihydrobenzothio-pyranyl sulfone, furyle, imidazolidinyle, imidazolinyle, imidazolyle, indolinyle, indolyle, isochromanyle, isoindolinyle, isoquinolinyle, isothiazolidinyle, isothiazolyle, isothiazolidinyle, morpholinyle, naphthyridinyle, oxadiazolyle, 2-oxoazépinyle, 2-oxopipérazinyle, 2-oxopipéridinyle, 2-oxopyrrolidinyle, pipéridyle, pipérazinyle, pyridyle, pyridyl-N-oxyde, quinoxalinyle, tétrahydrofuryle, tétrahydroisoquinolinyle, tétrahydro-quinolinyle, thiamorpholinyle, thiamorpholinyle sulfoxyde, thiazolyle, thiazolinyle, thiénofuryle, thiénothiényle ou thiényle ;
      ledit radical aryle étant phényle ou naphthyle ;
      étant entendu que : lorsque n1 = 1, R¹⁰ est C et R⁶ représente H, alors R¹⁰ et R⁷ peuvent former, pris
      ensemble, le radical 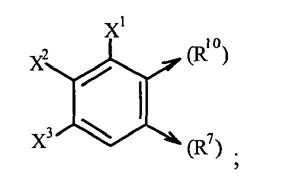 ou lorsque n1 = 1, R¹⁰ est C, et R⁷ est =O, -H, ou =S, alors R¹⁰ et R⁶ peuvent former, pris ensemble, le radical 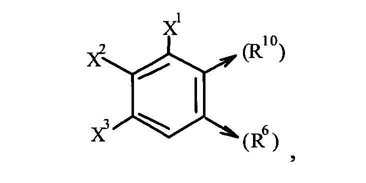 avec chacun de X¹, X², et X³ représentant, indépendamment, H, un atome halogène, - NO₂, -NCO-R⁸, -CO₂R⁸, -CN, ou -CON(R⁸R⁹); et
      lorsque R¹ est N(R²⁴R²⁵), alors n3 représente 1, chacun de n4 et n5 représente 0, Z est une liaison, et R³ et R¹¹ peuvent former, pris ensemble, le radical 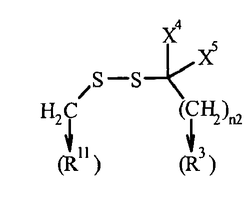 avec n2 représentant un entier de 1 à 6, et chacun de X⁴ et X⁵ représentant, indépendamment, H, (C₁₋₆)alkyle ou aryle, ou X⁴ et X⁵ formant, pris ensemble, un radical (C₃₋₆)cycloalkyle ;
      - d'un composé de formule générale (VI) 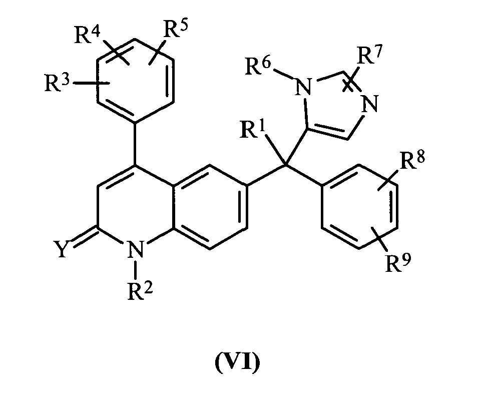 dans laquelle :
         R¹ représente H ou un radical alkyle, OR¹⁰, SR¹⁰ ou NR¹¹R¹²;
         R² représente H ou un radical alkyle ;
         R³, R⁴ et R⁵ représentent, indépendamment, H, un atome halogène ou un radical alkyle, trihalométhyle, hydroxy, cyano ou alkoxy ;
         R⁶ représente H ou un radical alkyle ;
         R⁷ représente H, un atome halogène ou un radical alkyle, hydroxyalkyle, amino, hydroxycarbonyle ;
         R⁸ et R⁹ représentent, indépendamment, H, un atome halogène ou un radical cyano, alkyle, trihalométhyle, alkoxy, alkylthio ou dialkylamino ;
         R¹⁰ représente H ou un radical alkyle ou alkylcarbonyle ;
         R¹¹ représente H ou un radical alkyle ;
         R¹² représente H ou un radical alkyle ou alkylcarbonyle ;
         et Y représente O ou S ;
      - et d'un sel pharmaceutiquement acceptable d'un composé de formule générale (V) ou d'un composé de formule générale **(VI).**

Lorsqu'une structure chimique telle qu'utilisée ici possède une flèche émanant d'elle, la flèche indique le point d'attachement. Par exemple, la structure 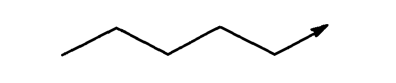 est un radical pentyle. Lorsqu'une valeur entre parenthèses apparaît près de la flèche, la valeur indique où le point d'attachement peut être trouvé dans le composé. Par exemple, dans la formule générale (**V**) 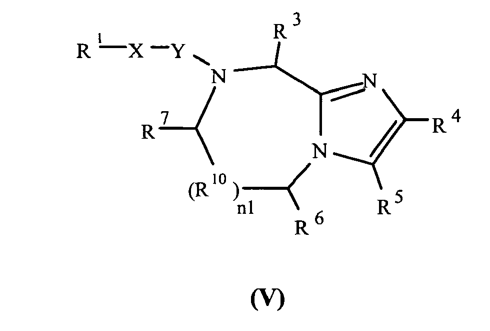 telle que définie précédemment, lorsque R¹⁰ et R⁷ sont pris ensemble pour former le radical 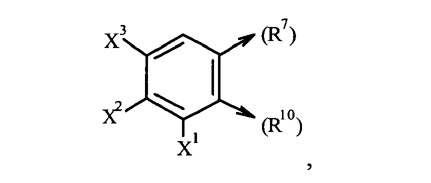 la structure suivante en résulte :

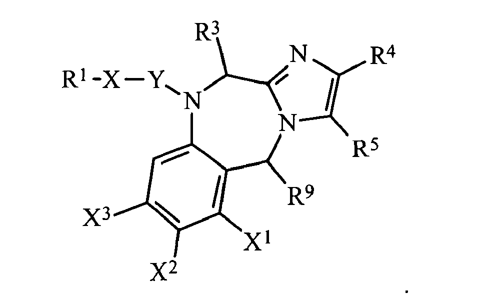

Parmi les composés de formule générale (**V**), on préférera en particulier la 1-(2-(1-((4-cyano)phénylméthyl)imidazol-4-yl)-1-oxoéthyl-2,5-dihydro-4-(2-méthoxyphényl)imidazo[1,2c][1,4]benzodiazépine, la 4-(2-bromophényl)-1-(2-(1-((4-cyano-3-méthoxy)phénylméthyl)imidazo-5-yl)-1-oxoéthyl)-1,2-dihydro-8-fluoroimidazo[1,2a][1,4]-benzodiazépine ou un de ses sels pharmaceutiquement acceptables (et tout particulièrement la 4-(2-bromophényl)-1-(2-(1-((4-cyano-3-méthoxy)phénylméthyl)imidazo-5-yl)-1-oxoéthyl)-1,2-dihydro-8-fluoroimidazo[1,2a][1,4]-benzodiazépine ou un de ses sels pharmaceutiquement acceptables).

En ce qui concerne les inhibiteurs des CDKs, ceux-ci seront de préférence choisis parmi les composés de formule générale **(VII)** 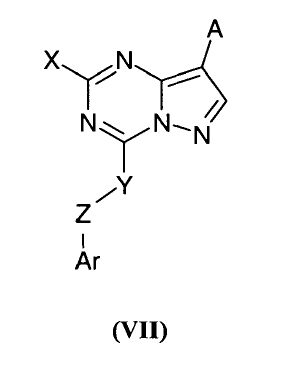 sous forme racémique, d'énantiomère ou toute combinaison de ces formes, dans laquelle A représente un atome d'hydrogène, un atome halogène, un radical formyle, cyano, nitro, guanidinoaminométhylènyle, (1,3-dihydro-2-oxoindol)-3-ylidèneméthyle, alkylcarbonyle, aralkylcarbonyle ou hétéroaralkylcarbonyle, ou encore un radical -L-NR¹R² dans lequel L représente un radical alkylène et R¹ et R² sont choisis indépendamment parmi un atome d'hydrogène et un radical alkyle ou R¹ et R² pris ensemble avec l'atome d'azote qui les porte forment un hétérocycle de 5 à 7 chaînons, les chaînons complémentaires étant choisis indépendamment parmi le groupe composé de -CH₂-, -NR³-, -S- et -O-, R³ représentant indépendamment à chaque fois qu'il intervient un atome d'hydrogène ou un radical alkyle ;
X représente un atome d'hydrogène, un radical alkylthio, aralkylthio, alkylthioxo ou aralkylthioxo, ou encore un radical NR⁴R⁵ dans lequel R⁴ représente un radical alkyle, un radical hydroxyalkyle, un radical cycloalkyle éventuellement substitué par un ou des radicaux choisis parmi les radicaux alkyle, hydroxy et amino, un radical aralkyle dont le radical aryle est éventuellement substitué par un ou des radicaux choisis parmi un atome halogène, le radical cyano, le radical nitro et les radicaux alkyle ou alkoxy, ou encore R⁴ représente un radical hétéroaryle ou hétéroarylalkyle, le radical hétéroaryle des radicaux hétéroaryle ou hétéroarylalkyle étant éventuellement substitué par un ou des radicaux alkyle et R⁵ représente un atome d'hydrogène, ou alors R⁴ et R⁵ pris ensemble avec l'atome d'azote qui les porte forment un hétérocycle de 5 à 7 chaînons, les chaînons complémentaires étant choisis indépendamment parmi le groupe composé de -CH₂-, -NR⁶-, -S- et -O-, R⁶ représentant indépendamment à chaque fois qu'il intervient un atome d'hydrogène ou un radical alkyle ou hydroxyalkyle ;
Y représente NH ou un atome d'oxygène ;
Z représente une liaison ou un radical alkyle ou alkylthioalkyle ; et
Ar représente un radical aryle carbocyclique éventuellement substitué de 1 à 3 fois par des radicaux choisis indépendamment parmi un atome halogène, le radical cyano, le radical nitro, un radical alkyle ou alkoxy et un radical NR⁷R⁸ dans lequel R⁷ et R⁸ représentent indépendamment un atome d'hydrogène ou un radical alkyle ou R⁷ et R⁸ pris ensemble avec l'atome d'azote qui les porte forment un hétérocycle de 5 à 7 chaînons, les chaînons complémentaires étant choisis indépendamment parmi le groupe composé de -CH₂-, -NR⁹-, -S- et -O-, R⁹ représentant indépendamment à chaque fois qu'il intervient un atome d'hydrogène ou un radical alkyle, ou encore Ar représente un radical aryle hétérocyclique comptant 5 ou 6 chaînons et dont le ou les hétéroatomes sont choisis parmi des atomes d'azote, d'oxygène ou de soufre, lesdits hétéroatomes pouvant éventuellement être oxydés (Ar peut représenter par exemple le radical oxidopyridyle) et ledit radical aryle hétérocyclique pouvant être éventuellement substitué par un ou des radicaux choisis indépendamment parmi les radicaux alkyle, aminoalkyle, alkylaminoalkyle et dialkylaminoalkyle ;
ou bien les sels pharmaceutiquement acceptables de ces composés.

Selon l'invention, les composés de formule générale **(VII)** (et de même leurs sels pharmaceutiquement acceptables) seront de préférence tels qu'ils comportent au moins l'une des caractéristiques suivantes :
- A représente un atome halogène, un radical formyle, guanidinoaminométhylènyle, (1,3-dihydro-2-oxoindol)-3-ylidèneméthyle ou alkylcarbonyle, ou encore un radical -L-NR¹R² dans lequel L représente un radical méthylène et R¹ et R² sont choisis indépendamment parmi un atome d'hydrogène et un radical alkyle ou R¹ et R² pris ensemble avec l'atome d'azote qui les porte forment un hétérocycle de 5 à 7 chaînons, les chaînons complémentaires étant choisis indépendamment parmi le groupe composé de -CH₂-, -NR³- et -O-, R³ représentant indépendamment à chaque fois qu'il intervient un atome d'hydrogène ou un radical alkyle ;
- X représente un radical alkylthio (et de préférence méthylthio) ou alkylthioxo (et de préférence méthylthioxo), ou encore un radical NR⁴R⁵ dans lequel R⁴ représente un radical alkyle, un radical hydroxyalkyle, un radical cycloalkyle (et de préférence cyclohexyle) éventuellement substitué par un ou des radicaux amino, ou encore R⁴ représente un radical hétéroaryle ou hétéroarylalkyle, le radical hétéroaryle des radicaux hétéroaryle ou hétéroarylalkyle étant éventuellement substitué par un ou des radicaux alkyle et R⁵ représente un atome d'hydrogène, ou alors R⁴ et R⁵ pris ensemble avec l'atome d'azote qui les porte forment un hétérocycle de 5 à 7 chaînons, les chaînons complémentaires étant choisis indépendamment parmi le groupe composé de -CH₂- et -NR⁶- R⁶ représentant indépendamment à chaque fois qu'il intervient un atome d'hydrogène ou un radical alkyle ou hydroxyalkyle ;
- Y représente NH ;
- Z représente une liaison ou un radical -CH₂- ;
- Ar représente un radical aryle carbocyclique (ledit radical aryle carbocyclique étant de préférence un radical phényle) éventuellement substitué de 1 à 3 fois par des radicaux choisis indépendamment parmi un atome halogène et un radical NR⁷R⁸ dans lequel R⁷ et R⁸ représentent indépendamment un atome d'hydrogène ou un radical alkyle ou R⁷ et R⁸ pris ensemble avec l'atome d'azote qui les porte forment un hétérocycle de 5 à 7 chaînons, les chaînons complémentaires étant choisis indépendamment parmi le groupe composé de -CH₂- et -NR⁹-, R⁹ représentant indépendamment à chaque fois qu'il intervient un radical alkyle, ou encore Ar représente un radical aryle hétérocyclique comptant 5 ou 6 chaînons et dont le ou les hétéroatomes sont choisis parmi des atomes d'azote et d'oxygène (ledit radical aryle hétérocyclique étant de préférence un radical pyridyle), lesdits hétéroatomes pouvant éventuellement être oxydés et ledit radical aryle hétérocyclique pouvant être éventuellement substitué par un ou des radicaux choisis indépendamment parmi les radicaux alkyle, aminoalkyle, alkylaminoalkyle et dialkylaminoalkyle.

Parmi les composés de formule générale (**VII**), l'on préférera en particulier les composés choisis parmi le groupe constitué des composés suivants :
- 8-bromo-4-[(3-pyridyl)méthylamino]-2-méthylthio-pyrazolo[1,5-a]-1,3,5-triazine ;
- 8-bromo-2-(1R-isopropyl-2-hydroxyéthylamino)-4-(3-fluorophénylméthylamino)-pyrazolo[1,5-a]-1,3,5-triazine ;
- 8-bromo-2-(1*R*-isopropyl-2-hydroxyéthylamino)-4-(3-pyridylméthylamino)pyrazolo[1,5-a]-1,3,5-triazine ;
   et de leurs sels pharmaceutiquement acceptables.

Les inhibiteurs des CDKs pourront alternativement être choisis parmi la roscovitine et ses analogues, ou encore parmi l'olomoucine, le purvalanol, le composé connu sous le nom de CVT-313, le flavopiridol, la γ-butyrolactone, les indirubines, les paullones et la staurosporine (cf. Gray et coll., Curr. Med. Chem. (1999), 6(9), 859-75 et références citées).

L'invention a encore pour objet un composé de formule générale (**IV**) particulièrement utile, c'est-à-dire la (1*R*)-1-[({(2*R*)-2-amino-3-[(8*S*)-8-(cyclohexylméthyl)-2-phényl-5,6-dihydroimidazo[1,2-a]pyrazin-7(8*H*)-yl]-3-oxopropyl}dithio)méthyl]-2-[(8*S*)-8-(cyclohexylméthyl)-2-phényl-5,6-dihydroimidazo[1,2-*a*]pyrazin-7(8*H*-yl]-2-oxoéthylamine, ou ses sels pharmaceutiquement acceptables.

Ce composé et ses sels se sont montrés particulièrement stables sous forme de poudres.

Toutefois, l'avantage majeur de ce composé est sa puissante activité anti-cancéreuse (qu'il soit utilisé seul ou en association avec d'autres agents anti-cancéreux) associée à d'excellentes données de toxicité *in vivo.* Par ailleurs, ce composé est aussi un puissant agent anti-douleur, ce qui est aussi une caractéristique souhaitable pour un agent anti-cancéreux.

En outre, un sel particulièrement préféré de ce composé est le tétrachlorhydrate de (1*R*)-1-[({(2*R*)-2-amino-3-[(8*S*)-8-(cyclohexylméthyl)-2-phényl-5,6-dihydroimidazo[1,2-a]pyrazin-7(8*H*)-yl]-3-oxopropyl}dithio)méthyl]-2-[(8*S*)-8-(cyclohexylméthyl)-2-phényl-5,6-dihydroimidazo[1,2-*a*]pyrazin-7(8*H*)-yl]-2-oxoéthylamine.

L'invention offre également un procédé de préparation très pratique et économique pour l'obtention dudit tétrachlorhydrate, ledit procédé comprenant les étapes suivantes:
1) la réaction d'environ 2 équivalents de (8*S*)-8-(cyclohexylméthyl)-2-phényl-5,6,7,8-tétrahydroimidazo[1,2-*a*]pyrazine avec environ un équivalent de Boc-Cys-Cys-Boc dans un solvant polaire aprotique; et
2) la réaction dans un alcool inférieur du dérivé disulfure obtenu après l'étape 1) avec un excès d'acide chlorhydrique en solution dans un alcool inférieur.

Par solvant polaire aprotique on entend dans le procédé mentionné ci-dessus le diméthylformamide ou le tétrahydrofuranne, et de préférence le diméthylformamide.

Par excès d'acide chlorhydrique on entend dans le procédé mentionné ci-dessus au moins 4 équivalents d'acide chlorhydrique (p.ex. de 4 à 5 équivalents d'acide chlorhydrique).

Par alcool inférieur on entend un alcool ayant de 1 à 4 atomes de carbone, notamment le méthanol, l'éthanol ou l'isopropanol. Un alcool inférieur préféré pour l'étape 2) du procédé mentionné ci-dessus est l'isopropanol.

L'étape 1) du procédé mentionné ci-dessus sera de préférence effectuée en présence d'un agent de couplage peptidique (p.ex. l'hexafluorophosphate de O-benzotriazole-*N*,*N*,*N*',*N*'-tétraméthyluronium ou HBTU) et d'une base (p.ex. la diisopropyléthylamine ou la triéthylamine et de préférence la diisopropyléthylamine).

De préférence, l'addition de la solution d'acide chlorhydrique à l'étape 2) sera effectuée à une température n'excédant pas 25 °C (et plus préférentiellement à une température n'excédant pas 5 °C).

Le cas échéant, le milieu réactionnel sera refroidi après l'étape 2) (p.ex. à une température d'environ 0 °C) pour isoler le sel tétrachlorhydrate attendu par cristallisation.

Parmi les cancers destinés à être traités par un produit selon l'invention, on peut notamment citer le cancer du sein, les lymphomes, les cancers du cou et de la tête, le cancer du poumon, le cancer du colon, le cancer de la prostate et le cancer du pancréas.

L'invention a aussi pour objet une méthode de traitement du cancer, ladite méthode comprenant l'administration d'une dose thérapeutiquement efficace d'un produit selon l'invention au patient ayant besoin de ce traitement.

Les compositions pharmaceutiques contenant un produit de l'invention peuvent se présenter sous forme de solides, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'un médicament selon l'invention pourra se faire par voie topique, orale, parentérale, par injection intramusculaire, etc.

La dose d'administration envisagée pour un médicament selon l'invention est comprise entre 0,1 mg à 10 g suivant le type de composé actif utilisé.

Conformément à l'invention, on peut préparer les composés inclus dans des produits selon l'invention par exemple par les procédés décrits ci-après.

### PRÉPARATION DE CERTAINS COMPOSÉS INCLUS DANS DES PRODUITS

### SELON L'INVENTION

### Préparation des composés de formule générale (I)

Les procédés de préparation ci-après sont donnés à titre illustratif et l'homme du métier pourra leur faire subir les variations qu'il juge utiles, aussi bien en ce qui concerne les réactifs que les conditions et techniques des réactions.

### Méthode générale

D'une façon générale, les composés de formule générale **(I)** peuvent être préparés selon la procédure résumée dans le schéma 1 ci-après.

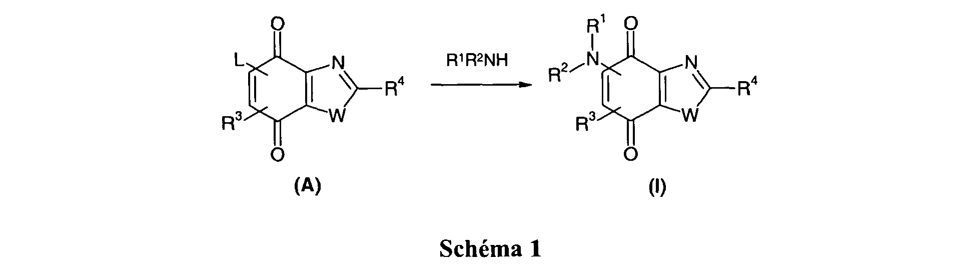

Selon cette méthode, les composés de formule générale **(I),** dans laquelle R¹, R², R³, R⁴ et W sont tels que décrits ci-dessus, sont obtenus par traitement des composés de formule générale **(A),** dans laquelle L représente un radical méthoxy, un atome halogène ou un atome d'hydrogène et R³, R⁴ et W ont la même signification que dans la formule générale **(I),** avec des amines de formule générale NR¹R²H dans un solvant protique tel que le méthanol ou l'éthanol, à une température comprise entre 0 °C et 50 °C et éventuellement en présence d'une base telle que, par exemple, la diisopropyléthylamine (Yasuyuki Kita et coll., J. Org. Chem. (1996), 61, 223-227).

Dans le cas particulier où les composés de formule générale **(A)** sont tels que L et R³ représentent chacun un atome halogène, les composés de formule générale **(I)** peuvent être obtenus sous la forme d'un mélange des 2 isomères de position, mais il est alors possible de les séparer par chromatographie sur colonne de silice dans un éluant approprié.

Alternativement, les composés de formule générale **(I)** dans lesquels R³ représente un atome halogène (Hal) peuvent être obtenus, schéma 1*bis,* à partir des composés de formule générale **(I)** dans lesquels R³ représente un atome d'hydrogène, par exemple par action de N-chlorosuccinimide ou N-bromosuccinimide dans un solvant aprotique tel que le dichlorométhane ou le tétrahydrofuranne (Paquette et Farley, J. Org. Chem. (1967), 32, 2725-2731), par action d'une solution aqueuse d'hypochlorite de sodium (eau de Javel) dans un solvant tel que l'acide acétique (Jagadeesh et coll., Synth Commun. (1998), 28, 3827-3833), par action de Cu(II) (dans un mélange CuCl₂/HgCl₂) en présence d'une quantité catalytique d'iode dans un solvant tel que l'acide acétique à chaud (Thapliyal, Synth. Commun. (1998), 28, 1123-1126), par action d'un agent tel que le dichloroiodate de benzyltriméthylammonium en présence de NaHCO₃ dans un solvant tel qu'un mélange dichlorométhane / méthanol (Kordik et Reitz, J. Org. Chem. (1996), 61, 5644-5645), ou encore par utilisation de chlore, de brome ou d'iode dans un solvant tel que le dichlorométhane (J. Renault, S. Giorgi-Renault et coll., J. Med. Chem. (1983), 26, 1715-1719).

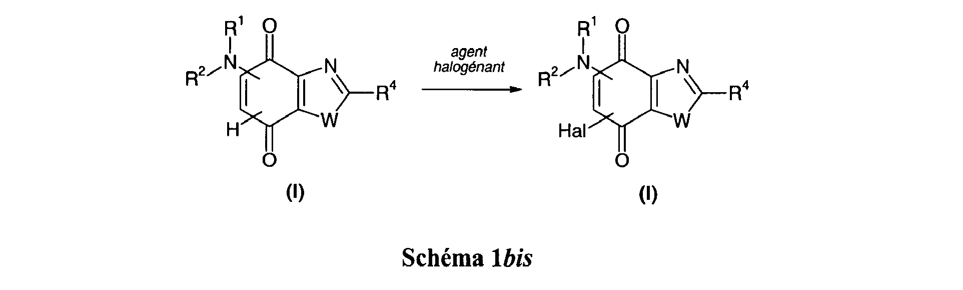

Alternativement également, les composés de formule générale **(I)** dans lesquels R³ représente un radical alkoxy ou alkylthio peuvent être obtenus, schéma 1*ter,* à partir des composés de formule générale **(I)** dans lesquels R³ représente un atome halogène, par exemple par action d'un alcool de formule générale R^{3'}-OH ou d'un thiol de formule générale R^{3'}-SH (R^{3'} étant tel que R³ = R^{3'}O ou R^{3'}S) dans un solvant tel que l'éthanol anhydre en présence d'une base telle que, par exemple, la diisopropyléthylamine.

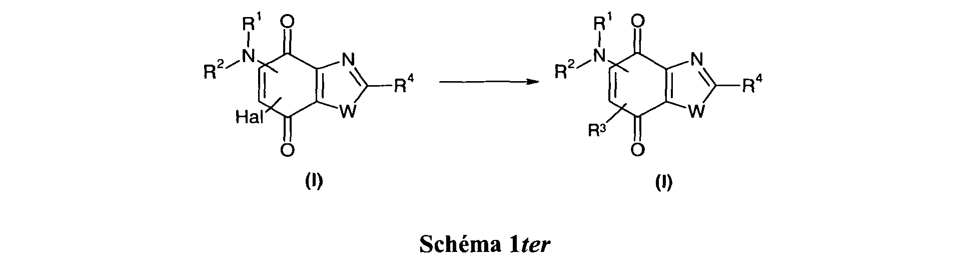

### Préparation des intermédiaires de formule générale (A)

Les composés de formule générale **(A)** dans lesquels L, R³, R⁴ et W sont tels que définis ci-dessus peuvent être obtenus, schéma 2, à partir des composés de formule générale **(B)** dans lesquels L, R³, R⁴ et W sont tels que définis ci-dessus et :
- l'un de Q et Q' représente un radical amino ou hydroxyle et l'autre représente un atome d'hydrogène ; ou
- Q et Q' représentent chacun un radical amino ; ou
- Q et Q' représentent chacun un radical hydroxyle ; ou enfin
- Q et Q' représentent chacun un radical méthoxy.

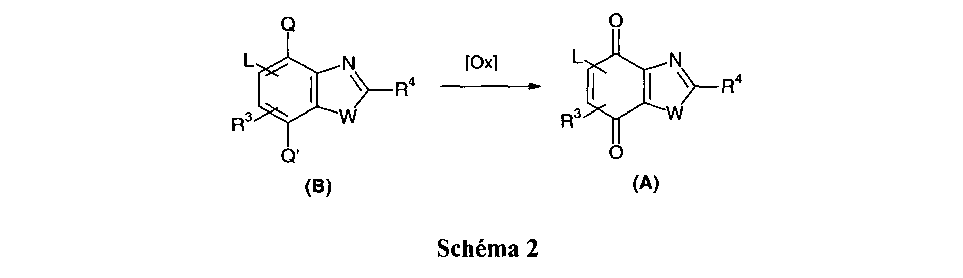

Dans le cas où les composés de formule générale **(B)** sont tels que Q et Q' représentent des radicaux méthoxy, les composés de formule générale **(A)** sont obtenus par traitement par du nitrate de cérium(IV) et d'ammonium (Beneteau et coll., Eur. J. Med. Chem. (1999), 34(12), 1053-1060). Dans les autres cas, les composés de formule générale **(A)** sont obtenus par oxydation des composés de formule générale **(B),** par exemple par utilisation de FeCl₃ en milieu acide (Antonini et coll., Heterocycles (1982), 19(12), 2313-2317) ou de sel de Fremy (nitrosodisulfonate de potassium). (Ryu et coll., Bioorg. Med. Chem. Lett. (2000), 10, 461-464), ou par l'utilisation d'un réactif comportant un iode hypervalent tel que le [bis(acétoxy)iodo]benzène ou le [bis(trifluoroacétoxy)iodo]benzène dans l'acétonitrile aqueux à une température de préférence comprise entre -20 °C et la température ambiante (soit environ 25 °C), et de préférence à environ -5 °C (Kinugawa et coll., Synthesis, (1996), 5, 633-636).

Dans le cas particulier où L et R³ représentent des atomes halogènes, les composés de formule générale **(A)** peuvent être obtenus, schéma 3, par halooxydation des composés de formule générale **(B)** dans lesquels L et R³ représentent des atomes d'hydrogène et Q et/ou Q' est (sont) choisi(s) parmi un radical amino et un radical hydroxy par action, par exemple, de perchlorate de potassium ou de sodium en milieu acide (Ryu et coll., Bioorg. Med. Chem. Lett. (1999), 9, 1075-1080).

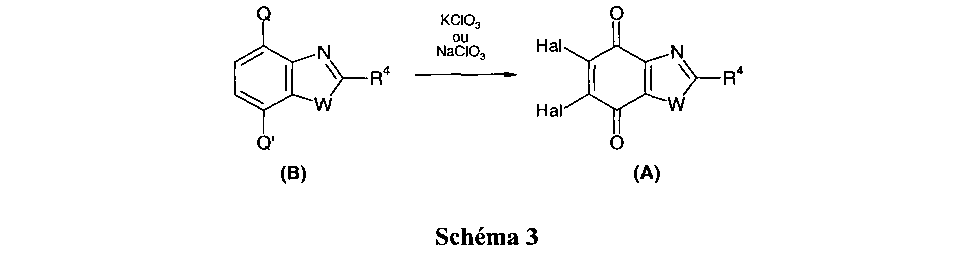

### Préparation des intermédiaire de formule générale (B)

Certains des composés de formule générale **(B)** dans lesquels L, R³, R⁴, Q, Q' et W sont tels que définis ci-dessus sont des produits industriels connus disponibles chez les fournisseurs usuels.

S'ils ne sont pas commerciaux et dans le cas particulier où Q ou Q' représente un radical amino, les composés de formule générale **(B)** peuvent notamment être obtenus à partir des dérivés nitro de formule **(B.ii)** dans lesquels Q ou Q' représente un radical nitro par des méthodes de réduction bien connues de l'homme de l'art telles que, par exemple l'hydrogénation en présence d'un catalyseur palladié ou le traitement par du chlorure d'étain dans l'acide chlorhydrique. S'ils ne sont pas commerciaux, les composés de formule **(B.ii)** peuvent eux-mêmes être obtenus à partir des composés de formule générale **(B.i)** dans lesquels les positions correspondant aux radicaux Q et Q' sont substituées par des atomes d'hydrogène par des méthodes de nitration bien connues de l'homme de l'art telles que, par exemple, le traitement par un mélange d'acide nitrique et d'acide sulfurique (cf. le schéma 4 où seul le cas dans lequel les composés de formule générale **(B)** sont tels que Q = NH₂ et Q' = H est représenté).

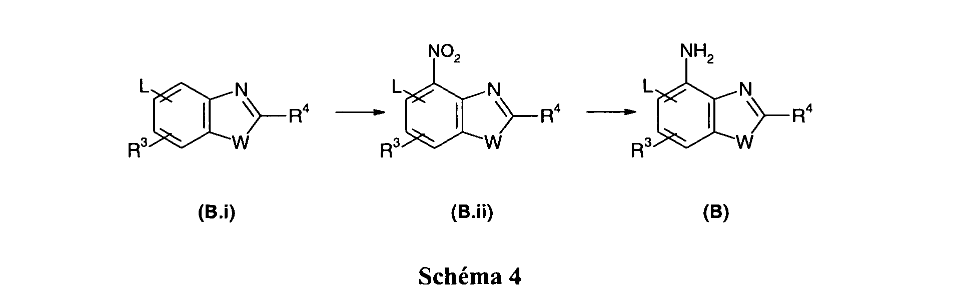

Alternativement, les composés de formule générale **(B)** non commerciaux dans lesquels Q représente un radical amino, Q' un atome d'hydrogène et W un atome d'oxygène, peuvent être obtenus par traitement des tétrahydrobenzoxazoles de formule générale **(B.vi)** par le chlorhydrate d'hydroxylamine pour donner les oximes de formule générale **(B.v),** eux-mêmes traités par de l'acide polyphosphorique à chaud (cf. Young Kook Koh et coll., J. Heterocyclic Chem. (2001), 38, 89-92) pour fournir les composés de formule générale **(B).** Les composés de formule générale **(B.vi)** peuvent eux-mêmes être obtenus à partir des 1,3-dicétones cycliques de formule générale **(B.viii)** tout d'abord par conversion en diazodicétones de formule générale **(B.vii)** par réaction de diazotransfert, par exemple, par action de tosyl azide ou de 4-acétamidobenzènesulfonylazide en présence de triéthylamine dans un solvant tel que le dichlorométhane ou le chloroforme anhydres (V. V. Popic et coll., Synthesis (1991), 3, 195-198) suivie d'une cycloaddition de ces diazodicétones de formule générale **(B.vii)** par des nitriles de formule générale R⁴-CN en présence d'un catalyseur de type Rhodium (II) (Y. R. Lee, Heterocycles (1998), 48, 875-883) (cf. schéma 4*bis*).

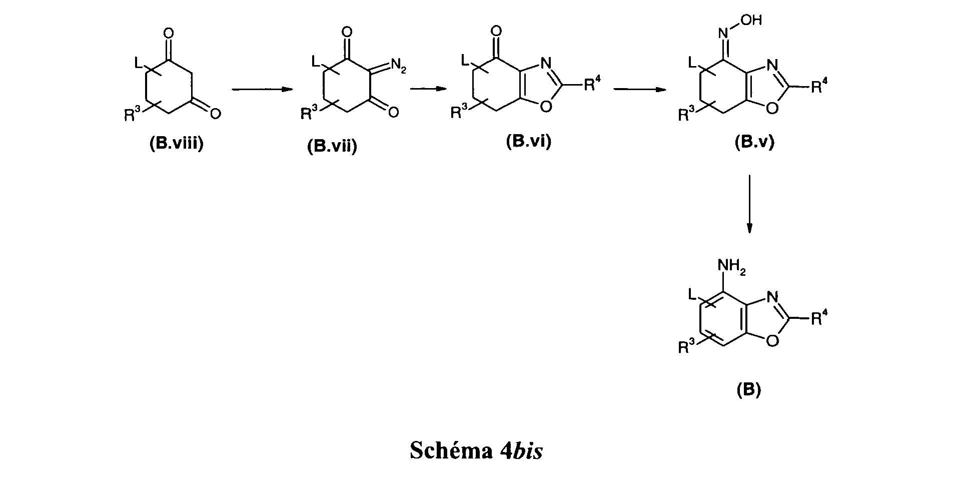

S'ils ne sont pas commerciaux et dans le cas particulier où Q représente hydroxyle, Q' un atome d'hydrogène et W un atome d'oxygène, les composés de formule générale **(B)** peuvent être obtenus par aromatisation des oxazolocyclohexanones de formule générale **(B.vi).** Une tell aromatisation peut être effectuée en deux étapes comme montré dans le Schéma 4*ter,* d'abord une halogénation en position α du carbonyle (qui conduit aux intermédiaires de formule générale **(B.ix)** dans laquelle Hal est un atome halogène), puis β-élimination de l'halogène par traitement avec une base. L'halogénation peut être faite, par exemple, à l'aide de brome dans de l'acide acétique à température ambiante, de tribromure pyridinium dans de l'acide acétique à 50 °C, de bromure de cuivre (II) dans de l'acétate d'éthyle ou de l'acétonitrile au reflux, ou aussi de chlorure de phenylsélényle dans de l'acétate d'éthyle à température ambiante. L'élimination de l'halogénure résultant peut être effectuée par du diazabicyclo[5.4.0]undec-7-ène (DBU) dans du tétrahydrofuranne à température ambiante ou par du carbonate de lithium dans du diméthylformamide. Des exemples de ces réactions sont fournis par M. Tany et coll., Chem. Pharm. Bull. (1996), 44, 55-61; M.A. Ciufolini et coll., J. Am. Chem. Soc. (1995), 117, 12460-12469; et M.E. Jung et L.S. Starkey, Tetrahedron (1997), 53, 8815-8824. 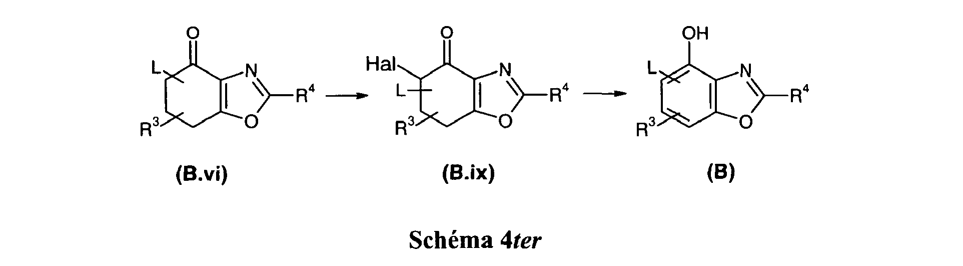 S'ils ne sont pas commerciaux et dans le cas particulier où R⁴ représente un radical -CH₂-NR²¹R²², les composés de formule générale **(B)** peuvent être obtenus, schéma 5, à partir des composés de formule générale **(B.iii)** dans lesquels R⁴ représente le radical méthyle, que l'on soumet d'abord à une réaction de bromation radicalaire à l'aide de *N*-bromosuccinimide en présence d'un initiateur tel que le 2,2'-azobis(2-méthylpropionitrile) ou le dibenzoylperoxyde dans un solvant aprotique tel que le tétrachlorure de carbone (CCl₄) à une température de préférence comprise entre la température ambiante (i.e. environ 25 °C) et 80 °C et sous irradiation par une lampe UV (Mylari et coll., J. Med. Chem. (1991), 34, 108-122), suivie d'une substitution de l'intermédiaire de formule générale **(B.iv)** par des amines de formule HNR²¹R²² avec R²¹ et R²² sont tels que définis ci-dessus.

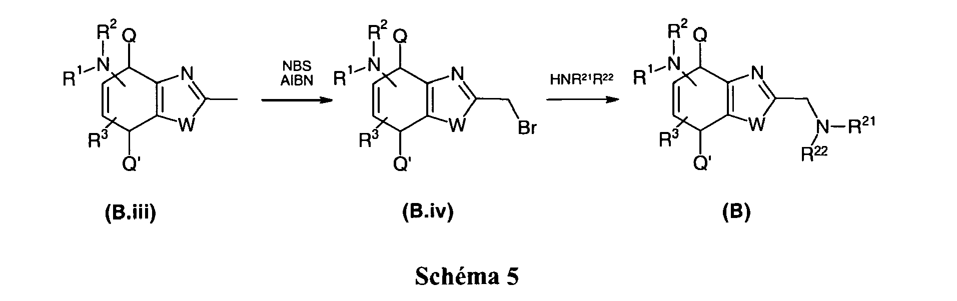

Alternativement, les composés de formule générale **(B)** non commerciaux dans lesquels R⁴ représente un radical -CH₂-NR²¹R²² peuvent être obtenus selon la méthode représentée dans le schéma 4 plus haut, à partir des composés de formule générale **(B.i)** dans laquelle R⁴ représente un radical -CH₂-NR²¹R²², ceux-ci étant eux-mêmes obtenus à partir des composés de formule générale **(B.i)** dans laquelle R⁴ représente un radical CH₂-Br par substitution par des amines de formule HNR²¹R²² avec R²¹ et R²² tels que définis ci-dessus. Les composés de formule générale **(B.i)** dans laquelle R⁴ représente un radical CH₂-Br peuvent être obtenus, comme décrit ci-dessus, à partir des composés de formule générale **(B.i)** dans lesquels R⁴ représente le radical méthyle, que l'on soumet à une réaction de bromation radicalaire.

S'ils ne sont pas commerciaux et dans le cas particulier où R⁴ représente un radical -CH₂-CO-NR¹⁹R²⁰, les composés de formule générale **(B)** peuvent être obtenus à partir des composés de formule générale **(B)** dans lesquels R⁴ représente le radical -CH₂-COOH, par les méthodes classiques de la synthèse peptidique (M. Bodansky, The Practice of Peptide Synthesis, 145 (Springer-Verlag, 1984)), par exemple dans le tétrahydrofuranne, le dichlorométhane ou le diméthylformamide en présence d'un réactif de couplage tel que le cyclohexylcarbodiimide (DCC), le 1,1'-carbonyldiimidazole (CDI) *(*J. Med. Chem. (1992), 35(23), 4464-4472) ou l'hexafluorophosphate de benzotriazol-1-yl-oxy-tris-pyrrolidino-phosphonium (PyBOP) (Coste et coll., Tetrahedron Lett. (1990), 31, 205).

Les composés de formule générale **(B)** dans lesquels R⁴ représente -CH₂-COOH peuvent être obtenus à partir des composés de formule générale **(B)** dans lesquels R⁴ représente le radical -CH₂-COOR¹⁸ dans lequel R¹⁸ représente un radical alkyle par hydrolyse de la fonction ester dans des conditions connues de l'homme du métier.

Les composés de formule générale **(B)** dans lesquels W représente S, Q et Q' représentent chacun un radical méthoxy et L représente un atome halogène ou un atome d'hydrogène peuvent être obtenus, schéma 6, par traitement des N-(2,5-diméthoxyphényl)thioamides de formule générale **(B.x)** par une solution aqueuse de ferricyanure de potassium en milieu sodique à température ambiante (Lyon et coll., J. Chem. Soc., Perkin Trans. 1 (1999), 437-442). Les composés de formule générale **(B.x)** peuvent eux-mêmes être obtenus en partant des 2,5-diméthoxyanilines acylées correspondantes de formule générale **(B.xii),** par exemple par action d'un chlorure d'acide de formule générale R⁴COCl ou d'un acide carboxylique de formule générale R⁴COOH activé selon des méthodes connues de l'homme du métier, pour donner les N-(2,5-dimethoxyphenyl)amides de formule générale **(B.xi)** eux-mêmes convertis en les thioamides de formule générale **(B.x)** par action de réactif de Lawesson dans du toluène au reflux.

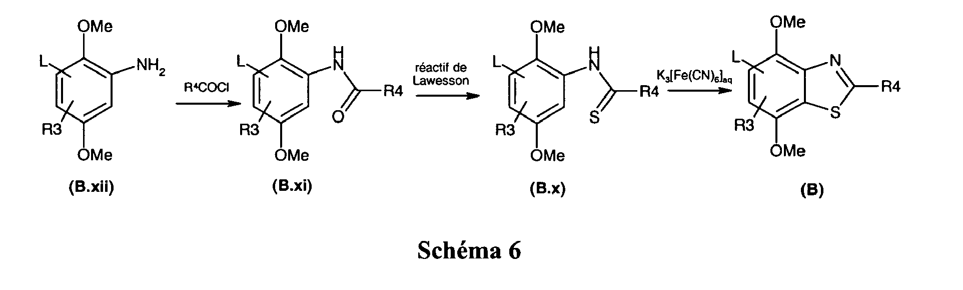

Dans les autres cas, les composés de formule générale **(B)** peuvent être obtenus, schéma 6*bis,* à partir des composés de formule générale **(C)** dans lesquels L, R³ et W sont tels que définis ci-dessus et Q ou Q' représente le radical NO₂ par condensation avec l'orthoester de formule générale R⁴C(OR)₃ dans laquelle R est un radical alkyle, par exemple en présence d'une quantité catalytique d'un acide tel que, par exemple, l'acide paratoluènesulfonique, à une température comprise entre la température ambiante et 200°C et de préférence à environ 110 °C (Jenkins et coll., J. Org. Chem . (1961), 26, 274) ou encore dans un solvant protique tel que l'éthanol à une température comprise entre la température ambiante (i.e. environ 25 °C) et 80 °C et de préférence à environ 60 °C (Scott et coll., Synth. Commun. (1989), 19, 2921). Un certain nombre d'orthoesters sont des produits industriels connus disponibles chez les fournisseurs usuels. La préparation d'orthoesters en traitant des composés nitriles variés par du gaz chlorhydrique dans un alcool est connue de l'homme du métier.

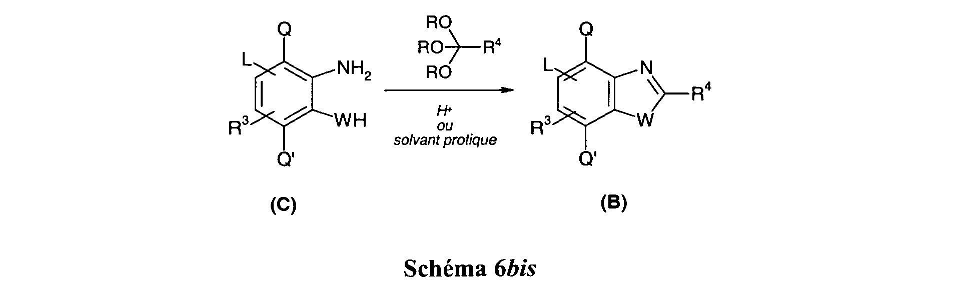

Les composés de formule générale **(B)** dans lesquels L, R³, R⁴ et W sont tels que définis ci-dessus et Q ou Q' représente le radical NO₂ peuvent aussi être obtenus à partir des composés de formule générale **(C)** dans lesquels L, R³, R⁴ et W sont tels que définis ci-dessus et l'un de Q et Q' représente le radical NO₂ tandis que l'autre représente un atome d'hydrogène par condensation de ces derniers avec un chlorure d'acide de formule R⁴-COCl sous atmosphère inerte et dans un solvant polaire et légèrement basique tel que la N-méthyl-2-pyrrolidinone (Brembilla et coll., Synth. Commun (1990), 20, 3379-3384) ou par condensation de ces derniers avec un acide carboxylique de formule générale R⁴-COOH en présence d'acide polyphosphorique à haute température (Ying-Hung So et coll., Synth. Commun. (1998), 28, 4123-4135) ou en présence d'acide borique dans un solvant tel que le xylène au reflux (M. Terashima, Synthesis (1982), 6, 484-485).

Les composés de formule générale **(B)** dans lesquels L, R³, R⁴ et W sont tels que définis ci-dessus et Q ou Q' représente le radical NO₂ peuvent également être obtenus à partir des composés de formule générale **(C)** dans lesquels L, R³ R⁴ et W sont tels que définis ci-dessus et l'un de Q et Q' représente le radical NO₂ tandis que l'autre représente un atome d'hydrogène par condensation avec un aldéhyde de formule générale R⁴-CHO puis traitement de la base de Schiff obtenue par un agent oxydant tel que le [bis(acétoxy)iodo]benzène, le chlorure ferrique ou le diméthylsulfoxyde (Racane et coll., Monatsh. Chem. (1995), 126(12), 1375-1381) ou par déshydratation par l'acide acétique glacial à une température comprise entre la température ambiante (i.e. environ 25 °C) et 100 °C (Katritzky et Fan, J. Heterocyclic Chem. (1988), 25, 901-906).

Les composés de formule générale **(B)** dans lesquels L, R³, R⁴ et W sont tels que définis ci-dessus et l'un de Q et Q' représente le radical NO₂ tandis que l'autre représente un atome d'hydrogène peuvent encore être obtenus à partir des composés de formule générale **(C)** par condensation avec un nitrile de formule générale R⁴-CN dans un mélange de solvants du type méthanol / acide acétique glacial à une température comprise entre la température ambiante (i.e. environ 25 °C) et 100 °C (Nawwar et Shafik, Collect. Czech Chem. Commun. (1995), 60(12), 2200-2208).

### Préparation des intermédiaires de formule générale (C)

Certains des composés de formule générale **(C)** dans lesquels L, R³, Q, Q' et W sont tels que définis ci-dessus sont des produits industriels connus disponibles chez les fournisseurs usuels.

Certains composés de formule générale **(C)** dans lesquels l'un de Q et Q' représente le radical NO₂ tandis que l'autre représente un atome d'hydrogène peuvent être obtenus à partir des composés de formule générale **(D)** 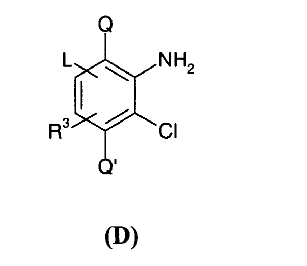 dans lesquels L, R³, Q et Q' sont tels que définis ci-dessus par réaction, dans le cas où W représente S, avec du sulfure de sodium hydraté à une température comprise entre la température ambiante (i.e. environ 25 °C) et 100 °C (Katritzky et Fan, J. Heterocyclic Chem. (1988), 25, 901-906).

Enfin, dans le cas particulier où W représente O, les composés de formule générale **(C)** sont des produits industriels connus disponibles chez les fournisseurs usuels ou peuvent être synthétisés à partir de tels produits selon des méthodes courantes pour l'homme du métier.

### Séparation de mélanges de régioisomères

Dans certains cas, il peut arriver que les composés de formule générale **(I)** préparés selon les métyhodes susmentionnées soient obtenus sous forme de mélanges de régioisomères.

Dans de telles situations, le mélange peut être séparé grâce à des techniques standard de chromatographie liquide sur colonne ou sur couche mince préparative (en utilisant un support tel que de la silice ou encore un gel comme un gel de polydextranes réticulés formant un réseau tridimensionnel comme un gel de type Sephadex^{®} LH-20). L'homme du métier choisira l'éluant le mieux adapté à la séparation du mélange ; un tel éluant pourra être par exemple un mélange ternaire isopropanol/acétate d'éthyle/eau 1/1/1.

### Préparation des composés de formule générale (II)

Les composés de formule générale **(II)** ont été décrits dans la demande de brevet PCT WO 02/09686.

### Préparation des composés de formule générale (III)

Les composés de formule générale **(III)** ont notamment été décrits dans la demande de brevet PCT WO 97/00876.

### Préparation des composés de formule générale (IV)

Les composés de formule générale **(IV)** ont été décrits dans la demande de brevet PCT WO 97/30053.

Le composé le plus préféré, la (1*R*)-1-[({(2*R*)-2-amino-3-[(8*S*)-8-(cyclohexylméthyl)-2-phényl-5,6-dihydroimidazo[1,2-*a*]pyrazin-7(8*H*)-yl]-3-oxopropyl}dithio)méthyl]-2-[(8*S*)-8-(cyclohexylméthyl)-2-phényl-5,6-dihydroimidazo[1,2-*a*]pyrazin-7(8*H*)-yl]-2-oxoéthylamine, peut être préparé sous forme de son sel tétrahydrochlorhydrate selon le procédé en 2 étapes représenté dans le schéma 7 ci-après.

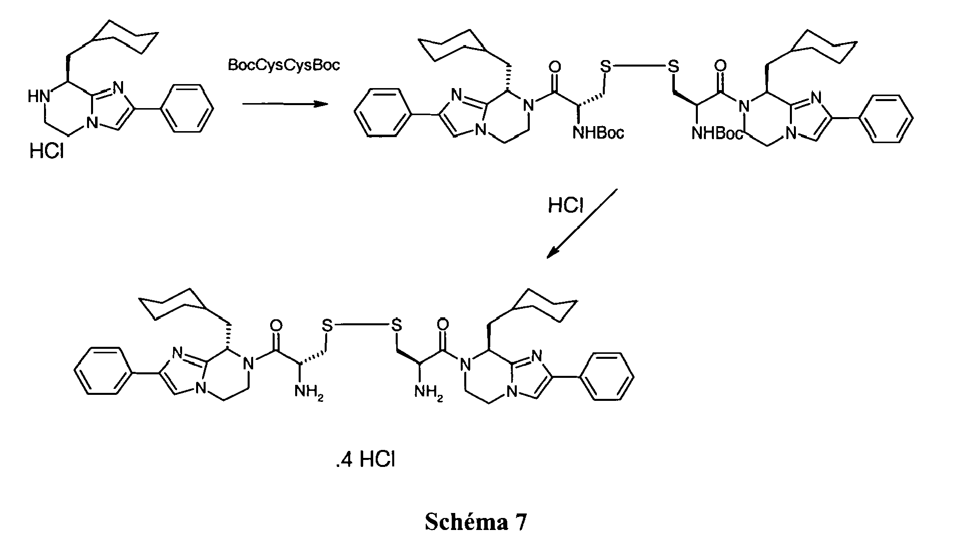

Selon ce procédé, la (8*S*)-8-(cyclohexylméthyl)-2-phényl-5,6,7,8-tétrahydroimidazo[1,2-*a*]pyrazine (éventuellement sous la forme de son sel chlorhydrate ; produit obtenu selon le protocole décrit dans la demande PCT WO 97/30053) peut d'abord être condensée avec un équivalent de Boc-Cys-Cys-Boc en présence d'un agent de couplage peptidique (p.ex. le HTBU) et d'une base (p.ex. la diisopropyléthylamine). Le composé intermédiaire peut ensuite être déprotégé et converti en le sel tétrachlorhydrate désiré en une seule étape par addition d'une solution de HCl dans un alcool inférieur (p.ex. l'isopropanol), cette réaction étant de préférence effectuée dans le même alcool inférieur.

### Préparation des composés de formule générale (V)

Les composés de formule générale **(V)** ont été décrits dans la demande de brevet PCT WO 00/39130.

### Préparation des composés de formule générale (VI)

Les composés de formule générale **(VI)** ont été décrits dans la demande de brevet PCT WO 97/21701.

### Préparation des composés de formule générale (VII)

Les composés de formule générale (**VII**) ont été décrits dans la demande de brevet PCT WO 02/50073.

En ce qui concerne les températures auxquelles il est fait référence dans le présent texte, le terme « environ *XX*°C » indique que la température en question correspond à un intervalle de plus ou moins 10 °C autour de la température de *XX* °C, et de préférence à un intervalle de plus ou moins 5 °C autour de la température de *XX*°C. En ce qui concerne les autres valeurs numériques auxquelles il est fait référence dans le présent texte, le terme « environ *YY* » indique que la valeur en question correspond à un intervalle de plus ou moins 10% autour de la valeur *YY*, et de préférence à un intervalle de plus ou moins 5% autour de la valeur *YY.*

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnées ici sont incorporées par référence.

Les exemples suivants sont présentés pour illustrer les procédures ci-dessus et ne doivent en aucun cas être considérés comme une limite à la portée de l'invention.

### EXEMPLES DE COMPOSÉS DE FORMULE GÉNÉRALE (I)

### Méthode employée pour la mesure du temps de rétention (t.r) et du pic moléculaire (MH+)

Les composés sont caractérisés par leur temps de rétention (t.r.), exprimé en minutes, déterminé par chromatographie liquide (CL), et leur pic moléculaire (MH+) déterminé par spectrométrie de masse (SM), un spectromètre de masse simple quadripôle (Micromass, modèle Platform) équipé d'une source électrospray est utilisé avec une résolution de 0,8 da à 50% de vallée.

Pour les exemples 1 à 138 ci-après, les conditions d'élution correspondant aux résultats indiqués sont les suivantes: passage d'un mélange acétonitrile-eau-acide trifluoroacétique 50-950-0,2 (A) à un mélange acétonitrile-eau 950-50 (B) par un gradient linéaire sur une période de 8,5 minutes, puis élution avec le mélange B pur pendant 10,5 minutes.

### Exemple 1 : 2-méthyl-5-{[2-(4-morpholinyl)éthyl]amino}-1,3-benzothiazole-4,7-dione :

51,2 µl (0,39 mmol ; 3 équivalents) de 4-(2-aminoéthyl)morpholine sont ajoutés à 27 mg (0,129 mmol) de 5-méthoxy-2-méthyl-4,7-dioxobenzothiazole en solution dans 2 ml d'éthanol anhydre. Le mélange réactionnel est agité au reflux pendant 18 heures puis le solvant est évaporé sous pression réduite. Le résidu est purifié sur colonne de silice (éluant : méthanol à 5% dans du dichlorométhane). Le composé attendu est obtenu sous forme d'une poudre rouge.

RMN ¹H (DMSO d6, 400 MHz, δ): 7,45 (t, 1H, NH); 5,49 (s, 1H, CH); 3,58-3,55 (m, 4H, 2 CH₂) ; 3,26 (t, 2H, CH₂) ; 2,75 (s, 3H, CH₃) ; 2,54 (t, 2H, CH₂) ; 2,42-2,40 (m, 4H, 2 CH₂)-SM-CL : MH+ = 308,25 ; t.r. = 6,89 min.

### Exemple 2 : chlorhydrate de 5-{[2-(diméthylamino)éthyl]amino}-2-méthyl-1,3-benzothiazole-4,7-dione :

### 2.1) 5-{[2-(diméthylamino)éthyl]amino}-2-méthyl-1,3-benzothiazole-4,7-dione:

Ce composé est obtenu de façon analogue à celle employée pour le composé de l'exemple 1.

RMN ¹H (DMSO d6, 400 MHz, δ): 7,34 (t, 1H, NH); 5,48 (s, 1H, CH); 3,24-3,20 (m, H, CH₂) ; 2,77 (s, 3H, CH₃) ; 2,47 (m, 2H, CH₂) ; 2,18 (s, 6H, 2 CH₃). SM-CL : MH+ = 266,27 ; t.r. = 6,83 min.

### 2.2) chlorhydrate de 5-{[2-(diméthylamino)éthyl]amino}-2-méthyl-1,3-benzothiazole-4, 7-dione :

0,166 g de l'intermédiaire 2.1 sont dissous dans 1,88 ml (1,88 mmol; 3 éq.) d'une solution molaire d'acide chlorhydrique dans de l'éther et le mélange réactionnel est agité pendant 3 heures à température ambiante. Le précipité résultant est recueilli par filtration, lavé avec de l'éther éthylique et séché sous pression réduite pour donner un solide rouge foncé. Point de fusion : 138-140 °C.

RMN ¹H (DMSO d6, 400 MHz, δ): 10,00 (s, 1H, NH⁺) ; 7,78 (t, 1H, NH) ; 5,68 (s, 1H, CH); 3,59-3,55 (m, 2H, CH₂); 3,32-3,27 (m, 2H, CH₂); 2,85-2,80 (s, 6H, 2 CH₃) ; 2,76 (s, 3H, CH₃).
SM-CL : MH+ = 266,12 ; t.r. = 6,92 min.

### Les composés des exemples 3 à 14 sont obtenus de façon analogue à celle employée pour l'exemple 1.

### Exemple 3 : 5-{[6-(diméthylamino)hexyl]amino}-2-méthyl-1,3-benzothiazole-4,7-dione :

SM-CL : MH+ = 322,33 ; t.r. = 7,36 min.

### Exemple 4 : 5-{[3-(diméthylamino)-2,2-diméthylpropyl]amino}-2-méthyl-1,3-benzothiazole-4,7-dione :

RMN ¹H (DMSO d6, 400 MHz, δ): 8,62 (t, 1H, NH); 5,45 (s, 1H, CH); 3,07-3,06 (m, 2H, CH₂); 2,74 (s, 3H, CH₃) ; 2,29-2,30 (m, 2H, CH₂); 2,27 (s, 6H, 2CH₃) ; 0,93 (s, 6H, 2 CH₃).
LC-MS : MH+ = 308,32 ; t.r. = 7,16 min.

### Exemple 5 : 2-méthyl-5-{[3-(4-méthyl-1-pipérazinyl)propyl]amino}-1,3-benzothiazole-4,7-dione :

RMN ¹H (DMSO d6, 400 MHz, δ): 8,14 (t, 1H, NH) ; 5,46 (s, 1H, CH) ; 3,25-3,26 (m, 2H, CH₂); 3,21-3,19 (m, 2H, CH₂); 2,74 (s, 3H, CH₃) ; 2,49-2,48 (m, 2H, CH₂); 2,37-2,32 (m, 6H, 3CH₂); 2,16 (s, 3H, CH₃); 1,72 (t, 2H, CH₂).
SM-CL : MH+ = 335,34 ; t.r. = 6,87 min.

### Exemple 6 : 5-[(1-éthylhexyl)amino]-2-méthyl-1,3-benzothiazole-4,7-dione :

SM-CL : MH+ = 307,32 ; t.r. = 11,45 min.

### Exemple 7 : 5-[(1-adamantylméthyl)amino]-2-méthyl-1,3-benzothiazole-4,7-dione :

SM-CL : MH+ = 343,31 ; t.r. = 11,73 min.

### Exemple 8 : 2-méthyl-5-[(2-thiénylmèthyl)amino]-1,3-benzothiazole-4,7-dione :

SM-CL : MH+ = 291,16 ; t.r. = 9,24 min.

### Exemple 9 : 5-[(3-chlorobenzyl)amino]-2-méthyl-1,3-benzothiazole-4,7-dione :

SM-CL : MH+ = 319,24 ; t.r. = 9,95 min.

### Exemple 10 : 2-méthyl-5-[(4-pyridinylméthyl)amino]-1,3-benzothiazole-4,7-dione :

SM-CL : MH+ = 286,13 ; t.r. = 6,97 min.

### Exemple 11 : 2-méthyl-5-(propylamino)-1,3-benzothiazole-4,7-dione :

SM-CL : MH+ = 237,16 ; t.r. = 8,74 min.

### Exemple 12 : 5-{[3-(1H-imidazol-1-yl)propyl]amino}-2-méthyl-1,3-benzothiazole-4,7-dione :

SM-CL : MH+ = 303,17 ; t.r. = 7,07 min.

### Exemple 13 : 4-{2-[(2-méthyl-4,7-dioxo-4,7-dihydro-1,3-benzothiazol-5-yl)amino]éthyl} benzènesulfonamide :

SM-CL : MH+ = 378,10 ; t.r. = 8,31 min.

### Exemple 14 : 5-(4-benzyl-1-pipérazinyl)-2-méthyl-1,3-benzothiazole-4,7-dione :

SM-CL : MH+ = 354,19 ; t.r. = 7,53 min.

### Exemple 15 : 5-anilino-2-éthyl-1,3-benzoxazole-4,7-dione ou 6-anilino-2-éthyl-1,3-benzoxazole-4,7-dione :

### 15.1) 2-éthyl-4-nitro-1,3-benzoxazole :

Un mélange de 2-amino-3-nitrophénol (1 éq.), de triéthyl orthopropionate (2 éq.) et d'acide *p*-toluènesulfonique (en quantité catalytique) est agité à 110 °C jusqu'à disparition de l'aminophénol vérifiée par chromatographie sur couche mince (2 h). Après refroidissement, le mélange réactionnel est repris au toluène et évaporé sous vide puis traité à l'isopropanol. Le précipité résultant est recueilli par filtration, lavé avec de l'isopropanol et de l'isopentane, puis séché sous pression réduite pour donner un solide brun-violet.
RMN ¹H (DMSO d6, 400 MHz, δ): 8,15 (dd, 2H) ; 7,58 (t, 1H) ; 3,06 (q, 2H) ; 1,38 (t, 3H).
SM-CL : MH+ = 193,02 ; t.r. = 9,23 min.

### 15.2) 2-éthyl-1,3-benzoxazol-4-amine :

Le 2-éthyl-4-nitro-1,3-benzoxazole est hydrogéné sous une pression de 8 bars en présence de charbon palladié à 10% (0,01 éq.) en employant du méthanol comme solvant. Le catalyseur est séparé par filtration et le méthanol est éliminé sous pression réduite. Le résidu est repris dans l'éther éthylique pour donner un solide violet-pâle qui est recueilli par filtration et séché. Point de fusion : 46 °C.
RMN ¹H (DMSO d6, 400 MHz, δ): 6,97 (t, 1H) ; 6,72 (d, 1H) ; 6,47, d, 1H) ; 5,45 (s, 2H) ; 2,87 (q, 2H) ; 1,32 (t, 3H).
SM-CL : MH+ = 162,99 ; t.r. = 8,72 min.

### 15.3) 2-éthyl-1,3-benzoxazole-4,7-dione :

Une solution de [bis(trifluoroacétoxy)iodo]benzène (2,2 éq.) dans un mélange d'acétonitrile et d'eau (80/20) est ajouté au goutte-à-goutte à une solution de 2-éthyl-1,3-benzoxazol-4-amine (1 éq.) dans un même mélange acétonitrile/eau maintenue à -5 °C. Le milieu réactionnel est ensuite dilué avec de l'eau et extrait au dichlorométhane. La phase organique résultante est lavée avec de l'eau, séchée sur sulfate de sodium et concentrée pour donner une pâte brune. Une purification par chromatographie à moyenne pression sur gel de silice donne, après reprise dans de l'éther diisopropylique, un solide cristallin jaune. Point de fusion : 99 °C.
RMN ¹H (CDCl₃, 400 MHz, δ): 6,75 (dd, 2H) ; 2,99 (q, 2H) ; 1,45 (t, 3H).
SM-CL : MH+ = 177,83 ; t.r. = 8,29 min.

### 15.4) 5-anilino-2-éthyl-1,3-benzoxazole-4,7-dione ou 6-anilino-2-éthyl-1,3-benzoxazole-4,7-dione :

Un mélange de 2-éthyl-1,3-benzoxazole-4,7-dione (1 éq) et d'aniline (1,1 éq.) dans de l'éthanol est maintenu sous agitation pendant 1 heure. Le milieu réactionnel tourne au violet foncé. Après concentration, le résidu est purifié par chromatographie à moyenne pression sur silice pour donner une poudre violette. Point de fusion : 200 °C.
RMN ¹H (DMSO d6, 400 MHz, δ) : 9,38 (s, 1H) ; 7,44 (t, 2H) ; 7,36 (d, 2H) ; 7,22 (t, 1H) ; 5,69 (s ; 1H) ; 2,94 (q, 2H) ; 1,29 (t, 3H).
SM-CL : MH+ = 269,11 ; t.r. = 9,76 min.

### Exemple 16 : 5-anilino-6-chloro-2-éthyl-1,3-benzoxazole-4,7-dione ou 6-anilino-5-chloro-2-éthyl-1,3-benzoxazole-4,7-dione :

Une solution d'un mélange de 5-anilino-2-éthyl-1,3-benzoxazole-4,7-dione et 6-anilino-2-éthyl-1,3-benzoxazole-4,7-dione (1 éq.) dans l'acide acétique est traitée par du *N*-chlorosuccinimide (1,1 éq.) à température ambiante. Le milieu réactionnel est maintenu sous agitation pendant 2 heures avant d'être concentré, repris dans de l'éthanol et concentré de nouveau. Le résidu est purifié par chromatographie à moyenne pression sur silice pour donner une poudre violette. Point de fusion : 159 °C.
RMN ¹H (DMSO d6, 400 MHz, δ) : 9,39 (s, 1H) ; 7,30 (t, 2H); 7,11 (m, 3H); 2,96 (q, 2H) ; 1,30 (t, 3H).
SM-CL : MH+ = 303,01 ; t.r. = 10,28 min.

### Exemple 17 : 2-éthyl-5-[(4-fluorophényl)amino]-1,3-benzoxazole-4,7-dione ou 2-éthyl-6-[(4-fluorophényl)amino]-1,3-benzoxazole-4,7-dione :

Le protocole expérimental utilisé est identique à celui décrit pour l'exemple 15, la 4-fluoroaniline remplaçant l'aniline dans la quatrième et dernière étape. Point de fusion : 232 °C.
RMN ¹H (DMSO d6, 400 MHz, δ) : 9,38 (s, 1H) ; 7,37 (t, 2H) ; 7,26 (t, 2H) ; 5,57 (s, 1H) ; 2,93 (q, 2H) ; 1,30 (t, 3H).
SM-CL : MH+ = 287,09 ; t.r. = 9,88 min.

### Les composés des exemples 18 à 31 sont obtenus de façon analogue à celle décrite pour l'exemple 1.

### Exemple 18 : 5-[(2-méthoxyéthyl)amino]-2-méthyl-1,3-benzothiazole-4,7-dione :

SM-CL : MH+ = 253,20 ; t.r. = 8,00 min.

### Exemple 19 : 2-méthyl-5-[(2-pyrrolidin-1-yléthyl)amino]-1,3-benzothiazole-4,7-dione :

RMN ¹H (DMSO d6, 400 MHz, δ) : 7,45 (m, 1H, NH); 5,47 (s, 1H, CH); 3,28-3,23 (m, 2H, CH₂); 2,75 (s, 3H, CH₃) ; 2,66-2,63 (m, 2H, CH₂); 2,48-2,49 (m, 4H, 2CH₂) ; 1,68-1,67 (m, 4H, 2CH₂).
SM-CL : MH+ = 292,13 ; t.r. = 7,11 min.

### Exemple 20 : 2-méthyl-5-[(2-pipéridin-1-yléthyl)amino]-1,3-benzothiazole-4,7-dione :

SM-CL : MH+ = 306,24 ; t.r. = 7,22 min.

### Exemple 21 : 5-{[2-(diisopropylamino)éthyl]aminol-2-méthyl-1,3-benzothiazole-4,7-dione :

SM-CL : MH+ = 322,33 ; t.r. = 7,37 min.

### Exemple 22 : 5-[(1-benzylpyrrolidin-3-yl)amino]-2-méthyl-1,3-benzothiazole-4,7-dione :

SM-CL : MH+ = 354,28 ; t.r. = 7,70 min.

### Exemple 23 : 5-{[3-(diméthylamino)propyl]amino}-2-méthyl-1,3-benzothiazole-4,7-dione :

SM-CL : MH+ = 280,15 ; t.r. = 7,01 min.

### Exemple 24 : 2-méthyl-5-{[2-(1-méthylpyrrolidin-2-yl)éthyl]amino}-1,3-benzothiazole-4,7-dione :

SM-CL : MH+ = 306,30 ; t.r. = 7,23 min.

### Exemple 25 : 2-méthyl-5-{[3-(2-méthylpipéridin-1-yl)propyl]amino}-1,3-benzothiazole-4,7-dione :

SM-CL : MH+ = 334,29 ; t.r. = 7,38 min.

### Exemple 26 : 5-{[4-(diméthylamino)butyl]amino}-2-méthyl-1,3-benzothiazole-4,7-dione :

SM-CL : MH+ = 294,16 ; t.r. = 7,11 min.

### Exemple 27 : 5-{[5-(diméthylamino)pentyl]amino}-2-méthyl-1,3-benzothiazole-4,7-dione :

SM-CL : MH+ = 308,16 ; t.r. = 7,22 min.

### Exemple 28 : 5-(2,3-dihydro-1H-indén-1-ylamino)-2-méthyl-1,3-benzothiazole-4,7-dione :

SM-CL : MH+ = 311,26 ; t.r. = 10,16 min.

### Exemple 29 : 5-{benzyl[2-(diméthylamino)éthyl]amino}-2-méthyl-1,3-benzothiazole-4,7-dione :

RMN ¹H (DMSO d6, 400 MHz, δ) : 7,37-7,28 (m, 5H, H arom.) ; 5,61 (s, 1H, CH) ; 4,57 (s, 2H, CH₂); 3,71-3,68 (m, 2H, CH₂); 2,75 (s, 3H, CH₃) ; 2,39-2,37 (m, 2H, CH₂) ; 1,95 (s, 6H, 2 CH₃).
SM-CL : MH+ = 365,10 ; t.r. = 7,70 min.

### Exemple 30 : méthyl{3-[(2-méthyl-4,7-dioxo-4,7-dihydro-1,3-benzothiazol-5-yl)amino]propyl}carbamate de tert-butyle :

RMN ¹H (DMSO d6, 400 MHz, δ) : 7,75 (m, 1H, NH); 5,45 (s, 1H, CH); 3,22-3,18 (m, 2H, CH₂); 3,15-3,12 (m, 2H, CH₂); 2,76 (m, 3H, CH₃) ; 2,75 (s, 3H, CH₃) ; 1,78-1,75 (m, 2H, CH₂) ; 1,35 (m, 9H, 3 CH₃).
SM-CL : MH+ = 366,15 ; t.r. = 9,61 min.

### Exemple 31 : 3-[(2-méthyl-4,7-dioxo-4,7-dihydro-1,3-benzothiazol-5-yl)amino]propylcarbamate de tert-butyle :

SM-CL : MH+ = 352,22 ; t.r. = 9,09 min.

### Exemple 32 : chlorhydrate de 2-méthyl-5-{[3-(méthylamino)propyl]amino}-1,3-benzothiazole-4,7-dione :

25 mg (68,5 µmol) du composé de l'exemple 30 sont mis en suspension dans 10 ml de diéthyléther. 4 ml d'une solution molaire d'acide chlorhydrique dans de l'éther sont ajoutés puis le mélange réactionnel est agité à température ambiante pendant 2 heures. Le précipité résultant est recueilli par filtration, lavé avec de l'éther puis séché sous pression réduite pour donner un solide brun-rouge. RMN ¹H (DMSO d6, 400 MHz, δ) : 8,61 (m, 2H, NH₂⁺) ; 7,84-7,81 (m, 1H, NH) ; 5,55 (s, 1H, CH) ; 3,29-3,24 (m, 2H, CH₂) ; 2,91-2,88 (m, 2H, CH₂); 2,75 (s, 3H, CH₃) ; 2,53-2,52 (m, 3H, CH₃) ; 1,89-1,86 (m, 2H, CH₂). SM-CL : MH+ = 266,06 ; t.r. = 7,04 min.

### Exemple 33 : 5-[(3-aminopropyl)amino]-2-méthyl-1,3-benzothiazole-4,7-dione :

20 mg (57 µmol) du composé de l'exemple 30 sont mis en suspension dans 10 ml de diéthyléther. 840 µl d'une solution molaire d'acide chlorhydrique dans de l'éther sont ajoutés puis le mélange réactionnel est agité à température ambiante pendant 2 heures. Le précipité résultant est recueilli par filtration, lavé avec de l'éther puis séché sous pression réduite pour donner un solide brun-rouge.
RMN ¹H (DMSO d6, 400 MHz, δ) : 7,84-7,78 (m, 3H, NH, NH₂) ; 5,56 (s, 1H, CH) ; 3,28-3,23 (m, 2H, CH₂); 2,86-2,81 (m, 2H, CH₂); 2,75 (s, 3H, CH₃) ; 1,85-1,82 (m, 2H, CH₂).
SM-CL : MH+ = 280,15 ; t.r. = 7,01 min.

### Exemple 34 : 6-chloro-5-{[2-(diméthylamino)éthyl]amino}-2-méthyl-1,3-benzothiazole-4,7-dione :

58,6 mg (0,22 mmol) d'intermédiaire 2.1 sont mis en solution dans 5 ml d'acide acétique. 32,5 mg (0,24 mmol ; 1,1 éq.) de *N*-chlorosuccinimide sont ajoutés et le mélange réactionnel est agité pendant 3 heures à température ambiante. Après concentration, le résidu est purifié par chromatographie sur colonne de silice

(éluant : dichlorométhane/méthanol 90/10) et le produit attendu est obtenu, après reprise dans de l'éther éthylique, sous forme d'une poudre violette. RMN ¹H (DMSO d6, 400 MHz, δ) : 7,31 (m, 1H, NH) ; 3,79-3,74 (m, 2H, CH₂) 2,75 (s, 3H, CH₃) ; 2,47-2,44 (m, 2H, CH₂) ; 2,13 (s, 6H, 2 CH₃).
SM-CL : MH+= 300,09 ; t.r. = 7,17 min.

### Exemple 35 : 6-bromo-5-{[2-(diméthylamino)éthyl]amino}-2-méthyl-1,3-benzothiazole-4,7-dione :

102 mg (0,38 mmol) d'intermédiaire 2.1 sont mis en solution dans 10 ml d'acide acétique. 77,3 mg (0,43 mmol ; 1,1 éq.) de *N*-bromosuccinimide sont ajoutés et le mélange réactionnel est agité pendant 3 heures à température ambiante. Après concentration sous pression réduite, le résidu est purifié par chromatographie sur colonne de silice (éluant : dichlorométhane/méthanol 90/10) et le produit attendu est obtenu, après reprise dans de l'éther éthylique, sous forme d'une poudre violette.
RMN ¹H (DMSO d6, 400 MHz, δ) : 7,24 (m, 1H, NH) ; 3,78-3,74 (m, 2H, CH₂) ; 2,75 (s, 3H, CH₃) ; 2,45-2,42 (m, 2H, CH₂) 2,11 (s, 6H, 2 CH₃).
SM-CL : MH+ = 343,97 ; t.r. = 7,22 min.

### Exemple 36 : 6-(butylthio)-5-{[2-(diméthylamino)éthyl]amino}-2-méthyl-1,3-benzothiazole-4,7-dione :

À 33 mg (96 µmol) du composé de l'exemple 35 en solution dans 4 ml d'éthanol anhydre sont ajoutés 20 µl (0,115 mmol ; 1,2 éq.) de diisopropyléthylamine et 16 µl (0,154 mmol ; 1,6 éq.) de butanethiol. Le mélange réactionnel est maintenu sous agitation pendant 24 heures à 60 °C, puis après concentration sous pression réduite, le résidu est purifié par chromatographie sur colonne de silice (éluant : dichlorométhane/méthanol 95/5) et le produit attendu est obtenu, après reprise dans de l'éther éthylique, sous forme d'une poudre violette.
RMN ¹H (DMSO d6, 400 MHz, δ) : 7,56 (m, 1H, NH) ; 3,84-3,83 (m, 2H, CH₂) ; 2,75 (s, 3H, CH₃) ; 2,64-2,60 (t, 2H, CH₂); 2,45-2,42 (m, 2H, CH₂); 2,20 (s, 6H, 2 CH₃) ; 1,44-1,46 (m, 2H, CH₂); 1,37-1,33 (m, 2H, CH₂); 0,85-0,82 (t, 3H, CH₃).

### Exemple 37 : 5-{[2-(diméthylamino)éthyl]amino}-2-(morpholin-4-ylméthyl)-1,3-benzothiazole-4,7-dione :

### 37.1) 2-(bromométhyl)-5-méthoxy-1,3-benzothiazole :

2,58 g (14,5 mmol ; 1,3 éq.) de *N*-bromosuccinimide et une pointe de spatule d'aza-bis-isobutyronitrile sont ajoutés à 2 g (11,16 mmol) de 2-méthyl-5-méthoxy-1,3-benzothiazole en solution dans 25 ml de tétrachlorure de carbone anhydre. Le mélange réactionnel est chauffé au reflux sous irradiation pendant 6 heures, avec ajout d'une pointe de spatule d'aza-bis-isobutyronitrile toutes les 2 heures. Après retour à température ambiante, l'insoluble formé est filtré, le solvant est évaporé sous pression réduite et le résidu purifié par chromatographie sur colonne de silice (éluant : acétate d'éthyle/heptane 1/4). Le produit attendu est obtenu sous forme d'un solide blanc.
RMN ¹H (DMSO d6, 400 MHz, δ) : 7,98-7,96 (m, 1H, H arom.) ; 7,54-7,53 (m, 1H, H arom.) ; 7,13-7,10 (m, 1H, H arom.) ; 5,09 (s, 2H, CH₂) ; 3,84 (s, 3H, CH₃).
SM-CL : MH+ = 258,38 ; t.r. = 10,36 min.

### 37.2) 5-méthoxy-2-(morpholin-4-ylméthyl)-1,3-benzothiazole :

678 µl de diisopropyléthylamine (3,9 mmol ; 2 éq.) sont ajoutés à 0,5 g de l'intermédiaire 37.1 en solution dans 20 ml de toluène anhydre. 187 µl (2,14 mmol ; 1,1 éq.) de morpholine et une pointe de spatule d'iodure de sodium sont ajoutés à la solution précédente, puis le mélange réactionnel est maintenu sous agitation à 80 °C pendant 3 heures. Après refroidissement, le milieu réactionnel est lavé avec de l'eau (3 fois 20 ml), puis la phase organique est séchée sur sulfate de magnésium et concentrée. Une purification par chromatographie sur colonne de silice (éluant : acétate d'éthyle/heptane 1/1) permet d'obtenir le produit attendu sous forme d'un solide beige.
RMN ¹H (DMSO d6, 400 MHz, δ) : 7,91-7,89 (m, 1H, H arom.) ; 7,47-7,46 (m, 1H, H arom.) ; 7,05-7,02 (m, 1H, H arom.) ; 3,92 (s, 2H, CH₂) ; 3,82 (s, 3H, CH₃) ; 3,63-3,61 (m, 4H, 2CH₂) ; 2,56-2,53 (m, 4H, 2CH₂).
SM-CL : MH+ = 265,10 ; t.r. = 7,55 min.

### 37.3) 5-méthoxy-2-(morpholin-4-ylméthyl)-4-nitro-1,3-benzothiazole :

84 mg (0,83 mmol ; 1,2 éq.) de nitrate de potassium sont ajoutés par portions à une solution à 0 °C de 0,2 g (0,76 mmol) d'intermédiaire 37.2 dans 0,7 ml d'acide sulfurique concentré. Après retour à température ambiante, le mélange réactionnel est agité pendant 18 heures, neutralisé par addition d'une solution aqueuse de soude 10*M* puis extrait par 3 fois 50 ml de dichlorométhane. La phase organique résultante est séchée sur sulfate de magnésium et concentrée, puis purifiée par chromatographie sur colonne de silice (éluant : acétate d'éthyle / heptane 1/1). Le produit attendu est obtenu sous forme d'une huile jaune.
RMN ¹H (DMSO d6, 400 MHz, δ) : 8,26-8,24 (m, 1H, H arom.) ; 7,48-7,46 (m, 1H, H arom.) ; 3,98-3,96 (2s, 5H, CH₃, CH₂); 3,63-3,61 (m, 4H, 2CH₂) ; 2,59-2,56 (m, 4H, 2 CH₂).
SM-CL : MH+ = 310,11 ; t.r. = 8,03 min.

### 37.4) 5-méthoxy-2-(morpholin-4-ylméthyl)-1,3-benzothiazol-4-amine :

0,93 g (4,11 mmol ; 5 éq.) de chlorure d'étain sont ajoutés à une solution de 0,254 g (0,822 mmol) d'intermédiaire 37.3 dans 7 ml d'acide chlorhydrique concentré. Le mélange réactionnel est maintenu sous agitation pendant 3 heures à 70 °C. Après retour à température ambiante, le milieu est dilué par addition de 20 ml d'acétate d'éthyle, puis neutralisé par une solution saturée en NaHCO₃ et enfin lavé par 3 fois 20 ml d'eau. Les phases organiques sont regroupées, séchées sur sulfate de magnésium et concentrées pour fournir le produit attendu sous forme d'une poudre beige.
RMN ¹H (DMSO d6, 400 MHz, δ) : 7,12-7,10 (m, 1H, H arom.) ; 7,02-7,00 (m, 1H, H arom.); 5,04 (s, 2H, NH₂); 3,88 (s, 2H, CH₂); 3,81 (s, 3H, CH₃) ; 3,63-3,60 (m, 4H, 2 CH₂); 2,55-2,52 (m, 4H, 2CH₂).
SM-CL : MH+ = 280,11 ; t.r. = 7,29 min.

### 37.5) 5-méthoxy-2-(morpholin-4-ylméthyl)-1,3-benzothiazole-4,7-dione :

Une solution de 84 mg (0,31 mmol ; 1,8 éq.) de sel de Frémy, dissous dans 14 ml d'une solution tampon (0,3*M*) d'hydrogénophosphate de sodium, est ajoutée à 0,0483 mg (0,17 mmol) d'intermédiaire 37.4 en solution dans 10 ml d'acétone. Le mélange réactionnel est agité pendant 18 heures à température ambiante, puis extrait par 3 fois 30 ml de dichlorométhane, les phases organiques étant ensuite lavées avec 2 fois 20 ml d'eau. Les phases organiques sont ensuite regroupées, séchées sur sulfate de magnésium puis concentrées sous pression réduite. Le résidu est purifié par chromatographie sur colonne de silice (éluant : acétate d'éthyle / heptane 1/1) et le produit attendu est obtenu sous forme d'une huile jaune.
SM-CL : MH+ = 295,06 ; t.r. = 7,11 min.

### 37.6) 5-{[2-(diméthylamino)éthyl]amino}-2-(morpholin-4-ylméthyl)-1,3-benzothiazole-4,7-dione :

Le protocole expérimental utilisé est identique à celui décrit pour l'exemple 1, l'intermédiaire 37.5 remplaçant le 5-méthoxy-2-méthyl-4,7-dioxobenzothiazole.
SM-CL : MH+ = 351,38 ; t.r. = 3,07 min.

### Exemple 38 : 5-{[2-(diméthylamino)éthyl]amino}-2-[(4-phénylpipérazin-1-yl)méthyl]-1,3-benzothiazole-4,7-dione :

Le protocole expérimental utilisé est identique à celui décrit pour l'exemple 37, la *N*-phénylpipérazine remplaçant la morpholine dans la deuxième étape.
SM-CL : MH+ = 426,18 ; t.r. = 7,39 min.

### Exemple 39 : 5-{[2-(diméthylamino)éthyl]amino}-2-(pipéridin-1-ylméthyl)-1,3-benzothiazole-4,7-dione :

Le protocole expérimental utilisé est identique à celui décrit pour l'exemple 37, la pipéridine remplaçant la morpholine dans la deuxième étape.
SM-CL : MH+ = 349,13 ; t.r. = 2,82 min.

### Les composés des exemples 40 à 52 sont obtenus de façon analogue à celle décrite pour l'exemple 15, les amines primaires ou secondaires adéquates remplaçant l'aniline dans la quatrième et dernière étape.

### Exemple 40 : 5-{[2-(diméthylamino)éthyl]amino}-2-éthyl-1,3-benzoxazole-4,7-dione ou 6-{[2-(diméthylamino)éthyl]amino}-2-éthyl-1,3-benzoxazole-4,7-dione :

### Point de fusion : 123 °C.

RMN ¹H (DMSO d6, 400 MHz, δ) : 7,39 (t, 1H, NH); 5,30 (s, 1H, CH); 3,30-3,31 (m, 2H, CH₂); 3,24-3,20 (m, 2H, CH₂); 2,95-2,88 (q, 2H, CH₂); 2,17 (s, 6H, 2 CH₃) ; 1,30 (t, 3H, CH₃).
SM-CL : MH+ = 264,13 ; t.r. = 7,02 min.

### Exemple 41 : 2-[(2-éthyl-4,7-dioxo-4,7-dihydro-1,3-benzoxazol-5-yl)(méthyl)amino]éthylcarbamate de tert-butyle ou 2-[(2-éthyl-4,7-dioxo-4,7-dihydro-1,3-benzoxazol-6-yl)(méthyl)amino]éthylcarbamate de tert-butyle :

### Point de fusion: 135°C.

RMN ¹H (DMSO d6, 400 MHz, δ) : 7,82 (t, 1H, NH); 5,36 (s, 1H, CH); 3,38-3,36 (m, 2H, CH₂); 3,30-3,27 (m, 2H, CH₂); 2,93-2,88 (q, 2H, CH₂); 2,79 (s, 3H, CH₃) ; 1,37-1,26 (m, 12H, 4 CH₃).
SM-CL : MH+ = 350,14 ; t.r. = 9,72 min.

### Exemple 42 : 2-[(2-éthyl-4,7-dioxo-4,7-dihydro-1,3-benzoxazol-5-yl)amino]éthylcarbamate de tert-butyle ou 2-[(2-éthyl-4,7-dioxo-4,7-dihydro-1,3-benzoxazol-6-yl)amino]éthylcarbamate de tert-butyle :

### Point de fusion: 173 °C.

RMN ¹H (DMSO d6, 400 MHz, δ) : 7,73 (t, 1H, NH); 6,97 (t, 1H" NH); 5,36 (s, 1H, CH); 3,20-3,17 (m, 2H, CH₂); 3,15-3,12 (m, 2H, CH₂); 2,93-2,88 (q, 2H, CH₂) ; 1,36 (s, 9H, 3CH₃) ; 1,28 (t, 3H, CH₃).
SM-CL : MH+ = 336,23 ; t.r. = 9,24 min.

### Exemple 43 : 5-{[3-(diméthylamino)propyl]amino}-2-éthyl-1,3-benzoxazole-4,7-dione ou 6-{[3-(diméthylamino)propyl]amino}-2-éthyl-1,3-benzoxazole-4,7-dione :

### Point de fusion : 101 °C.

RMN ¹H (DMSO d6, 400 MHz, δ) : 8,09 (t, 1H, NH) ; 5,28 (s, 1H, CH) ; 3,21-3,16 (m, 2H, CH₂); 2,93-2,88 (q, 2H, CH₂) ; 2,28-2,25 (m, 2H, CH₂) ; 2,13 (s, 6H, 2 CH₃) ; 1,71-1,67 (m, 2H, CH₂); 1,28 (t, 3H, CH₃).
SM-CL : MH+ = 278,19 ; t.r. = 7,09 min.

### Exemple 44 : 2-éthyl-5-{[2-(1-méthylpyrrolidin-2-yl)éthyl]amino}-1,3-benzoxazole-4,7-dione ou 2-éthyl-6-{[2-(1-méthylpyrrolidin-2-yl)éthyl]amino}-1,3-benzoxazole-4,7-dione :

### Point de fusion: 121 °C.

RMN ¹H (DMSO d6, 400 MHz, δ) : 8,11 (t, 1H, NH); 5,24 (s, 1H, CH); 3,19-3,17 (m, 2H, CH₂); 2,95-2,93 (m, 1H, CH); 2,92-2,87 (q, 2H, CH₂); 2,21 (s, 3H, CH₃) ; 2,16-2,05 (m, 2H, CH₂); 1,88-1,84 (m, 2H, CH₂); 1,63-1,57 (m, 4H, 2 CH₂) ; 1,28 (t, 3H, CH₃).
SM-CL : MH+ = 304,20 ; t.r. = 7,20 min.

### Exemple 45 : 5-{[4-(diméthylamino)butyl]amino}-2-éthyl-1,3-benzoxazole-4,7-dione ou 6-{[4-(diméthylamino)butyl]amino}-2-éthyl-1,3-benzoxazole-4,7-dione :

RMN ¹H (DMSO d6, 400 MHz, δ) : 8,06 (t, 1H, NH); 5,28 (s, 1H, CH); 3,17-3,12 (m, 2H, CH₂); 2,93-2,88 (q, 2H, CH₂); 2,22-2,19 (m, 2H, CH₂); 2,11 (s, 6H, 2CH₃) ; 1,61-1,56 (m, 2H, CH₂) ; 1,46-1,42 (m, 2H, CH₂) ; 1,28 (t, 3H, CH₃).
SM-CL : MH+ = 292,20 ; t.r. = 7,10 min.

### Exemple 46 : 2-éthyl-5-[(4-pyrrolidin-1-ylbutyl)amino]-1,3-benzoxazole-4,7-dione ou 2-éthyl-6-[(4-pyrrolidin-1-ylbutyl)amino]-1,3-benzoxazole-4,7-dione :

### Point de fusion: 102°C.

RMN ¹H (DMSO d6, 400 MHz, δ) : 7,95 (t, 1H, NH); 5,28 (s, 1H, CH); 3,17-3,13 (m, 2H, CH₂) ; 2,93-2,87 (q, 2H, CH₂); 2,41-2,37 (m, 6H, 3CH₂); 1,63-1,58 (m, 2H, CH₂) ; 1,49-1,45 (m, 2H, CH₂) ; 1,28 (t, 3H, CH₃).
SM-CL : MH+ = 318,20 ; t.r. = 7,30 min.

### Exemple 47 : 5-{[5-(diméthylamino)pentyl]amino}-2-éthyl-1,3-benzoxazole-4,7-dione ou 6-{[5-(diméthylamino)pentyl]amino}-2-éthyl-1,3-benzoxazole-4,7-dione :

RMN ¹H (DMSO d6, 400 MHz, δ) : 7,83 (t, 1H, NH) ; 5,27 (s, 1H, CH) ; 3,17-3,13 (m, 2H, CH₂) ; 2,93-2,87 (q, 2H, CH₂) ; 2,18-2,14 (m, 2H, CH₂) ; 2,09 (s, 6H, 2CH₃) ; 1,58-1,54 (m, 2H, CH₂) ; 1,41-1,38 (m, 2H, CH₂) ; 1,28 (t, 3H, CH₃).
SM-CL : MH+ = 306,20 ; t.r. = 7,30 min.

### Exemple 48 : mélange de 5-{[6-(diméthylamino)hexyl]amino}-2-éthyl-1,3-benzoxazole-4,7-dione et 6-{[6-(diméthylamino)hexyl]amino}-2-éthyl-1,3-benzoxazole-4,7-dione :

SM-CL : MH+ = 320,20 ; t.r. = 7,50 min.

### Exemple 49 : mélange de 2-éthyl-5-(4-méthylpipérazin-1-yl)-1,3-benzoxazole-4,7-dione et 2-éthyl-6-(4-méthylpipérazin-1-yl)-1,3-benzoxazole-4,7-dione :

SM-CL : MH+ = 276,10 ; t.r. = 7,10 min.

### Exemple 50 : mélange de 2-éthyl-5-[(1-éthylhexyl)amino]-1,3-benzoxazole-4,7-dione et 2-éthyl-6-[(1-éthylhexyl)amino]-1,3-benzoxazole-4,7-dione :

SM-CL : MH+ = 305,20 ; t.r. = 11,50 min.

### Exemple 51 : mélange de 5-azocan-1-yl-2-éthyl-1,3-benzoxazole-4,7-dione et 6-azocan-1-yl-2-éthyl-1,3-benzoxazole-4,7-dione :

SM-CL : MH+ = 289,20 ; t.r. = 10,40 min.

### Exemple 52 : mélange de 2-éthyl-5-morpholin-4-yl-1,3-benzoxazole-4,7-dione et 2-éthyl-6-morpholin-4-yl-1,3-benzoxazole-4,7-dione :

SM-CL : MH+ = 263,10 ; t.r. = 8,60 min.

### Exemple 53 : 6-chloro-5-{[2-(diméthylamino)éthyl]amino}-2-éthyl-1,3-benzoxazole-4,7-dione ou 5-chloro-6-{[2-(diméthylamino)éthyl]amino}-2-éthyl-1,3-benzoxazole-4,7-dione :

Le protocole expérimental utilisé est identique à celui décrit pour l'exemple 34, le composé de l'exemple 40 remplaçant l'intermédiaire 2.1. Point de fusion :110°C. RMN ¹H (DMSO d6, 400 MHz, δ) : 7,35 (t, 1H, NH) ; 3,78-3,74 (m, 2H, CH₂) ; 2,94-2,89 (q, 2H, CH₂); 2,48-2,45 (m, 2H, CH₂); 2,15 (s, 6H, 2CH₃); 1,28 (t, 3H, CH₃).
SM-CL : MH+ = 298,10 ; t.r. = 7,20 min.

### Exemple 54 : 6-bromo-5-{[2-(diméthylamino)éthyl]amino}-2-éthyl-1,3-benzoxazole-4,7-dione ou 5-bromo-6-{[2-(diméthylamino)éthyl]amino}-2-éthyl-1,3-benzoxazole-4,7-dione :

Le protocole expérimental utilisé est identique à celui décrit pour l'exemple 35, le composé de l'exemple 40 remplaçant l'intermédiaire 2.1.
RMN ¹H (DMSO d6, 400 MHz, δ) : 7,27 (t, 1H, NH) ; 3,78-3,74 (m, 2H, CH₂) ; 2,94-2,89 (q, 2H, CH₂) ; 2,46-2,43 (m, 2H, CH₂); 2,13 (s, 6H, 2 CH₃) ; 1,26 (t, 3H, CH₃).
SM-CL : MH+ = 342,00 ; t.r. = 7,30 min.

### Exemple 55 : 5-{[2-(diméthylamino)éthyl]amino}-2-éthyl-6-méthyl-1,3-benzoxazole-4,7-dione :

### 55.1) 2-diazo-5-méthylcyclohexane-1,3-dione :

À une solution de 5 g (39,6 mmol) de 5-méthylcyclohexane-1,3-dione dans 100 ml de dichlorométhane, on ajoute 12,25 ml (87,2 mmol ; 2,2 éq.) de triéthylamine et 8,57 g (35,67 mmol ; 0,9 éq.) de 4-acétamidobenzenesulfonylazide. Le mélange réactionnel est agité pendant 75 min à température ambiante, puis refroidi à 0 °C et filtré sur un lit de silice. Après concentration sous pression réduite, la solution est lavée avec 3 fois 50 ml d'eau. Les phases organiques sont regroupées, séchées sur sulfate de sodium et concentrées. Le solide résultant est repris dans de l'éther éthylique puis filtré et séché sous pression réduite. Il est utilisé dans l'étape suivante sans autre purification.
SM-CL : MH+ = 153,49 ; t.r. = 7,21 min.

### 55.2) 2-éthyl-6-méthyl-6,7-dihydro-1,3-benzoxazol-4(5H)-one

A une solution de 4,9 g (32,2 mmol) d'intermédiaire 55.1 dans 50 ml de propionitrile, on ajoute 285 mg (0,644 mmol ; 0,02 éq.) d'acétate de rhodium. Le mélange réactionnel est maintenu sous agitation sous atmosphère inerte d'argon à 60 °C pendant 2 heures. Le solvant est ensuite évaporé et le résidu est purifié par chromatographie sur colonne de silice (éluant : acétate d'éthyle/heptane 1/1). Le produit attendu est obtenu sous forme d'une huile jaune.
RMN ¹H (DMSO d6, 400 MHz, δ) : 3,02-2,97 (m, 1H, CH) ; 2,80-2,74 (q, 2H, CH₂) ; 2,68-2,61 (m, 1H, CH₂); 2,44-2,39 (m, 2H, CH₂) ; 2,34-2,30 (m, 1H, CH₂); 1,23 (t, 3H, CH₃) ; 1,08 (s, 3H, CH₃).
SM-CL : MH+ = 180,25 ; t.r. = 8,55 min.

### 55.3) oxime de (4E)-2-éthyl-6-méthyl-6,7-dihydro-1,3-benzoxazol-4(5H)-one:

A une solution de 1,39 g (7,76 mmol) d'intermédiaire 55.2 dans 200 ml de méthanol, on ajoute 647 mg (9,31 mmol ; 1,2 éq.) de chlorhydrate d'hydroxylamine et 764 mg (9,31 mmol ; 1,2 éq.) d'acétate d'ammonium. Le mélange réactionnel est agité pendant 90 min au reflux du méthanol, puis le solvant est évaporé, le résidu est repris dans 50 ml d'eau puis neutralisé à l'aide d'une solution saturée en NaHCO₃. Le produit attendu est extrait par 2 fois avec 50 ml d'acétate d'éthyle puis lavé par 2 fois avec 30 ml d'eau. Les phases organiques sont regroupées, séchées sur sulfate de sodium et concentrées sous pression réduite. Le produit souhaité est obtenu sous forme d'un solide jaune foncé, utilisé sans autre purification dans l'étape suivante.
SM-CL : MH+ = 195,09 ; t.r. = 8,73 min.

### 55.4) 2-éthyl-6-méthyl-1,3-benzoxazol-4-amine :

1,45 g (7,46 mmol) d'intermédiaire 55.3 sont dissous dans 25 g d'acide polyphosphorique. Après 1 heure d'agitation à 140 °C, la solution est hydrolysée par addition d'eau glacée, puis neutralisée par une solution aqueuse de soude à 50%. Le produit obtenu est extrait avec du dichlorométhane, et la phase organique est lavée par 3 fois avec 25 ml d'eau, séchée sur sulfate de sodium et concentrée sous pression réduite. Le produit souhaité est obtenu après purification par chromatographie sur colonne de silice (éluant : dichlorométhane/éthanol 98/2).
SM-CL : MH+ = 177,21 ; t.r. = 9,12 min.

### 55.5) 2-éthyl-6-méthyl-1,3-benzoxazole-4,7-dione :

Le protocole expérimental utilisé est identique à celui décrit pour l'étape 15.3 de l'exemple 15, l'intermédiaire 55.4 remplaçant l'intermédiaire 15.2.
RMN ¹H (DMSO d6, 400 MHz, δ) : 6,72 (s, 1H, CH) ; 2,98-2,93 (q, 2H, CH₂); 2,04 (s, 3H, CH₃) ; 1,30 (t, 3H, CH₃).
SM-CL : MH+ = 192,06 ; t.r. = 8,93 min.

### 55.6) 5-{[2-(diméthylamino)éthyl]amino}-2-éthyl-6-méthyl-1,3-benzoxazole-4,7-dione :

Le protocole expérimental utilisé est identique à celui décrit pour l'étape 15.4 de l'exemple 15, l'intermédiaire 55.5 remplaçant l'intermédiaire 15.3 et la *N*,*N*-diméthyléthylènediamine remplaçant l'aniline. Point de fusion : 135 °C.
RMN ¹H (DMSO d6, 400 MHz, δ) : 6,63 (t, 1H, NH) ; 3,62-3,58 (m, 2H, CH₂) ; 2,92-2,86 (q, 2H, CH₂) ; 2,44-2,41 (m, 2H, CH₂); 2,14 (s, 6H, 2 CH₃) ; 1,97 (s, 3H, CH₃) ; 1,27 (t, 3H, CH₃).
SM-CL : MH+ = 278,12 ; t.r. = 7,27 min.

### Exemple 56: 2-cyclopropyl-5-{[2-(diméthylamino)éthyl]amino}-1,3-benzoxazole-4,7-dione ou 2-cyclopropyl-6-{[2-(diméthylamino)éthyl]amino}-1,3-benzoxazole-4,7-dione :

Le protocole expérimental utilisé est identique à celui décrit pour l'exemple 55, la cyclohexane-1,3-dione remplaçant la 5-méthylcyclohexane-1,3-dione dans la première étape et le cyclopropanecarbonitrile remplaçant le propionitrile dans la deuxième étape.
Point de fusion: 155 °C.
RMN ¹H (DMSO d6, 400 MHz, δ) : 7,35 (t, 1H, NH); 5,27 (s, 1H, CH); 3,30-3,18 (m, 2H, CH₂); 2,49-2,46 (m, 2H, CH₂); 2,28-2,25 (m, 1H, CH); 2,17 (s, 6H, 2 CH₃) ; 1,18-1,07 (m, 4H, 2 CH₂).
SM-CL : MH+ = 276,10 ; t.r. = 7,10 min.

### Exemple 57 : mélange de 5-{[2-(diméthylamino)éthyl]amino}-2-phényl-1,3-benzoxazole-4,7-dione et 6-{[2-(diméthylamino)éthyl]amino}-2-phényl-1,3-benzoxazole-4,7-dione :

Le protocole expérimental utilisé est identique à celui décrit pour l'exemple 15, l'orthobenzoate de triméthyle remplaçant l'orthopropionate de triéthyle dans la première étape et la *N,N*-diméthyléthylènediamine remplaçant l'aniline dans la quatrième et dernière étape. Point de fusion : 147 °C.
RMN ¹H (DMSO d6, 400 MHz, δ): 8,15-8,08 (m, 2H, H arom.); 7,70-7,61 (m, 3H, H arom.); 7,33 (t, 1H, NH); 5,38 (s, 1H, CH); 3,26-3,21 (m, 4H, 2 CH₂) ; 2,19 (s, 6H, 2 CH₃).
Les deux composants du mélange peuvent être caractérisés par les déplacements RMN (400 MHz) du seul proton du cycle benzoxazoledione qui sont 5,38 et 5,39 ppm. SM-CL : MH+ = 312,20 ; t.r. = 7,70 min.

### Exemple 58 : mélange de 5-{[6-(diméthylamino)hexyl]amino}-2-phényl-1,3-benzoxazole-4,7-dione et 6-{[6-(diméthylamino)hexyl]amino}-2-phényl-1,3-benzoxazole-4,7-dione :

Le protocole expérimental utilisé est identique à celui décrit pour l'exemple 15, l'orthobenzoate de triméthyle remplaçant l'orthopropionate de triéthyle dans la première étape et la 6-(diméthylamino)hexylamine remplaçant l'aniline dans la quatrième et dernière étape.

Les deux composants du mélange peuvent être caractérisés par les déplacements RMN (400 MHz) du seul proton du cycle benzoxazoledione qui sont 5,34 et 5,35 ppm. SM-CL : MH+ = 368,20 ; t.r. = 8,10 min.

### Exemple 59 : 5-[(1-éthylhexyl)amino]-2-phényl-1,3-benzoxazole-4,7-dione ou 6-[(1-éthylhexyl)amino]-2-phényl-1,3-benzoxazole-4,7-dione :

Le protocole expérimental utilisé est identique à celui décrit pour l'exemple 15, l'orthobenzoate de triméthyle remplaçant l'orthopropionate de triéthyle dans la première étape et la 2-éthylhexylamine remplaçant l'aniline dans la quatrième et dernière étape. SM-CL : MH+ = 353,20 ; t.r. = 12,50 min.

### Exemple 60 : mélange de 2-(2,6-difluorophényl)-5-[(2-pyrrolidin-1-yléthyl)amino]-1,3-benzoxazole-4,7-dione et 2-(2,6-difluorophényl)-6-[(2-pyrrolidin-1-yléthyl)amino]-1,3-benzoxazole-4,7-dione :

### 60.1) 2-(2,6-difluorophényl)-4-nitro-1,3-benzoxazole :

À une solution de 5 g (32,4 mmol) de 2-amino-3-nitrophénol et de 5,12 g (32,4 mmol ; 1 éq.) d'acide 2,6-difluorobenzoïque dans 50 ml de xylène, on ajoute 2 g (32,4 mmol ; 1 éq.) d'acide borique. Le mélange est chauffé au reflux du xylène pendant 8 heures avec élimination de l'eau formée par un Dean-Stark. Après retour à température ambiante, le milieu réactionnel est dilué par 100 ml d'acétate d'éthyle et neutralisé par une solution aqueuse de soude à 10%. La phase organique est lavée par 3 fois avec 50 ml d'eau puis avec une solution saturée en NaCl avant d'être séchée sur sulfate de sodium, filtrée et concentrée sous pression réduite. Le 2-(2,6-difluorophényl)-4-nitro-1,3-benzoxazole est utilisé sans autre purification dans l'étape suivante.
SM-CL : MH+ = 277,00 ; t.r. = 10,45 min.

### 60.2) 2-(2,6-difluorophényl)-1, 3-benzoxazol-4-amine :

À une solution de 3,5 g (12,7 mmol) de 2-(2,6-difluorophényl)-4-nitro-1,3-benzoxazole dans 60 ml d'acide chlorhydrique concentré, on ajoute 14,3 g (63,5 mmol ; 5 éq.) de chlorure d'étain. Le mélange est agité pendant 2 heures à 60 °C, puis, après retour à température ambiante et addition de 100 ml d'eau, est neutralisé par une solution aqueuse de soude à 50%. Le précipité formé est filtré sur lit de Célite et lavé avec de l'éthanol. La solution résultante est concentrée sous pression réduite, puis le produit souhaité est extrait par 3 fois avec 50 ml d'acétate d'éthyle. Les phases organiques sont regroupées, lavées par 2 fois avec 30 ml d'une solution saturée en chlorure de sodium, séchées sur sulfate de sodium et concentrées sous pression réduite. La 2-(2,6-difluorophényl)-1,3-benzoxazol-4-amine est utilisée sans autre purification dans l'étape suivante.
SM-CL : MH+ = 247,08 ; t.r. = 10,02 min.

### 60.3) 2-(2,6-difluorophényl)-1,3-benzoxazole-4,7-dione :

Le protocole expérimental utilisé est identique à celui décrit pour l'étape 15.3 de l'exemple 15, l'intermédiaire 60.2 remplaçant l'intermédiaire 15.2. Le produit attendu est obtenu sous forme de cristaux jaunes.
SM-CL : MH+ = 261,93 ; t.r. = 9,62 min.

### 60.4) mélange de 2-(2,6-difluorophényl)-5-[(2-pyrrolidin-l-yléthyl)amino]-1,3-benzoxazole-4,7-dione et 2-(2,6-difluorophényl)-6-[(2-pyrrolidin-1-yléthyl)amino]-1,3-benzoxazole-4,7-dione :

Le protocole expérimental utilisé est identique à celui décrit pour l'étape 15.4 de l'exemple 15, l'intermédiaire 60.3 remplaçant l'intermédiaire 15.3 et la (2-aminoéthyl)pyrrolidine remplaçant l'aniline. Point de fusion : 150 °C.
RMN ¹H (DMSO d6, 400 MHz, δ) : 7,78-7,76 (m, 1H, H arom.); 7,43-7,37 (m, 2H, H arom.); 5,41 (s, 1H, CH); 3,38-3,36 (m, 2H, CH₂) ; 3,28-3,26 (m, 4H, 2 CH₂) ; 2,68-2,64 (m, 2H, CH₂) ; 1,70-1,67 (m, 4H, 2 CH₂).
Les deux composants du mélange peuvent être caractérisés par les déplacements RMN (400 MHz) du seul proton du cycle benzoxazoledione qui sont 5,40 et 5,42 ppm. SM-CL : MH+ = 373,99 ; t.r. = 7,76 min.

### Les composés des exemples 61 à 65 sont obtenus de façon analogue à celle décrite pour l'exemple 60.

### Exemple 61 : mélange de 2-[4-(diéthylamino)phényl]-5-{[2-(diméthylamino)éthyl]amino}-1,3-benzoxazole-4,7-dione et 2-[4-(diéthylamino)phényl]-6-{[2-(diméthylamino)éthyl]amino}-1,3-benzoxazole-4,7-dione :

RMN ¹H (DMSO d6, 400 MHz, δ) : 7,91-7,89 (d, 2H, H arom.) ; 6,83-6,81 (d, 2H, H arom.) ; 5,29 (s, 1H, CH) ; 3,47-3,42 (m, 4H, 2 CH₂) ; 3,41-3,38 (m, 2H, CH₂) ; 3,25-3,21 (m, 2H, CH₂) ; 2,19 (s, 6H, 2 CH₃) ; 1,12 (t, 6H, 2 CH₃).
Les deux composants du mélange peuvent être caractérisés par les déplacements RMN (400 MHz) du seul proton du cycle benzoxazoledione qui sont 5,29 et 5,30 ppm. SM-CL : MH+ = 383,20 ; t.r. = 8,30 min.

### Exemple 62 : mélange de 2-[4-(diéthylamino)phényl]-5-[(2-pyrrolidin-1-yléthyl)amino]-1,3-benzoxazole-4,7-dione et 2-[4-(diéthylamino)phényl]-6-[(2-pyrrolidin-1-yléthyl)amino]-1,3-benzoxazole-4,7-dione :

RMN ¹H (DMSO d6, 400 MHz, δ) : 7,91-7,88 (d, 2H, H arom.); 6,83-6,81 (d, 2H, H arom.); 5,29 (s, 1H, CH); 3,47-3,42 (m, 4H, 2 CH₂); 3,37-3,35 (m, 2H, CH₂); 3,26-3,23 (m, 4H, 2 CH₂); 2,66 (t, 2H, CH₂); 1,70-1,68 (m, 4H, 2 CH₂) ; 1,14 (t, 6H, 2 CH₃). Les deux composants du mélange peuvent être caractérisés par les déplacements RMN (400 MHz) du seul proton du cycle benzoxazoledione qui sont 5,28 et 5,29 ppm. SM-CL : MH+ = 409,10 ; t.r. = 8,40 min.

### Exemple 63 : mélange de 2-(4-chlorophényl)-5-{[2-(diméthylamino)éthyl]amino}-1,3-benzoxazole-4,7-dione et 2-(4-chlorophényl)-6-{[2-(diméthylamino)éthyl]amino}-1,3-benzoxazole-4,7-dione :

### Point de fusion: 169°C.

Les deux composants du mélange peuvent être caractérisés par les déplacements RMN (400 MHz) du seul proton du cycle benzoxazoledione qui sont 5,39 et 5,41 ppm. SM-CL : MH+ = 346,20 ; t.r. = 8,10 min.

### Exemple 64 : mélange de 2-(4-chlorophényl)-5-{[3-(diméthylamino)propyl]amino}-1,3-benzoxazole-4,7-dione et 2-(4-chlorophényl)-6-{[3-(diméthylamino)propyl]amino}-1,3-benzoxazole-4,7-dione :

SM-CL : MH+ = 360,10 ; t.r. = 8,10 min.

### Exemple 65 : mélange de 2-(4-chlorophényl)-5-{[4-(diméthylamino)butyl]amino}-1,3-benzoxazole-4,7-dione et 2-(4-chlorophényl)-6-{[4-(diméthylamino)butyl]amino}-1,3-benzoxazole-4,7-dione :

RMN ¹H (DMSO d6, 400 MHz, δ) : 8,13-8,09 (m, 2H, H arom.) ; 7,70-7,67 (m, 2H, H arom.) ; 5,36 (s, 1H, CH) ; 3,18-3,15 (m, 2H, CH₂) 2,25-2,21 (m, 2H, CH₂) ; 2,13 (s, 6H, 2 CH₃) ; 1,62-1,58 (m, 2H, CH₂) ; 1,48-1,44 (m, 2H, CH₂).
Les deux composants du mélange peuvent être caractérisés par les déplacements RMN (400 MHz) du seul proton du cycle benzoxazoledione qui sont 5,35 et 5,37 ppm. SM-CL : MH+ = 374,10 ; t.r. = 8,20 min.

### Exemple 66 : mélange de 2-(2-fluorophényl)-5-{[2-(diméthylamino)éthyl]amino}-1,3-benzoxazole-4,7-dione et 2-(2-fluorophényl)-6-{[2-(diméthylamino)éthyl]amino}-1,3-benzoxazole-4,7-dione :

### 66.1) 2-diazocyclohexane-1,3-dione :

Un mélange de 4-acétamidobenzenesulfonylazide (25 g, 104 mmol) et de triéthylamine (36 ml, 250 mmol) dans du dichlorométhane maintenu à une température inférieure à 30°C par refroidissement externe est traité au goutte-à-goutte par une solution de cyclohexane-1,3-dione (13 g, 115 mmol) dans 200 ml de dichlorométhane. Le mélange réactionnel est agité pendant 75 min à température ambiante puis filtré sur Célite.

Après concentration à environ 300 ml, le filtrat est lavé à l'eau puis séché sur sulfate de sodium. Le solide jaune-brun (14 g ; 88%) obtenu par évaporation du solvant sous pression réduite est similaire à celui obtenu dans l'exemple 55.1, et est utilisé tel quel dans l'étape suivante.
RMN ¹H (DMSO-*d*₆, δ) : 1,93 (m, 2H) ; 2,50 (t, 4H).
RMN ¹³C (DMSO-*d*₆, δ): 18,20 ; 36,68 ; 190,96.

### 66.2) 2-(2-fluorophényl)-6,7-dihydro-1,3-benzoxazol-4(5H)-one :

Un mélange d'acétate de rhodium (32 mg, 72 µmol) et de 2-fluorobenzonitrile (2,31 ml ; 22 mmol) dans du perfluorobenzène (5 ml) est traité à 60 °C au goutte-à-goutte par une solution de diazocyclohexanedione (obtenue à l'étape 66.1 ; 1 g ; 7,24 mmol) dans 5 ml de perfluorobenzène. Le milieu réactionnel est maintenu à 60 °C jusqu'à épuisement du dégagement d'azote (1h ; CCM sur SiO₂ : 2% MeOH/CH₂Cl₂).
Après refroidissement à température ambiante et filtration, le solvant du filtrat est évaporé. Le résidu est purifié par chromatographie (SiO₂ : AcOEt/heptane : 1/1) pour donner une poudre jaune clair.
RMN ¹H (CDCl₃, δ) : 2,31 (m, 2H) ; 2,66 (m, 2H,) ; 3,09 (t, 2H) ; 7,19-7,28 (m, 2H) ; 7,48-7,50 (m, 1H) ; 8,15-8,19 (m, 1H).
SM-CL : MH+ = 232,08 ; t.r. = 9,28 min.

### 66.3) 5-bromo-2-(2-fluorophényl)-6,7-dihydro-1,3-benzoxazol-4(5H)-one :

Une solution de l'intermédiaire 66.2 (470 mg, 2 mmol) dans l'acide acétique (5 ml) est traitée avec du brome dans de l'acide acétique (0,2M; 10 ml ; 2 mmol) pendant 4 jours à température ambiante (CCM sur SiO₂ : AcOEt/heptane : 1/1). Le milieu réactionnel est ensuite dilué avec de l'eau et extrait à l'aide de dichlorométhane. Les phases organiques sont regroupées, lavées avec une solution saturée de bicarbonate puis avec une solution de disulfite de sodium à 5%. Après séchage sur sulfate de sodium et élimination des volatiles sous pression réduite, on obtient une huile jaune qui est purifiée par chromatographie (Si0₂ : AcOEt/heptane : 1/1) pour donner une poudre blanche.
RMN ¹H (DMSO-*d*₆, δ) : 2,49 (m, 2H) ; 2,73 (m, 1H,) ; 3,15 (m, 2H) ; 4,95 (t, 1H,) ; 7,39-7,48 (m, 2H) ; 7,63-7,67 (m, 1H) ; 8,03-8,08 (t, 1H).
SM-CL : MH+ = 309,93 ; t.r. = 10,08 min.

### 66.4) 2-(2-fluorophényl)-4-hydroxy-1,3-benzoxazole :

L'intermédiaire 66.3 (6,52 g ; 21 mmol) en solution dans du tétrahydrofuranne (100 ml) est traité au goutte-à-goutte par du diazabicyclo[5.4.0]undec-7-ène (4,7 ml ; 31 mmol).

Lorsque la réaction est complète (1,5 h ; CCM sur SiO₂ : AcOEt/heptane : 1/1), le mélange réactionnel est étendu avec de l'acétate d'éthyle puis lavé successivement avec de l'acide chlorhydrique 1*N* et une solution saturée de chlorure de sodium. Les phases organiques regroupées sont séchées et concentrées pour donner un résidu brun qui est purifié par chromatographie (SiO₂ : AcOEt/heptane : 1/1) pour donner une poudre beige.
RMN ¹H (DMSO-*d*₆, δ) : 6,80 (d, 1H) ; 7,19-7,26 (m, 2H); 7,41-7,49 (m, 2H); 7,65 (m, 1H) ; 8,18 (t, 1H) ; 10,43 (s, 1H).
SM-CL : MH+ = 230,07 ; t.r. = 10,03 min.

### 66.5) 2-(2-fluorophényl)-1,3-benzoxazole-4,7-dione :

Le protocole expérimental utilisé est identique à celui décrit pour l'étape 15.3 de l'exemple 15, l'intermédiaire 66.4 remplaçant l'intermédiaire 15.2. On obtient une poudre jaune.
RMN ¹H (DMSO-*d*₆, δ) : 6,94 (large, 2H); 7,45-7,54 (m, 2H) ; 7,74 (m, 2H); 8,18 (t, 1H).
SM-CL : MH+ = 244,04 ; t.r. = 9,73 min.(61%) et MH₃+ = 246,06 ; t.r. = 8,70 min.

### 66.6) Mélange de 2-(2-fluorophényl)-5-{[2-(diméthylamino)éthyl]amino}-1,3-benzoxazole-4,7-dione et 2-(2-fluorophényl)-6-{[2-(diméthylamino)éthyl]amino}-1,3-benzoxazole-4,7-dione :

Le protocole expérimental utilisé est identique à celui décrit pour l'étape 15.4 de l'exemple 15, l'intermédiaire 66.5 remplaçant l'intermédiaire 15.3 et la *N,N*-diméthyléthylènediamine remplaçant l'aniline. On obtient une poudre rubis Point de fusion: 191 °C.
RMN ¹H (DMSO-*d*₆, δ) : 2,19 (s, 6H) ; 2,5 (m, 2H) ; 3,27 (m, 2H) ; 5,41 (s, 1H) ; 7,42-7,52 (m, 3H) ; 7,70 (m, 2H) ; 8,13 (m, 1H).

Les deux composants du mélange peuvent être caractérisés par les déplacements RMN (400 MHz) du seul proton du cycle benzoxazoledione qui sont 5,40 et 5,41 ppm. SM-CL : MH+ = 330,14 ; t.r. = 7,69 min.

### Exemple 67 : mélange de 2-(2-fluorophényl)-5-[(2-pyrrolidin-l-yléthyl)amino]-1,3-benzoxazole-4,7-dione et 2-(2-fluorophényl)-6-[(2-pyrrolidin-1-yléthyl)amino]-1,3-benzoxazole-4,7-dione :

Le protocole expérimental utilisé est identique à celui décrit pour l'exemple 66, la *N*-(2-aminoéthyl)-pyrrolidine remplaçant la *N,N-*diméthyléthylènediamine. Point de fusion: 152 °C.

Les deux composants du mélange peuvent être caractérisés par les déplacements RMN (400 MHz) du seul proton du cycle benzoxazoledione qui sont 5,39 et 5,41 ppm. SM-CL : MH+= 356,1 ; t.r. = 7,8 min.

### Exemple 68 : mélange de 2-(2-bromophényl)-5-{[2-(diméthylamino)éthyl]amino}-1,3-benzoxazole-4,7-dione et 2-(2-bromophényl)-6-{[2-(diméthylamino)éthyl]amino}-1,3-benzoxazole-4,7-dione :

### 68.1) 2-(2-bromophényl)-6,7-dihydro-l,3-benzoxazol-4(5H)-one :

Le protocole expérimental utilisé est identique à celui décrit pour l'étape 66.2, le 2-bromobenzonitrile remplaçant le 2-fluorobenzonitrile. On obtient un solide jaune. SM-CL : MH+ = 292,0 ; t.r. = 9,8 min.

### 68.2) 5-bromo-2-(2-bromophényl)-6,7-dihydro-1,3-benzoxazol-4(SH)-one :

Un mélange de l'intermédiaire 68.1 (6,6 g, 22 mmol) et de CuBr₂ (10 g ; 45 mmol) dans de l'acétate d'éthyle (250 ml) additionné d'environ 1 ml d'acide acétique est porté au reflux durant 3,5 h (CCM sur SiO₂ : AcOEt/heptane : 1/1). Le milieu réactionnel est ensuite filtré sur Célite, le filtrat est évaporé sous pression réduite et le résidu est purifié sur colonne (SiO₂ : AcOEt/heptane : 1/1) pour donner une poudre jaune clair.
SM-CL : MH+ = 371,8 ; t.r. = 10,5 min.

### 68.3) 2-(2-bromophényl)-5-{[2-(diméthylamino)éthyl]amino}-1,3-benzoxazole-4,7-dione :

Ce composé est obtenu à partir de l'intermédiaire 68.2 selon les modes opératoires décrits pour les étapes 66.4, 66.5 et 66.6. Point de fusion : 138 °C.

Les deux composants du mélange peuvent être caractérisés par les déplacements RMN (400 MHz) du seul proton du cycle benzoxazoledione qui sont 5,41 et 5,43 ppm. SM-CL : MH+ = 390,0 ; t.r. = 7,9 min.

### Exemple 69 : mélange de 2-(2-bromophényl)-5-[(2-pyrrolidin-1-yléthyl)amino]-1,3-benzoxazole-4,7-dione et 2-(2-bromophényl)-6-[(2-pyrrolidin-1-yléthyl)amino]-1,3-benzoxazole-4,7-dione :

Le protocole expérimental utilisé est identique à celui décrit pour l'exemple 68, la *N*-(2-aminoéthyl)-pyrrolidine remplaçant la *N,N*-diméthyléthylènediamine. Point de fusion: 122°C.

Les deux composants du mélange peuvent être caractérisés par les déplacements RMN (400 MHz) du seul proton du cycle benzoxazoledione qui sont 5,40 et 5,42 ppm. SM-CL : MH+ = 416,0 ; t.r. = 8,0 min.

### Exemple 70 : mélange de 2-(2-bromophényl)-5-{[3-(diméthylamino)propyl]amino}-1,3-benzoxazole-4,7-dione et 2-(2-bromophényl)-6-{[3-(diméthylamino)propyl]amino}-1,3-benzoxazole-4,7-dione :

Le protocole expérimental utilisé est identique à celui décrit pour l'exemple 68, la *N,N*-diméthylpropylènediamine remplaçant la *N,N*-diméthyléthylènediamine. Point de fusion: 119 °C.

Les deux composants du mélange peuvent être caractérisés par les déplacements RMN (400 MHz) du seul proton du cycle benzoxazoledione qui sont 5,38 et 5,40 ppm. SM-CL : MH+ = 404,0 ; t.r. = 8,0 min.

### Exemple 71 : mélange de 2-(2-chlorophényl)-5-{[2-(diméthylamino)éthyl]amino}-1,3-benzoxazole-4,7-dione et 2-(2-chlorophényl)-6-{[2-(diméthylamino)éthyl]amino}-1,3-benzoxazole-4,7-dione :

Le protocole expérimental utilisé est identique à celui décrit pour l'exemple 66, le 2-chlorobenzonitrile remplaçant le 2-fluorobenzonitrile. Point de fusion : 137 °C.

Les deux composants du mélange peuvent être caractérisés par les déplacements RMN (400 MHz) du seul proton du cycle benzoxazoledione qui sont 5,39 et 5,41 ppm. SM-CL : MH+ = 346,1 ; t.r. = 7,8 min.

### Exemple 72 : mélange de 2-(2-chlorophényl)-5-[(2-pyrrolidin-l-yléthyl)amino]-1,3-benzoxazole-4,7-dione et 2-(2-chlorophényl)-6-[(2-pyrrolidin-1-yléthyl)amino]-1,3-benzoxazole-4,7-dione :

Le protocole expérimental utilisé est identique à celui décrit pour l'exemple 71, la *N*-(2-aminoéthyl)-pyrrolidine remplaçant la *N,N*-diméthyléthylènediamine. Point de fusion : 85 °C.

Les deux composants du mélange peuvent être caractérisés par les déplacements RMN (400 MHz) du seul proton du cycle benzoxazoledione qui sont 5,40 et 5,41 ppm. SM-CL : MH+= 372,1 ; t.r. = 8,0 min.

### Exemple 73 : mélange de 2-(3-bromophényl)-5-{[2-(diméthylamino)éthyl]amino}-1,3-benzoxazole-4,7-dione et 2-(3-bromophényl)-6-{[2-(diméthylamino)éthyl]amino}-1,3-benzoxazole-4,7-dione :

Le protocole expérimental utilisé est identique à celui décrit pour l'exemple 68, le 3-bromobenzonitrile remplaçant le 2-bromobenzonitrile. Point de fusion : 133 °C.

Les deux composants du mélange peuvent être caractérisés par les déplacements RMN (400 MHz) du seul proton du cycle benzoxazoledione qui sont 5,39 et 5,41 ppm. SM-CL : MH+ = 390,0 ; t.r. = 8,1 min.

### Exemple 74 : mélange de 2-(4-bromophényl)-5-[(2-pyrrolidin-1-yléthyl)amino]-1,3-benzoxazole-4,7-dione et 2-(4-bromophényl)-6-[(2-pyrrolidin-1-yléthyl)amino]-1,3-benzoxazole-4,7-dione :

Le protocole expérimental utilisé est identique à celui décrit pour l'exemple 68, le 4-bromobenzonitrile étant employé au lieu du 2-bromobenzonitrile, et la *N*-(2-aminoéthyl)-pyrrolidine remplaçant la *N,N*-diméthyléthylènediamine. Point de fusion : 181 °C.

Les deux composants du mélange peuvent être caractérisés par les déplacements RMN (400 MHz) du seul proton du cycle benzoxazoledione qui sont 5,37 et 5,39 ppm. SM-CL : MH+ = 415,0 ; t.r. = 8,3 min.

### Exemple 75 : mélange de 2-(4-bromophényl)-5-{[2-(diméthylamino)éthyl]amino}-1,3-benzoxazole-4,7-dione et 2-(4-bromophényl)-6-{[2-(diméthylamino)éthyl]amino}-1,3-benzoxazole-4,7-dione :

Le protocole expérimental utilisé est identique à celui décrit pour l'exemple 74, la *N,N*-diméthyléthylènediamine remplaçant la *N*-(2-aminoéthyl)-pyrrolidine. Point de fusion: 184°C.

Les deux composants du mélange peuvent être caractérisés par les déplacements RMN (400 MHz) du seul proton du cycle benzoxazoledione qui sont 5,38 et 5,40 ppm. SM-CL : MH+ = 390,1 ; t.r. = 8,2 min.

### Exemple 76 : mélange de 2-(4-fluorophényl)-5-[(2-pyrrolidin-1-yléthyl)amino]-1,3-benzoxazole-4,7-dione et 2-(4-fluorophényl)-6-[(2-pyrrolidin-1-yléthyl)amino]-1,3-benzoxazole-4,7-dione :

### 76.1) 2-(4-fluorophényl)-6,7-dihydro-1,3-benzoxazol-4(5H)-one :

Le protocole expérimental utilisé est identique à celui décrit pour l'étape 66.2, le 4-fluorobenzonitrile remplaçant le 2-fluorobenzonitrile. On obtient un solide jaune. SM-CL : MH+ = 232,1 ; t.r. = 9,4 min.

### 76.2) 5-bromo-2-(4-fluorophényl)-6,7-dihydro-1,3-benzoxazol-4(5H)-one :

À une solution de l'intermédiaire 76.1 (600 mg ; 2,59 mmol) dans de l'acide acétique glacial (25 ml) porté à 50°C est ajouté en trois portions égales espacées par des intervalles de 2-3 min du tribromure de pyridinium (996 mg ; 3,11 mmol). Le mélange réactionnel est maintenu à 50 °C durant 4 h (CCM sur SiO₂ : AcOEt/heptane : 1/1). Les volatiles sont évaporés sous pression réduite, puis le résidu est repris à l'eau et extrait avec du dichlorométhane. Le milieu réactionnel est ensuite filtré sur Célite, le filtrat est évaporé sous pression réduite et le résidu est purifié sur colonne (SiO₂ : AcOEt/heptane : 1/1) pour donner une poudre jaune clair. Les phases organiques sont regroupées et lavées avec une solution de bicarbonate à 10% puis avec une solution saturée de chlorure de sodium. Après séchage sur sulfate de sodium et élimination des volatiles sous pression réduite, le résidu est purifié par chromatographie sur colonne (SiO₂ : AcOEt/heptane : 1/1) pour donner une poudre beige.
SM-CL : MH+ = 312,0 ; t.r. = 10,3 min.

### 76.3) Mélange de 2-(4-fluorophényl)-5-{[2-(diméthylamino)éthyl]amino}-1,3-benzoxazole-4,7-dione et 2-(4-fluorophényl)-6-{[2-(diméthylamino)éthyl]amino}-1,3-benzoxazole-4,7-dione :

Ce composé est obtenu à partir de l'intermédiaire 76.2 selon les modes opératoires décrits pour les étapes 66.4, 66.5 et 66.6. Point de fusion : 162 °C.

Les deux composants du mélange peuvent être caractérisés par les déplacements RMN (400 MHz) du seul proton du cycle benzoxazoledione qui sont 5,37 et 5,39 ppm. SM-CL : MH+= 356,1 ; t.r. = 8,0 min.

### Exemple 77 : mélange de 5-{[2-(diméthylamino)éthyl]amino}-2-(4-fluorophényl)-1,3-benzoxazole-4,7-dione et 6-{[2-(diméthylamino)éthyl]amino}-2-(4-fluorophényl)-1,3-benzoxazole-4,7-dione :

Le protocole expérimental utilisé est identique à celui décrit pour l'exemple 76, la *N*-(2-aminoéthyl)-pyrrolidine remplaçant la *N,N*-diméthyléthylènediamine. Point de fusion: 170°C.

Les deux composants du mélange peuvent être caractérisés par les déplacements RMN (400 MHz) du seul proton du cycle benzoxazoledione qui sont 5,38 et 5,39 ppm. SM-CL : MH+ = 330,1 ; t.r. = 7,8 min.

### Exemple 78 : mélange de 5-[(1-benzylpyrrolidin-3-yl)amino]-2-(4-fluorophényl)-1,3-benzoxazole-4,7-dione et 6-[(1-benzylpyrrolidin-3-yl)amino]-2-(4-fluorophényl)-1,3-benzoxazole-4,7-dione :

Le protocole expérimental utilisé est identique à celui décrit pour l'exemple 76, la (1-benzylpyrrolidin-3-yl)-amine remplaçant la *N,N*-diméthyléthylènediamine. Point de fusion: 180 °C.

Les deux composants du mélange peuvent être caractérisés par les déplacements RMN (400 MHz) du seul proton du cycle benzoxazoledione qui sont 5,37 et 5,39 ppm. SM-CL : MH+ = 418,1 ; t.r. = 8,5 min.

### Exemple 79 : mélange de 5-{[3-(diméthylamino)propyl]amino}-2-(4-fluorophényl)-1,3-benzoxazole-4,7-dione et 6-{[3-(diméthylamino)propyl]amino}-2-(4-fluorophényl)-1,3-benzoxazole-4,7-dione :

Le protocole expérimental utilisé est identique à celui décrit pour l'exemple 76, la *N,N*-diméthylpropylènediamine remplaçant la *N,N*-diméthyléthylènediamine. Point de fusion : 149 °C.

Les deux composants du mélange peuvent être caractérisés par les déplacements RMN (400 MHz) du seul proton du cycle benzoxazoledione qui sont 5,35 et 5,37 ppm. SM-CL : MH+ = 344,2 ; t.r. = 7,9 min.

### Exemple 80 : mélange de 2-(3,5-difluorophényl)-5-[(2-pyrrolidin-1-yléthyl)amino]-1,3-benzoxazole-4,7-dione et 2-(3,5-difluorophényl)-6-[(2-pyrrolidin-1-yléthyl)amino]-1,3-benzoxazole-4,7-dione :

Le protocole expérimental utilisé est identique à celui décrit pour l'exemple 68, le 3,5-difluorobenzonitrile remplaçant le 2-bromobenzonitrile et la *N*-(2-aminoéthyl)-pyrrolidine remplaçant la *N,N*-diméthyléthylènediamine. Point de fusion: 158 °C.

Les deux composants du mélange peuvent être caractérisés par les déplacements RMN (400 MHz) du seul proton du cycle benzoxazoledione qui sont 5,41 et 5,43 ppm. SM-CL : MH+ = 374,0 ; t.r. = 8,0 min.

### Exemple 81 : mélange de 2-(3,5-difluorophényl)-5-{[2-(diméthylamino)éthyl]amino}-1,3-benzoxazole-4,7-dione et 2-(3,5-difluorophényl)-6-{[2-(diméthylamino)éthyl]amino}-1,3-benzoxazole-4,7-dione :

Le protocole expérimental utilisé est identique à celui décrit pour l'exemple 68, le 3,5-difluorobenzonitrile remplaçant le 2-bromobenzonitrile. Point de fusion : 175 °C. Les deux composants du mélange peuvent être caractérisés par les déplacements RMN (400 MHz) du seul proton du cycle benzoxazoledione qui sont 5,33 et 5,41 ppm.
SM-CL : MH+ = 348,0 ; t.r. = 7,9 min.

### Exemple 82 : mélange de 2-(2,5-difluorophényl)-5-[(2-pyrrolidin-1-yléthyl)amino]-1,3-benzoxazole-4,7-dione et 2-(2,5-difluorophényl)-6-[(2-pyrrolidin-1-yléthyl)amino]-1,3-benzoxazole-4,7-dione :

Le protocole expérimental utilisé est identique à celui décrit pour l'exemple 68, le 2,5-difluorobenzonitrile remplaçant le 2-bromobenzonitrile et la *N*-(2-aminoéthyl)-pyrrolidine remplaçant la *N,N*-diméthyléthylènediamine.
Point de fusion: 163 °C.

Les deux composants du mélange peuvent être caractérisés par les déplacements RMN (400 MHz) du seul proton du cycle benzoxazoledione qui sont 5,40 et 5,42 ppm. SM-CL : MH+ = 374,0 ; t.r. = 7,9 min.

### Exemple 83 : mélange de 2-(2,5-difluorophényl)-5-{[2-(diméthylamino)éthyl]amino}-1,3-benzoxazole-4,7-dione et 2-(2,5-difluorophényl)-6-{[2-(diméthylamino)éthyl]amino}-1,3-benzoxazole-4,7-dione :

Le protocole expérimental utilisé est identique à celui décrit pour l'exemple 68, le 2,5-difluorobenzonitrile remplaçant le 2-bromobenzonitrile.

Les deux composants du mélange peuvent être caractérisés par les déplacements RMN (400 MHz) du seul proton du cycle benzoxazoledione qui sont 5,41 et 5,43 ppm. SM-CL : MH+ = 348,0 ; t.r. = 7,7 min.

### Exemple 84 : mélange de 2-(2,3-difluorophényl)-5-{[2-(diméthylamino)éthyl]amino}-1,3-benzoxazole-4,7-dione et 2-(2,3-difluorophényl)-6-{[2-(diméthylamino)éthyl]amino}-1,3-benzoxazole-4,7-dione :

Le protocole expérimental utilisé est identique à celui décrit pour l'exemple 68, le 2,3-difluorobenzonitrile remplaçant le 2-bromobenzonitrile. Point de fusion : 167 °C. Les deux composants du mélange peuvent être caractérisés par les déplacements RMN (400 MHz) du seul proton du cycle benzoxazoledione qui sont 5,41 et 5,43 ppm. SM-CL : MH+ = 348,1 ; t.r. = 7,8 min.

### Exemple 85 : mélange de 2-(2,3-difluorophényl)-5-[(2-pyrrolidin-1-yléthyl)amino]-1,3-benzoxazole-4,7-dione et 2-(2,3-difluorophényl)-6-[(2-pyrrolidin-1-yléthyl)amino]-1,3-benzoxazole-4,7-dione :

Le protocole expérimental utilisé est identique à celui décrit pour l'exemple 68, le 2,3-difluorobenzonitrile remplaçant le 2-bromobenzonitrile et la *N*-(2-aminoéthyl)-pyrrolidine remplaçant la *N,N*-diméthyléthylènediamine. Point de fusion : 150 °C.

Les deux composants du mélange peuvent être caractérisés par les déplacements RMN (400 MHz) du seul proton du cycle benzoxazoledione qui sont 5,40 et 5,42 ppm. SM-CL : MH+ = 374,1 ; t.r. = 7,9 min.

### Exemple 86 : mélange de 2-(2,3-difluorophényl)-5-{[3-(diméthylamino)propyl]amino}-1,3-benzoxazole-4,7-dione et 2-(2,3-difluorophényl)-6-{[3-(diméthylamino)propyl]amino}-1,3-benzoxazole-4,7-dione :

Le protocole expérimental utilisé est identique à celui décrit pour l'exemple 68, le 2,3-difluorobenzonitrile étant employé au lieu du 2-bromobenzonitrile, et la *N,N*-diméthylpropylènediamine remplaçant la *N,N*-diméthyléthylènediamine. Point de fusion : 169 °C.

Les deux composants du mélange peuvent être caractérisés par les déplacements RMN (400 MHz) du seul proton du cycle benzoxazoledione qui sont 5,38 et 5,41 ppm. SM-CL : MH+ = 362,1 ; t.r. = 7,8 min.

### Exemple 87 : mélange de 5-[(2-pyrrolidin-1-yléthyl)amino]-2-(3,4,5-trifluorophényl)-1,3-benzoxazole-4,7-dione et 6-[(2-pyrrolidin-1-yléthyl)amino]-2-(3,4,5-trifluorophényl)-1,3-benzoxazole-4,7-dione :

Le protocole expérimental utilisé est identique à celui décrit pour l'exemple 68, le 3,4,5-trifluorobenzonitrile remplaçant le 2-bromobenzonitrile, et la *N*-(2-aminoéthyl)-pyrrolidine remplaçant la *N,N*-diméthyléthylènediamine.

Les deux composants du mélange peuvent être caractérisés par les déplacements RMN (400 MHz) du seul proton du cycle benzoxazoledione qui sont 5,39 et 5,41 ppm. SM-CL : MH+ = 392,0 ; t.r. = 8,2 min.

### Exemple 88 : mélange de 5-{[2-(diméthylamino)éthyl]amino}-2-(3,4,5-trifluorophényl)-1,3-benzoxazole-4,7-dione et 6-{[2-(diméthylamino)éthyl]amino}-2-(3,4,5-trifluorophényl)-1,3-benzoxazole-4,7-dione :

Le protocole expérimental utilisé est identique à celui décrit pour l'exemple 68, le 3,4,5-trifluorobenzonitrile remplaçant le 2-bromobenzonitrile.

Les deux composants du mélange peuvent être caractérisés par les déplacements RMN (400 MHz) du seul proton du cycle benzoxazoledione qui sont 5,40 et 5,42 ppm. SM-CL : MH+ = 366,1 ; t.r. = 8,1 min.

### Exemple 89 : mélange de 5-[(2-pyrrolidin-1-yléthyl)amino]-2-(2,3,4,5-tétrafluorophényl)-1,3-benzoxazole-4,7-dione et 6-[(2-pyrrolidin-1-yléthyl)amino]-2-(2,3,4,5-tétrafluorophényl)-1,3-benzoxazole-4,7-dione :

Le protocole expérimental utilisé est identique à celui décrit pour l'exemple 68, le 2,3,4,5-tétrafluorobenzonitrile remplaçant le 2-bromobenzonitrile et la *N*-(2-aminoéthyl)-pyrrolidine remplaçant la *N,N*-diméthyléthylènediamine.

Les deux composants du mélange peuvent être caractérisés par les déplacements RMN (400 MHz) du seul proton du cycle benzoxazoledione qui sont 5,42 et 5,44 ppm. SM-CL : MH+ = 410,0 ; t.r. = 8,2 min.

### Exemple 90 : mélange de 5-{[2-(diméthylamino)éthyl]amino}-2-(2,3,4,5-tétrafluorophényl)-1,3-benzoxazole-4,7-dione et 6-{[2-(diméthylamino)éthyl]amino}-2-(2,3,4,5-tétrafluorophényl)-1,3-benzoxazole-4,7-dione :

Le protocole expérimental utilisé est identique à celui décrit pour l'exemple 68, le 2,3,4,5-tétrafluorobenzonitrile remplaçant le 2-bromobenzonitrile. Point de fusion : 160 °C.

Les deux composants du mélange peuvent être caractérisés par les déplacements RMN (400 MHz) du seul proton du cycle benzoxazoledione qui sont 5,42 et 5,45 ppm. SM-CL : MH+ = 384,0 ; t.r. = 8,1 min.

### Exemple 91 : mélange de 5-{[2-(diméthylamino)éthyl]amino}-2-[2-fluoro-6-(trifluorométhyl)phényl]-1,3-benzoxazole-4,7-dione et 6-{[2-(diméthylamino)éthyl]amino}-2-[Z-fluoro-6-(trifluorométhyl)phényl]-1,3-benzoxazole-4,7-dione :

Le protocole expérimental utilisé est identique à celui décrit pour l'exemple 68, le 2-fluoro-6-(trifluorométhyl)-benzonitrile remplaçant le 2-bromobenzonitrile.

Les deux composants du mélange peuvent être caractérisés par les déplacements RMN (400 MHz) du seul proton du cycle benzoxazoledione qui sont 5,44 et 5,46 ppm. SM-CL : MH+ = 398,0 ; t.r. = 8,0 min.

### Exemple 92 : mélange de 2-[2-fluoro-6-(trifluorométhyl)phényl]-5-[(2-pyrrolidin-1-yléthyl)amino]-1,3-benzoxazole-4,7-dione et 2-[2-fluoro-6-(trifluorométhyl)phényl]-6-[(2-pyrrolidin-1-yléthyl)amino]-1,3-benzoxazole-4,7-dione :

Le protocole expérimental utilisé est identique à celui décrit pour l'exemple 68, le 2-fluoro-6-(trifluorométhyl)-benzonitrile remplaçant le 2-bromobenzonitrile et la *N*-(2-aminoéthyl)-pyrrolidine remplaçant la *N,N*-diméthyléthylènediamine. Point de fusion : 166 °C.

Les deux composants du mélange peuvent être caractérisés par les déplacements RMN (400 MHz) du seul proton du cycle benzoxazoledione qui sont 5,43 et 5,45 ppm. SM-CL : MH+ = 424,1 ; t.r. = 8,1 min.

### Exemple 93 : mélange de 5-{[3-(diméthylamino)propyl]amino}-2-[2-fluoro-6-(trifluorométhyl)phényl]-1,3-benzoxazole-4,7-dione et 6-{[3-(diméthylamino)propyl]amino}-2-[2-fluoro-6-(trifluorométhyl)phényl]-1,3-benzoxazole-4,7-dione :

Le protocole expérimental utilisé est identique à celui décrit pour l'exemple 68, le 2-fluoro-6-(trifluoromethyl)-benzonitrile remplaçant le 2-bromobenzonitrile et la *N,N*-diméthylpropylènediamine remplaçant la *N,N*-diméthyléthylènediamine. Point de fusion : 128 °C.

Les deux composants du mélange peuvent être caractérisés par les déplacements RMN (400 MHz) du seul proton du cycle benzoxazoledione qui sont 5,42 et 5,43 ppm. SM-CL : MH+ = 412,0 ; t.r. = 8,0 min.

### Exemple 94 : mélange de 2-[2-chloro-5-(trifluorométhyl)phényl]-5-{[2-(diméthylamino)éthyl]amino}-1,3-benzoxazole-4,7-dione et 2-[2-chloro-5-(trifluorométhyl)phényl]-6-{[2-(diméthylamino)éthyl]amino}-1,3-benzoxazole-4,7-dione :

Le protocole expérimental utilisé est identique à celui décrit pour l'exemple 68, le 2-chloro-5-(trifluorométhyl)-benzonitrile remplaçant le 2-bromobenzonitrile. Point de fusion: 182 °C.

Les deux composants du mélange peuvent être caractérisés par les déplacements RMN (400 MHz) du seul proton du cycle benzoxazoledione qui sont 5,43 et 5,46 ppm. SM-CL : MH+ = 414,0 ; t.r. = 8,3 min.

### Exemple 95 : mélange de 2-[2-chloro-5-(trifluorométhyl)phényl]-5-[(2-pyrrolidin-1-yléthyl)amino]-1,3-benzoxazole-4,7-dione et 2-[2-chloro-5-(trifluorométhyl)phényl]-6-[(2-pyrrolidin-1-yléthyl)amino]-1,3-benzoxazole-4,7-dione :

Le protocole expérimental utilisé est identique à celui décrit pour l'exemple 68, le 2-chloro-5-(trifluoromethyl)-benzonitrile remplaçant le 2-bromobenzonitrile et la *N*-(2-aminoéthyl)-pyrrolidine remplaçant la *N,N*-diméthyléthylènediamine. Point de fusion: 152°C.

Les deux composants du mélange peuvent être caractérisés par les déplacements RMN (400 MHz) du seul proton du cycle benzoxazoledione qui sont 5,43 et 5,45 ppm. SM-CL : MH+ = 440,0 ; t.r. = 8,5 min.

### Exemple 96 : mélange de 2-[2-chloro-5-(trifluorométhyl)phényl]-5-{[3-(diméthylamino)propyl]amino}-1,3-benzoxazole-4,7-dione et 2-[2-chloro-5-(trifluorométhyl)phényl]-6-{[3-(diméthylamino)propyl]amino}-1,3-benzoxazole-4,7-dione :

Le protocole expérimental utilisé est identique à celui décrit pour l'exemple 68, le 2-chloro-5-(trifluorométhyl)-benzonitrile remplaçant le 2-bromobenzonitrile et la *N,N*-diméthylpropylènediamine remplaçant la *N,N*-diméthyléthylènediamine. Point de fusion : 121 °C.

Les deux composants du mélange peuvent être caractérisés par les déplacements RMN (400 MHz) du seul proton du cycle benzoxazoledione qui sont 5,41 et 5,43 ppm. SM-CL : MH+ = 428,0 ; t.r. = 8,4 min.

### Exemple 97 : mélange de 2-[2-chloro-6-fluorophényl]-5-1[3-(diméthylamino)propyl]aminol-1,3-benzoxazole-4,7-dione et 2-[2-chloro-6-fluorophényl]-6-{[3-(diméthylamino)propyl]amino}-1,3-benzoxazole-4,7-dione :

Le protocole expérimental utilisé est identique à celui décrit pour l'exemple 68, le 2-chloro-6-fluorobenzonitrile remplaçant le 2-bromobenzonitrile.

Les deux composants du mélange peuvent être caractérisés par les déplacements RMN (400 MHz) du seul proton du cycle benzoxazoledione qui sont 5,43 et 5,45 ppm. SM-CL : MH+= 364,1 ; t.r. = 7,8 min.

### Exemple 98 : mélange de 2-[2-chloro-6-fluorophényl]-5-[(2-pyrrolidin-1-yléthyl)amino]-1,3-benzoxazole-4,7-dione et 2-[2-chloro-6-fluorophényl]-6-[(2-pyrrolidin-1-yléthyl)amino]-1,3-benzoxazole-4,7-dione :

Le protocole expérimental utilisé est identique à celui décrit pour l'exemple 68, le 2-chloro-6-fluorobenzonitrile remplaçant le 2-bromobenzonitrile et la *N*-(2-aminoéthyl)-pyrrolidine remplaçant la *N,N*-diméthyléthylènediamine. Point de fusion : 124 °C.

Les deux composants du mélange peuvent être caractérisés par les déplacements RMN (400 MHz) du seul proton du cycle benzoxazoledione qui sont 5,42 et 5,44 ppm. SM-CL : MH+ = 390,1 ; t.r. = 7,9 min.

### Exemple 99 : mélange de 2-[3,4-diméthoxyphényl]-5-{[2-(diméthytamino)éthyt]amino}-!,3-benzoxazote-4,7-dioneet 2-[3,4-diméthoxyphenyl]-6-{[2-(diméthylamino)éthyl]amino}-1,3-benzoxazole-4,7-dione :

### 99.1) 2-(3,4-diméthoxyphényl)-6,7-dihydro-1,3-benzoxazol-4(5H)-one :

Le protocole expérimental utilisé est identique à celui décrit pour l'étape 66.2, le 3,4-diméthoxybenzonitrile remplaçant le 2-fluorobenzonitrile. On obtient un solide jaune.
SM-CL : MH+ = 274,0 ; t.r. = 8,9 min.

### 99.2) 5-iodo-2-(3,4-diméthoxyphényl)-6,7-dihydro-1,3-benzoxazol-4(5H)-one :

A une solution de l'intermédiaire 99.1 (500 mg, 1,83 mmol) dans l'acide acétique (30 ml) est traitée pendant 96 h à température ambiante par du poly[styrène-co-(4-vinylpyridinium dichloroiodate(1-))] (2,6 g ; 8,25 mEq ; préparé selon B Sket et coll., Bull. Chem. Soc. Jpn (1989), 62, 3406-3408) (contrôle CCM sur SiO₂: 2% MeOH/CH₂Cl₂). Le polymère est retiré par filtration et les volatiles sont évaporés sous pression réduite. Le résidu est purifié sur colonne (SiO₂ : 1% MeOH/CH₂Cl₂) pour donner une huile jaune.
SM-CL : MH+ = 399,9 ; t.r. = 9,8 min.

### 99.3) Mélange de 2-(3,4-diméthoxyphényl)-5-{[2-(diméthylamino)éthyl]amino}-1,3-benzoxazole-4,7-dione et 2-(3,4-diméthoxyphényl)-6-{]2-(diméthylamino)éthyl]amino}-1,3-benzoxazole-4,7-dione :

Ce composé est obtenu à partir de l'intermédiaire 99.2 selon les modes opératoires décrits pour les étapes 66.4, 66.5 et 66.6. Point de fusion : 181 °C.

Les deux composants du mélange peuvent être caractérisés par les déplacements RMN (400 MHz) du seul proton du cycle benzoxazoledione qui sont 5,35 et 5,36 ppm. SM-CL : MH+ = 372,1 ; t.r. = 7,6 min.

### Exemple 100 : mélange de 2-[2-bromo-3-pyridyl]-5-{[2-(diméthylamino)éthyl]amino}-1,3-benzoxazole-4,7-dione et 2-[2-bromo-3-pyridyl]-6-{[2-(diméthylamino)éthyl]amino}-1,3-benzoxazole-4,7-dione :

Le protocole expérimental utilisé est identique à celui décrit pour l'exemple 68, le 2-bromonicotinonitrile remplaçant le 2-bromobenzonitrile. Point de fusion : 133 °C.
Les deux composants du mélange peuvent être caractérisés par les déplacements RMN (400 MHz) du seul proton du cycle benzoxazoledione qui sont 5,43 et 5,45 ppm. SM-CL : MH+ = 391,0 ; t.r. = 7,4 min.

### Exemple 101 : mélange de 2-cyclohexyl-5-[(2-pyrrolidin-l-yléthyl)amino]-1,3-benzothiazole-4,7-dione et 2-cyclohexyl-6-[(2-pyrrolidin-1-yléthyl)amino]-1,3-benzothiazole-4,7-dione :

### 101.1) N-(2,5-diméthoxyphényl)cyclohexanecarboxamide :

A une solution de 1,05 g (6,89 mmol) de 2,5-diméthoxyaniline dans 10 ml d'un mélange toluène/méthanol (1/1) est ajouté 1 ml (7,62 mmol, 1,1 éq.) de chlorure d'acide cyclohexanoïque. Le mélange réactionnel est maintenu sous agitation à 70 °C pendant 1,5 heure, et, après retour à température ambiante, est versé sur 50 ml d'eau. Le produit attendu est extrait par 2 fois avec 50 ml de toluène, puis lavé 2 fois avec 50 ml d'eau.

Les phases organiques sont réunies, séchées sur sulfate de magnésium et le solvant évaporé sous pression réduite. 1,46 g (rendement = 67%) de *N*-(2,5-diméthoxyphényl)cyclohexanecarboxamide sont obtenus et utilisés sans autre purification dans l'étape suivante.
RMN ¹H (DMSO d6, 400 MHz, δ) : 8,84 (s, 1H, NH) ; 7,72-7,71 (m, 1H, H arom.) ; 6,93-6,91 (d, 1H, H arom.); 6,60-6,57 (m, 1H, H arom.); 3,76 (s, 3H, CH₃) ; 3,66 (s, 3H, CH₃) ; 1,78-1,70 (m, 6H, CH₂, CH) ; 1,38-1,24 (m, 5H, CH₂).
SM-CL : MH+ = 264,14 ; t.r. = 10,76 min.

### 101.2) N-(2,5-diméthoxyphényl)cyclohexanecarbothioamide :

1,46 g (5,54 mmol) de *N*-(2,5-diméthoxyphényl)cylohexanecarboxamide est mis en solution dans 40 ml de toluène anhydre. La solution est portée à 100 °C, et 3,34 g (8,26 mmol ; 1,5 éq.) de réactif de Lawesson sont ajoutés au milieu réactionnel qui est ensuite maintenu sous agitation à 100 °C pendant 4 heures. Après retour à température ambiante, la solution est versée sur 50 ml d'eau glacée et extraite à l'aide de toluène. Les phases organiques sont séchées sur sulfate de magnésium et le solvant évaporé. Le *N*-(2,5-diméthoxyphenyl)cyclohexanecarbothioamide est ensuite purifié par chromatographie sur colonne de silice (éluant : dichlorométhane/heptane : 1/1 puis 3/2). 1,26 g (rendement = 81 %) de produit sont obtenus sous forme d'huile jaune.
RMN ¹H (DMSO d6, 400 MHz, δ) : 10,76 (s, 1H, NH) ; 7,28-7,27 (m, 1H, H arom.) ; 7,02-6,99 (d, 1H, H arom.); 6,82-6,80 (m, 1H, H arom.); 3,73 (s, 3H, CH₃) ; 3,68 (s, 3H, CH₃) ; 1,77-1,75 (m, 4H, CH₂) ; 1,67-1,58 (m, 3H, CH₂, CH) ; 1,31-1,15 (m, 4H, 2CH₂).
SM-CL : MH+ = 280,12 ; t.r. = 11,38 min.

### 101.3) 2-cyclohexyl-4,7-diméthoxy-1,3-benzothiazole :

1,26 g (4,50 mmol) de *N*-(2,5-diméthoxyphényl)cylohexanecarbothioamide sont dissous dans 100 ml d'une solution d'hydroxyde de sodium à 1,5 M (100 ml) et le milieu réactionnel est refroidi à 0 °C avant d'ajouter 25 ml d'une solution aqueuse fraîchement préparée de ferricyanure de potassium à 20 % (5,05 g de K₃[Fe(CN)₆] ; 3,4 éq.). Le mélange réactionnel est maintenu sous agitation à température ambiante pendant 24 heures, puis 1,1 g (rendement = 88 %) du dérivé de benzothiazole attendu est obtenu par filtration, lavage à l'eau froide et séchage sous pression réduite en présence de P₂O₅.
RMN ¹H (DMSO d6, 400 MHz, δ) : 6,95-6,85 (dd, 2H, H arom.) ; 3,88 (s, 6H, 2CH₃) ; 3,10-3,04 (m, 1H, CH) ; 2,10-2,07 (m, 2H, CH₂) ; 1,81-1,77 (m, 2H, CH₂) ; 1,70-1,67 (m, 1H, CH) ; 1,57-1,51 (m, 2H, CH₂) ; 1,42-1,39 (m, 2H, CH₂); 1,26-1,28 (m, 1H, CH).
SM-CL : MH+ = 278,09 ; t.r. = 11,91 min.

### 101.4) 2-cyclohexyl-1,3-benzothiazole-4,7-dione :

1 g (3,61 mmol) de 2-cyclohexyl-4,7-diméthoxy-1,3-benzothiazole est mis en suspension dans un mélange acétonitrile/eau (3/1) à 0 °C puis 4,36 g (7,96 mmol ; 2,2 éq.) de nitrate de cérium (IV) et d'ammonium sont ajoutés à la suspension. Le mélange réactionnel est maintenu 1,5 heure sous agitation à température ambiante, puis 0,78 g (rendement = 88 %) de 2-cyclohexyl-1,3-benzothiazole-4,7-dione est obtenu après filtration, lavage à l'eau froide et séchage sous pression réduite.
RMN ¹H (DMSO d6, 400 MHz, δ) : 6,90 (s, 2H); 3,15-3,10 (m, 1H, CH); 2,10-2,07 (m, 2H, CH₂); 1,81-1,77 (m, 2H, CH₂); 1,65-1,70 (m, 1H, CH); 1,55-1,39 (m, 5H, CH, CH₂).
SM-CL : MH+ = 248,12 ; t.r. = 10,82 min.

### 101.5) N-(2,5-diméthoxyphényl)cyclohexanecarboxamide :

Le protocole expérimental utilisé est identique à celui décrit pour l'étape 15.4 de l'exemple 15, l'intermédiaire 101.4 remplaçant l'intermédiaire 15.3 et la *N,N*-diméthyléthylène diamine remplaçant l'aniline. Un mélange de 80 % et 9 % de 2-cyclohexyl-5-{[2-(diméthylamino)éthyl]amino}-1,3-benzothiazole-4,7-dione et de 2-cyclohexyl-6-{[2-(diméthylamino)éthyl]amino}-1,3-benzothiazole-4,7-dione est obtenu.

RMN ¹H (DMSO d6, 400 MHz, δ) : 7,20 (t, 1H, NH); 5,49 et 5,43 (2s, H); 3,24-3,21 (m, 2H, CH₂) ; 3,09-3,12 (m, 3H, CH, CH₂) ; 2,19 (s, 6H, 2CH₃) ; 2,09-2,06 (m, 2H, CH₂) ; 1,80-1,77 (m, 3H, CH, CH₂) ; 1,53-1,49 (m, 4H, 2CH₂) ; 1,41-1,38 (m, 1H, CH).
SM-CL : MH+ = 334,17 ; t.r. = 7,99 et 8,06 min.

### Les composés des exemples 102 à 113 sont obtenus de façon analogue à celle décrite pour l'exemple 101.

### Exemple 102 : mélange de 2-cyclohexyl-5-[(2-pyrrolidin-1-yléthyl)amino]-1,3-benzothiazole-4,7-dione et 2-cyclohexyl-6-[(2-pyrrolidin-1-yléthyl)amino]-1,3-benzothiazole-4,7-dione :

SM-CL : MH+ = 360,16 ; t.r. = 8,14 et 8,19 min.

### Exemple 103 : mélange de 5-[(2-pyrrolidin-1-yléthyl)amino]-2-thién-2-yl-1,3-benzothiazole-4,7-dione et 6-[(2-pyrrolidin-1-yléthyl)amino]-2-thién-2-yl-1,3-benzothiazole-4,7-dione :

SM-CL : MH+ = 360,01 ; t.r. = 7,78 et 7,86 min.

### Exemple 104 : mélange de 2-(2,5-dichlorothién-3-yl)-5-{[2-(diméthylamino)éthyl]amino}-1,3-benzothiazole-4,7-dione et 2-(2,5-dichlorothién-3-yl)-6-{[2-(diméthylamino)éthyl]amino}-1,3-benzothiazole-4,7-dione :

SM-CL : MH+ = 401,86 ; t.r. = 8,44 et 8,59 min.

### Exemple 105 : mélange de 2-(2,5-dichlorothién-3-yl)-5-[(2-pyrrolidin-1-yléthyl)amino]-1,3-benzothiazole-4,7-dione et 2-(2,5-dichlorothién-3-yl)-6-[(2-pyrrolidin-1-yléthyl)amino]-1,3-benzothiazole-4,7-dione :

SM-CL : MH+ = 427,87 ; t.r. = 8,63 et 8,80 min.

### Exemple 106 : mélange de 2-(2-furyl)-5-[(2-pyrrolidin-1-yléthyl)amino]-1,3-benzothiazole-4,7-dione et 2-(2-furyl)-6-[(2-pyrrolidin-1-yléthyl)amino]-1,3-benzothiazole-4,7-dione :

SM-CL : MH+ = 344,04 ; t.r. = 7,57 et 7,64 min.

### Exemple 107 : mélange de 5-{[2-(diméthylamino)éthyl]amino}-2-(2-méthoxyphényl)-1,3-benzothiazole-4,7-dione et 6-{[2-(diméthylamino)éthyl]amino}-2-(2-méthoxyphényl)-1,3-benzothiazole-4,7-dione :

SM-CL : MH+ = 358,18 ; t.r. = 7,88 et 7,97 min.

### Exemple 108 : mélange de 5-{[2-(diméthylamino)éthyl]amino}-2-(2-fluorophényl)-1,3-benzothiazole-4,7-dione et 6-{[2-(diméthylamino)éthyl]amino}-2-(2-fluorophényl)-1,3-benzothiazole-4,7-dione :

SM-CL : MH+ = 346,14 ; t.r. = 7,85 et 7,94 min.

### Exemple 109 : mélange de 2-(2-fluorophényl)-5-[(2-pyrrolidin-1-yléthyl)amino]-1,3-benzothiazole-4,7-dione et 2-(2-fluorophényl)-6-[(2-pyrrolidin-1-yléthyl)amino]-1,3-benzothiazole-4,7-dione :

SM-CL : MH+ = 372,14 ; t.r. = 7,97 et 8,06 min.

### Exemple 110 : mélange de 2-(4-fluorophényl)-5-[(2-pyrrolidin-1-yléthyl)amino]-1,3-benzothiazole-4,7-dione et 2-(4-fluorophényl)-6-[(2-pyrrolidin-1-yléthyl)amino]-1,3-benzothiazole-4,7-dione :

SM-CL : MH+ = 372,05 ; t.r. = 7,98 et 8,07 min.

### Exemple 111 : mélange de 5-{[2-(diméthylamino)éthyl]amino}-2-(4-fluorophényl)-1,3-benzothiazole-4,7-dione et 6-{[2-(diméthylamino)éthyl]amino}-2-(4-fluorophényl)-1,3-benzothiazole-4,7-dione :

SM-CL : MH+ = 346,05 ; t.r. = 7,87 et 7,95 min.

### Exemple 112 : mélange de 2-(2,6-difluorophényl)-5-[(2-pyrrolidin-1-yléthyl)amino]-1,3-benzothiazole-4,7-dione et 2-(2,6-difluorophényl)-6-[(2-pyrrolidin-1-yléthyl)amino]-1,3-benzothiazole-4,7-dione :

SM-CL : MH+ = 390,04 ; t.r. = 7,89 et 7,95 min.

### Exemple 113 : mélange de 2-(2,6-difluorophényl)-5-{[2-(diméthylamino)éthyl]amino}-1,3-benzothiazole-4,7-dione et 2-(2,6-difluorophényl)-6-{[2-(diméthylamino)éthyl]amino}-1,3-benzothiazole-4,7-dione :

SM-CL : MH+ = 364,05 ; t.r. = 7,78 et 7,83 min.

### Exemple 114 : 5-[[2-(diméthylamino)éthyl](éthyl)amino]-2-méthyl-1,3-benzothiazole-4,7-dione :

Ce composé est obtenu de façon analogue à celle décrite pour l'exemple 1, la *N,N,N*'-triméthyléthylènediamine remplaçant la 4-(2-aminoéthyl)morpholine.
RMN ¹H (DMSO d6, 400 MHz, δ) : 5,53 (s, 1H, CH) ; 3,73-3,70 (t, 2H, CH₂) ; 2,93 (s, 3H, CH₃) ; 2,74 (s, 3H, CH₃) ; 2,32-2,30 (t, 2H, CH₂) ; 1,92 (s, 6H, 2CH₃). SM-CL : MH+ = 280,11 ; t.r. = 7,03 min.

### Exemple 115 : 5-[[2-(diméthylamino)éthyl](méthyl)amino]-2-méthyl-1,3-benzothiazole-4,7-dione :

Ce composé est obtenu de façon analogue à celle décrite pour l'exemple 1, la *N,N*-diméthyl-*N*'-éthylènediamine remplaçant la 4-(2-aminoéthyl)morpholine. SM-CL : MH+ = 294,07 ; t.r. = 7,20 min.

### Exemple 116 : mélange de 2-[2,6-dichloro-5-fluoro-3-pyridyl]-5-{[2-(diméthylamino)éthyl]amino}-1,3-benzoxazole-4,7-dione et 2-[2,6-dichloro-5-fluoro-3-pyridyl]-6-{[2-(diméthylamino)éthyl]amino}-1,3-benzoxazole-4,7-dione :

Le protocole expérimental utilisé est identique à celui décrit pour l'exemple 68, le 2,6-dichloro-5-fluoronicotinonitrile remplaçant le 2-bromobenzonitrile.
SM-CL : MH+ = 399,1 ; t.r. = 8,1 min.

### Exemple 117 : mélange de 2-[2,6-dichloro-5-fluoro-3-pyridyl]-5-[(2-pyrrolidin-1-yléthyl)amino]-1,3-benzoxazole-4,7-dione et 2-[2,6-dichloro-5-fluoro-3-pyridyl]-6-[(2-pyrrolidin-1-yléthyl)amino]-1,3-benzoxazole-4,7-dione :

Le protocole expérimental utilisé est identique à celui décrit pour l'exemple 68, le 2,6-dichloro-5-fluoronicotinonitrile remplaçant le 2-bromobenzonitrile et la *N*-(2-aminoéthyl)-pyrrolidine remplaçant la *N,N-*diméthyléthylènediamine.
SM-CL : MH+ = 399,1 ; t.r. = 8,1 min.

### Exemple 118 : mélange de 2-(2,4-difluorophényl)-5-{[2-(diméthylamino)éthyl]amino}-1,3-benzoxazole-4,7-dione et 2-(2,4-difluorophényl)-6-{[2-(diméthylamino)éthyl]amino}-1,3-benzoxazole-4,7-dione :

Le protocole expérimental utilisé est identique à celui décrit pour l'exemple 68, le 2,4-difluorobenzonitrile remplaçant le 2-bromobenzonitrile.
SM-CL : MH+ = 348,1 ; t.r. = 7,8 min.

### Exemple 119 : mélange de 5-{[2-(diméthylamino)éthyl]amino}-2-(2,3,4-trifluorophényl)-1,3-benzoxazole-4,7-dione et 6-{[2-(diméthylamino)éthyl]amino}-2-(2,3,4-trifluorophényl)-1,3-benzoxazole-4,7-dione :

Le protocole expérimental utilisé est identique à celui décrit pour l'exemple 68, le 2,3,4-trifluorobenzonitrile remplaçant le 2-bromobenzonitrile. Point de fusion: 156 °C. SM-CL : MH+ = 366,1 ; t.r. = 8,0 min.

### Exemple 120 : mélange de 5-[(2-pyrrolidin-1-yléthyl)amino]-2-(2,3,4-trifluorophényl)-1,3-benzoxazole-4,7-dione et 6-[(2-pyrrolidin-1-yléthyl)amino]-2-(2,3,4-trifluorophényl)-1,3-benzoxazole-4,7-dione :

Le protocole expérimental utilisé est identique à celui décrit pour l'exemple 68, le 2,3,4-trifluorobenzonitrile remplaçant le 2-bromobenzonitrile et la *N*-(2-aminoéthyl)-pyrrolidine remplaçant la *N,N*-diméthyléthylènediamine.
SM-CL : MH+ = 392,1 ; t.r. = 8,1 min.

### Exemple 121 : mélange de 2-(3-fluoro-4-méthylphényl)-5-{[2-(diméthylamino)éthyl]amino}-1,3-benzoxazole-4,7-dione et 2-(3-fluoro-4-méthylphényl)-6-{[2-(diméthylamino)éthyl]amino}-1,3-benzoxazole-4,7-dione :

Le protocole expérimental utilisé est identique à celui décrit pour l'exemple 68, le 3-fluoro-4-méthylbenzonitrile remplaçant le 2-bromobenzonitrile. Point de fusion: 179 °C.
SM-CL : MH+ = 344,1 ; t.r. = 8,1 min.

### Exemple 122 : mélange de 2-(3-fluoro-4-méthylphényl)-5-[(2-pyrrolidin-1-yléthyl)amino]-1,3-benzoxazole-4,7-dione et 2-(3-fluoro-4-méthylphényl)-6-[(2-pyrrolidin-1-yléthyl)amino]-1,3-benzoxazole-4,7-dione :

Le protocole expérimental utilisé est identique à celui décrit pour l'exemple 68, le 3-fluoro-4-méthylbenzonitrile remplaçant le 2-bromobenzonitrile et la *N*-(2-aminoéthyl)-pyrrolidine remplaçant la *N,N*-diméthyléthylènediamine.
SM-CL : MH+ = 370,1 ; t.r. = 8,2 min.

### Les composés des exemples 123 à 127 sont obtenus de façon analogue à celle décrite pour l'exemple 101.

### Exemple 123 : mélange de 2-(4-chlorophényl)-5-{[2-(diméthylamino)éthyl]amino}-1,3-benzothiazole-4,7-dione et 2-(4-chlorophényl)-6-{[2-(diméthylamino)éthyl]amino}-1,3-benzothiazole-4,7-dione :

SM-CL : MH+ = 362,07 ; t.r. = 8,11 et 8,20 min.

### Exemple 124 : mélange de 2-(4-chlorophényl)-5-[(2-pyrrolidin-1-yléthyl)amino]-1,3-benzothiazole-4,7-dione et 2-(4-chlorophényl)-6-[(2-pyrrolidin-1-yléthyl)amino]-1,3-benzothiazole-4,7-dione :

SM-CL : MH+ = 388,04 ; t.r. = 8,23 et 8,34 min.

### Exemple 125 : mélange de 5-{[2-(diméthylamino)éthyl]amino}-2-(2,3,4,5-tétrafluorophényl)-1,3-benzothiazole-4,7-dione et 6-{[2-(diméthylamino)éthyl]amino}-2-(2,3,4,5-tétrafluorophényl)-1,3-benzothiazole-4,7-dione :

SM-CL : MH+ = 400,01 ; t.r. = 8,23 et 8,32 min.

### Exemple 126 : mélange de 5-{[2-(diméthylamino)éthyl]amino}-2-(3,4,5-trifluorophényl)-1,3-benzothiazole-4,7-dione et 6-{[2-(diméthylamino)éthyl]amino}-2-(3,4,5-trifluorophényl)-1,3-benzothiazole-4,7-dione :

SM-CL : MH+ = 382,03 ; t.r. = 8,10 et 8,19 min.

### Exemple 127 : mélange de 5-[(2-pyrrolidin-1-yléthyl)amino]-2-(2,4,6-trifluorophényl)-1,3-benzothiazole-4,7-dione et 6-[(2-pyrrolidin-1-yléthyl)amino]-2-(2,4,6-trifluorophényl)-1,3-benzothiazole-4,7-dione :

SM-CL : MH+ = 408,02 ; t.r. = 7,97 et 8,05 min.

### Les composés des exemples 128 à 131 sont obtenus de façon analogue à celle décrite pour l'exemple 66.

### Exemple 128 : mélange de 2-(1,3-benzodioxol-5-yl)-5-{[2-(diméthylamino)éthyl]amino}-1,3-benzoxazole-4,7-dione et de 2-(1,3-benzodioxol-5-yl)-6-{[2-(diméthylamino)éthyl]amino}-1,3-benzoxazole-4,7-dione :

Les deux composants du mélange peuvent être caractérisés par les déplacements RMN (400 MHz) du seul proton du cycle benzoxazoledione qui sont 5,35 et 5,37 ppm. SM-CL : MH+ = 356,07 ; t.r. = 7,72 min.

### Exemple 129 : mélange de 5-{[2-(diméthylamino)éthyl]amino}-2-(4-éthylphényl)-1,3-benzoxazole-4,7-dione et de 6-{[2-(diméthylamino)éthyl]amino}-2-(4-éthylphényl)-1,3-benzoxazole-4,7-dione :

Les deux composants du mélange peuvent être caractérisés par les déplacements RMN (400 MHz) du seul proton du cycle benzoxazoledione qui sont 5,36 et 5,38 ppm. SM-CL : MH+ = 340,18 ; t.r. = 8,24 min.

### Exemple 130 : mélange de 2-(4-éthylphényl)-5-[(2-pyrrolidin-1-yléthyl)amino]-1,3-benzoxazole-4,7-dione et 2-(4-éthylphényl)-6-[(2-pyrrolidin-1-yléthyl)amino]-1,3-benzoxazole-4,7-dione :

Les deux composants du mélange peuvent être caractérisés par les déplacements RMN (400 MHz) du seul proton du cycle benzoxazoledione qui sont 5,35 et 5,36 ppm. SM-CL : MH+ = 366,15 ; t.r. = 8,34 min.

### Exemple 131 : mélange de 5-{[2-(diméthylamino)éthyl]amino}-2-(2-fluoro-6-méthoxyphényl)-1,3-benzoxazole-4,7-dione et 6-{[2-(diméthylamino)éthyl]amino}-2-(2-fluoro-6-méthoxyphényl)-1,3-benzoxazole-4,7-dione :

Les deux composants du mélange peuvent être caractérisés par les déplacements RMN (400 MHz) du seul proton du cycle benzoxazoledione qui sont 5,39 et 5,40 ppm. SM-CL : MH+ = 360,09 ; t.r. = 7,67 min.

### Exemple 132 : 2-(2,6-difluorophényl)-5-{[2-(diméthylamino)éthyl]amino}-1,3-benzothiazole-4,7-dione :

### 132.1) N-(3,5-diméthoxyphényl)-2,6-difluorobenzamide :

5,5 ml (39,2 mmol ; 1,2 équivalent) de triéthylamine et 4,5 ml (35,9 mmol ; 1,1 équivalent) de chlorure de 2,6-difluorobenzoyle sont ajoutés à 5 g (32,6 mmol) de 3,5-diméthoxyaniline en solution dans 100 ml de toluène anhydre. Le milieu réactionnel est maintenu sous agitation à 70 °C pendant 1 h 30, puis, après retour à température ambiante, est lavé par 3 fois 50 ml d'eau. La phase organique résultante est séchée sur sulfate de magnésium puis le solvant est évaporé sous pression réduite. Le produit attendu est obtenu sous forme d'une poudre blanche (8,75 g ; rendement = 97 %) utilisée dans l'étape suivante sans autre purification.
SM-CL : MH+ = 294,11 ; t.r. = 9,93 min.

### 132.2) N-(3,5-diméthoxyphényl)-2,6-difluorobenzenecarbothioamide:

A 9,8 g (33,4 mmol) de *N*-(3,5-diméthoxyphényl)-2,6-difluorobenzamide en solution dans 150 ml de toluène anhydre sont ajoutés 20,3 g (50 mmol ; 1,5 équivalents) de réactif de Lawesson. Le milieu réactionnel est maintenu sous agitation à 120 °C pendant 8 heures, puis, après retour à température ambiante, est lavé par 3 fois 75 ml d'eau. La phase organique résultante est séchée sur sulfate de magnésium puis le solvant est évaporé sous pression réduite. Le résidu est purifié par chromatographie sur colonne de silice (éluant : dichlorométhane/méthanol 98/2) et le produit attendu est obtenu sous forme d'une huile verte (10 g ; rendement = 96 %).
SM-CL : MH+ = 310,06; t.r. = 10,53 min.

### 132.3) 2-(2,6-difluorophényl)-5,7-diméthoxy-l,3-benzothiazole:

A 10,3 g (33,3 mmol) de *N*-(3,5-diméthoxyphényl)-2,6-difluorobenzenecarbothioamide dissous dans 150 ml d'une solution de soude à 1,5M sont ajoutés 170 ml (103 mmol ; 3 équivalents) d'une solution aqueuse fraîchement préparée de ferricyanure de potassium à 20%. Le milieu réactionnel est maintenu sous agitation à température ambiante pendant 24 heures, puis le précipité beige formé est filtré, lavé par de l'eau et séché (6,8 g ; rendement = 66%). Les eaux-mères peuvent être extraites par 3 fois 75 ml de dichlorométhane, puis les phases organiques lavées par une solution saturée en chlorure de sodium. Après concentration sous pression réduite, le résidu obtenu peut être purifié sur colonne de silice (éluant : acétate d'éthyle/heptane : 1/3) pour fournir 2 g supplémentaires de produit attendu (rendement global = 86%). Point de fusion : 136-138°C.
RMN ¹H (DMSO d6, 400 MHz, δ) : 7,65 (m, 1H, H arom.) ; 7,36-7,31 (m, 3H, H arom.) ; 6,75 (m, 1H, H arom.) ; 3,96 (s, 3H, CH₃) ; 3,87 (s, 3H, CH₃). SM-CL : MH+ = 308,12 ; t.r. = 11,48 min.

### 132.4) 2-(2,6-difluorophényl)-5-méthoxy-1,3-benzothiazole-4,7-dione :

### 132.4.1) 2-(2,6-difluorophényl)-5,7-diméthoxy-4-nitro-1,3-benzothiazole :

A 3 g (9,76 mmol) de 2-(2,6-difluorophényl)-5,7-diméthoxy-1,3-benzothiazole en solution dans 75 ml d'acétate d'éthyle, est ajoutée goutte à goutte une solution de 16 g (29,3 mmol ; 3 équivalents) de nitrate de cérium et d'ammonium dans 40 ml d'eau. Le mélange réactionnel est maintenu sous agitation pendant 2 heures à température ambiante, puis lavé par 3 fois 20 ml d'eau. Les phases organiques sont séchées sur sulfate de magnésium, filtrées puis concentrées sous pression réduite. Le résidu est purifié par chromatographie sur colonne de silice (éluant : acétate d'éthyle/heptane : 3/7). Deux fractions sont séparées :
0,3 g (rendement = 10%) de 2-(2,6-difluorophényl)-5-méthoxy-1,3-benzothiazole-4,7-dione sont obtenus sous forme de poudre jaune.
SM-CL : MH+ = 308,08 ; t.r. = 10 min.

1,5 g de 2-(2,6-difluorophényl)-5,7-diméthoxy-4-nitro-1,3-benzothiazole (rendement de 45%) sont obtenus sous forme de poudre orangée.
RMN ¹H (DMSO d6, 400 MHz, δ) : 7,72 (m, 1H, H arom.) ; 7,38 (m, 2H, H arom.) ; 7,11 (m, 1H, H arom.) ; 4,12 (s, 3H, CH₃) ; 4,07 (s, 3H, CH₃).
SM-CL : MH+= 353,05 ; t.r. = 11,30 min.

### 132.4.2) 2-(2,6-difluorophényl)-5,7-diméthoxy-1,3-benzothiazol-4-amine :

230 mg (0,65 mmol) d'intermédiaire 132.4.1 mis en solution dans 15 ml d'acide chlorhydrique concentré sont mis en réaction avec 0,5 g (2,2 mmol ; 3,4 équivalents) de chlorure d'étain dihydraté dans 5 ml d'eau. Le mélange réactionnel est maintenu sous agitation pendant 2 heures à 50°C, puis après retour à température ambiante, est versé sur de la glace avant de le neutraliser avec une solution 5M de soude. Le produit est ensuite extrait par 3 fois 15 ml de dichlorométhane, les phases organiques sont réunies, lavées par une solution saturée en chlorure de sodium, séchées sur sulfate de magnésium, filtrées puis, après concentration sous pression réduite, le produit attendu est obtenu sous forme d'une huile jaune. Il sera utilisé dans l'étape suivante sans autre purification.
RMN ¹H (DMSO d6, 400 MHz, δ) : 7,67 (m, 1H, H arom.) ; 7,34 (m, 2H, H arom.) ; 6,92 (s, 1H, H arom.) ; 3,91 (s, 3H, CH₃) ; 3,90 (s, 3H, CH₃).
SM-CL : MH+ = 323,10 ; t.r. = 9,86 min.

### 132.4.3) 2-(2,6-difluorophényl)-5-méthoxy-1,3-benzothiazole-4,7-dione :

A 343 mg (1,06 mmol) de 2-(2,6-difluorophényl)-5,7-diméthoxy-1,3-benzothiazol-4-amine en solution dans 25 ml d'acétate d'éthyle est ajoutée une solution de 1,22 g de cérium ammonium nitrate (2,23 mmol, 2,1 équivalents) dans 8 ml d'eau. Le mélange réactionnel est maintenu sous vigoureuse agitation à température ambiante pendant 1 heure 30 puis la phase organique est séparée et lavée par 3 fois 20 ml d'eau, puis séchée sur sulfate de magnésium, filtrée et le solvant est évaporé sous pression réduite. Le résidu est purifié par chromatographie sur colonne de silice (éluant : acétate d'éthyle/heptane : 3/7) et 280 mg (rendement = 86%) de 2-(2,6-difluorophényl)-5-méthoxy-1,3-benzothiazole-4,7-dione sont obtenus sous forme de poudre jaune.
RMN ¹H (DMSO d6, 400 MHz, δ) : 7,72 (m, 1H, H arom.) ; 7,39 (m, 2H, H arom.) ; 6,32 (s, 1H, CH) ; 3,88 (s, 3H, CH₃).
SM-CL : MH+ = 308,05 ; t.r. = 9,99 min.

### 132.5) 2-(2,6-difluorophényl)-5-{[2-(diméthylamino)éthyl]amino}-1,3-benzothiazole-4,7-dione :

104 ml (0,95 mmol ; 1,5 équivalents) de N,N-diméthyléthylènediamine sont ajoutés à 195 mg de 2-(2,6-difluorophényl)-5-méthoxy-1,3-benzothiazole-4,7-dione en solution dans 20 ml d'éthanol anhydre. Le mélange réactionnel est agité à 70°C pendant 2 heures puis le solvant est évaporé sous pression réduite. Le résidu est purifié sur colonne de silice (éluant : méthanol à 5% dans du dichlorométhane). 130 mg (rendement = 57%) de composé attendu sont obtenus sous forme d'une poudre rouge.
RMN ¹H (DMSO d6, 400 MHz, δ) : 7,72 (m, 1H, H arom.) ; 7,52 (m, 1H, NH.) ; 7,38 (m, 2H, H arom.) ; 5,60 (s, 1H, CH) ; 3,28 (m, 2H, CH₂) ; 2,53 (m, 2H, CH₂); 2,20 (s, 6H, 2CH₃).
SM-CL : MH+ = 364,14 ; t.r. = 7,85 min.

*Les composés des exemples 133 à 138 sont obtenus de façon analogue à celle décrite pour l'exemple 132, les chlorures d'acyle adéquats remplaçant le chlorure de 2,6-difluorobenzoyle dans la première étape et la N-(2-aminoéthyl)pyrrolidine remplaçant la N,N-diméthyléthylènediamine dans la dernière étape pour les exemples 134, 136 et 138.*

### Exemple 133 : 2-(2,5-dichlorothién-3-yl)-5-{[2-(diméthylamino)éthyl]amino}-1,3-benzothiazole-4,7-dione :

### 133.1) 2,5-dichloro-N-(3,5-diméthoxyphényl)thiophène-3-carboxamide :

RMN ¹H (DMSO d6, 400 MHz, δ) : 10,20 (s, 1H, NH) ; 7,47 (s, 1H, H arom.) ; 6,95 (s, 1H, H arom.) ; 6,27 (s, 1H, H arom.) ; 3,72 (s, 6H, 2CH₃).
SM-CL : MH+ = 332,01 ; t.r. = 11,08 min.

### 133.2) 2,5-dichloro-N-(3,5-diméthoxyphényl)thiophène-3-carbothioamide :

RMN ¹H (DMSO d6, 400 MHz, δ) : 11,96 (s, 1H, NH) ; 7,30 (s, 1H, H arom.) ; 7,25 (s, 1H, H arom.) ; 6,44 (s, 1H, H arom.) ; 3,74 (s, 6H, 2CH₃).
SM-CL : MH+ = 348,00 ; t.r. = 11,55 min.

### 133.3) 2-(2,5-dichlorothién-3-yl)-5,7-diméthoxy-1,3-benzothiazole :

RMN ¹H (DMSO d6, 400 MHz, δ) : 7,72 (s, 1H, H arom.) ; 7,22 (s, 1H, H arom.) ; 6,73 (s, 1H, H arom.) ; 3,96 (s, 3H, CH₃) ; 3,86 (s, 3H, CH₃).
SM-CL : MH+ = 345,94 ; t.r. = 12,77 min.

### 133.4) 2-(2,5-dichlorothién-3-yl)-5-méthoxy-1,3-benzothiazole-4,7-dione :

RMN ¹H (DMSO d6, 400 MHz, δ) : 7,75 (s, 1H, H arom.) ; 6,31 (s, 1H, CH) ; 3,88 (s, 3H, CH₃).
SM-CL : MH+ = 345,98 ; t.r. = 11,52 min.

### 133.5) 2-(2,5-dichlorothién-3-yl)-5-{[2-(diméthylamino)éthyl]amino}-1,3-benzothiazole-4,7-dione :

RMN ¹H (DMSO d6, 400 MHz, δ) : 7,72 (s, 1H, H arom.) ; 7,51 (m, 1H, NH.) ; 5,58 (s, 1H, CH) ; 3,36 (m, 2H, CH₂) ; 2,54 (m, 2H, CH₂) ; 2,20 (s, 6H, 2CH₃).
SM-CL : MH+ = 402,06 ; t.r. = 8,42 min.

### Exemple 134 : 2-(2,5-dichlorothién-3-yl)-5-[(2-pyrrolidin-1-yléthyl)aminol-1,3-benzothiazole-4,7-dione :

SM-CL : MH+ = 427,97 ; t.r. = 8,70 min.

### Exemple 135 : 5-{[2-(diméthylamino)éthyl]amino}-2-(4-fluorophényl)-1,3-benzothiazole-4,7-dione :

### 135.1) N-(3,5-diméthoxyphényl)-4-fluorobenzamide

RMN ¹H (DMSO d6, 400 MHz**,** δ) : 10,15 (s, 1H, NH) ; 8,01 (m, 2H, H arom.) ; 7,36 (m, 2H, H arom.) ; 7,05 (m, 2H, H arom.); 6,26 (s, 1H, H arom.) ; 3,73 (s, 6H, 2CH₃).
SM-CL : MH+ = 276,17 ; t.r. = 10,07 min.

### 135.2) N-(3,5-diméthoxyphényl)-4-fluorobenzenecarbothioamide :

SM-CL : MH+ = 292,17 ; t.r. = 10,72 min.

### 135.3) 2-(4-fluorophényl)-5,7-diméthoxy-1,3-benzothiazole :

RMN ¹H (DMSO d6, 400 MHz, δ) : 8,11 (m, 2H, H arom.) ; 7,40 (m, 2H, H arom.) ; 7,22 (s, 1H, H arom.) ; 6,69 (s, 1H, H arom.) ; 3,95 (s, 3H, CH₃) ; 3,86 (s, 3H, CH₃). SM-CL : MH+ = 290,07 ; t.r. = 11,93 min.

### 135.4) 2-(4-fluorophényo-5-méthoxy-1,3-benzothiazole-4,7-dione :

RMN ¹H (DMSO d6, 400 MHz, δ) : 8,15 (m, 2H, H arom.) ; 7,42 (m, 2H, H arom.) ; 6,28 (s, 1H, CH) ; 3,87 (s, 3H, CH₃).
SM-CL : MH+ = 290,14 ; t.r. = 11,95 min.

### 135.5) 5-{[2-(diméthylamino)éthyl]amino}-2-(4-fluorophényl)-1,3-benzothiazole-4,7-dione :

RMN ¹H (DMSO d6, 400 MHz, δ) : 8,11 (m, 2H, H arom.); 7,48 (m, 1H, NH) ; 7,41 (m, 2H, H arom.) ; 5,57 (s, 1H, CH) ; 3,26 (m, 2H, CH₂) 2,55 (m, 2H, CH₂); 2,22 (s, 6H, 2CH₃).

SM-CL : MH+ = 346,18 ; t.r. = 8,01 min.

### Exemple 136 : 2-(4-fluorophényl)-5-[(2-pyrrolidin-1-yléthyl)amino]-1,3-benzothiazole-4,7-dione :

RMN ¹H (DMSO d6, 400 MHz, δ) : 8,12 (m, 2H, H arom.) ; 7,58 (m, 1H, NH) ; 7,41 (m, 2H, H arom.) ; 5,55 (s, 1H, CH) ; 3,41 (m, 2H, CH₂); 2,69 (m, 2H, CH₂) ; 2,51 (m, 2H, CH₂) ; 2,44 (m, 2H, CH2) ; 1,70 (m, 4H, 2CH2).
SM-CL : MH+ = 372,19 ; t.r. = 8,12 min.

### Exemple 137 : 2-(2-chloro-6-fluorophényl)-5-{[2-(diméthylamino)éthyl]amino}-1,3-benzothiazole-4,7-dione :

### 137.1) 2-chloro-N-(3,5-diméthoxyphényl)-6-fluorobenzamide :

RMN ¹H (DMSO d6, 400 MHz, δ) : 10,69 (s, 1H, NH) ; 7,53 (m, 1H, H arom.) ; 7,43 (m, 1H, H arom.); 7,37 (m, 1H, H arom.); 6,93 (m, 2H, H arom.); 6,29 (s, 1H, H arom.) ; 3,72 (s, 6H, 2CH₃).
SM-CL : MH+ = 310,15 ; t.r. = 10,11 min.

### 137.2) 2-chloro-N-(3,5-diméthoxyphényl)-6-fluorobenzenecarbothioamide :

RMN ¹H (DMSO d6, 400 MHz, δ) : 7,41 (m, 2H, H arom.) ; 7,27 (m, 3H, H arom.) ; 6,46 (s, 1H, H arom.) ; 3,75 (s, 6H, 2CH₃).
SM-CL : MH+ = 326,09 ; t.r. = 10,73 min.

### 137.3) 2-(2-chloro-6-fluorophényl)-5,7-diméthoxy-1,3-benzothiazole :

RMN ¹H (DMSO d6, 400 MHz, δ) : 7,66 (m, 1H, H arom.) ; 7,56 (m, 1H, H arom.) ; 7,47 (m, 1H, H arom.) ; 7,30 (s, 1H, H arom.) ; 6,77 (s, 1H, H arom.) ; 3,96 (s, 3H, CH₃) ; 3,88 (s, 3H, CH₃).
SM-CL : MH+ = 324,03 ; t.r. = 11,60 min.

### 137.4) 2-(2-chloro-6-fluorophényl)-5-méthoxy-1,3-benzothiazole-4,7-dione :

RMN ¹H (DMSO d6, 400 MHz, δ) : 7,69 (m, 1H, H arom.) ; 7,61 (m, 1H, H arom.) ; 7,52 (m, 1H, H arom.) ; 6,32 (s, 1H, CH) ; 3,88 (s, 3H, CH₃).
SM-CL : MH+ = 324,03 ; t.r. = 9,23 min.

### 137.5) 2-(2-chloro-6-fluorophényl)-5-{[2-(diméthylamino)éthyl]amino}-1,3-benzothiazole-4,7-dione :

RMN ¹H (DMSO d6, 400 MHz, δ) : 7,67 (s, 1H, H arom.) ; 7,59 (m, 1H, H arom.) ; 7,55 (m, 1H, NH.) ; 7,49 (m, 1H, H arom.) ; 5,61 (s, 1H, CH) ; 3,36 (m, 2H, CH₂) ; 2,54 (m, 2H, CH₂) ; 2,19 (s, 6H, 2CH₃).
SM-CL : MH+ = 380,10 ; t.r. = 7,88 min.

### Exemple 138 : 2-(2-chloro-6-fluorophényl)-5-[(2-pyrrolidin-1-yléthyl)amino]-1,3-benzothiazole-4,7-dione :

SM-CL : MH+ = 406,10 ; t.r. = 8,01 min.

### Etude pharmacologigue pour les exemples 1 à 131 de composés de formule générale (I)

### Protocoles des tests

### i) Mesure de l'activité phosphatase de l'enzyme recombinante Cdc25C purifiée

L'activité phosphatase de la protéine MBP-Cdc25C est évaluée par la déphosphorylation du 3-O-méthylfluorescéine-phosphate (OMFP) en 3-O-méthylfluorescéine (OMF) avec une détermination de la fluorescence à 475 nm du produit de la réaction. Cet essai permet d'identifier des inhibiteurs de l'enzyme recombinante Cdc25. La préparation de la protéine de fusion MBP-Cdc25C est décrite dans la demande de brevet PCT WO 01/44467.

La réaction est réalisée en format de plaque 384 puits sous un volume final de 50 µl. La protéine MBP-Cdc25C (préparée comme décrit ci-dessus) est conservée dans le tampon d'élution suivant : 20 mM Tris-HCl pH 7,4 ; 250 mM NaCl ; 1mM EDTA ; 1 mM de dithiothréitol (DTT) ; 10 mM maltose. Elle est diluée à la concentration de 60 µM dans le tampon de réaction suivant : 50 mM Tris-HCl pH 8,2 ; 50 mM NaCl ; 1 mM DTT ; 20% glycérol. La mesure du bruit de fond est effectuée avec le tampon sans addition de l'enzyme. Les produits sont testés à des concentrations décroissantes à partir de 40 µM. La réaction est initiée par l'ajout d'une solution OMFP à 500 µM finale (préparée extemporanément à partir d'une solution stock 12,5 mM dans du DMSO 100% (Sigma #M2629)) . Après 4 heures à 30 °C dans une plaque 384 puits à usage unique, la fluorescence mesurée à DO 475 nm est lue à l'aide d'un lecteur de plaque Victor² (EGG-Wallac). La détermination de la concentration inhibant de 50% la réaction enzymatique est calculée à partir de trois expériences indépendantes. Seules les valeurs contenues dans la partie linéaire de la sigmoïde sont retenues pour l'analyse de régression linéaire.

### ii) Caractérisation de l'activité anti-proliférative :

A titre d'exemple, on étudiera l'effet d'un traitement sur deux lignées de cellules humaines Mia-Paca2 et DU145 par les composés des exemples décrits précédemment. Les lignées cellulaires DU145 (cellules humaines de cancer de la prostate) et Mia-PaCa2 (cellules humaines de cancer du pancréas) ont été acquises auprès de American Tissue Culture Collection (Rockville, Maryland, USA). Les cellules placées dans 80 µl de milieu Eagle modifié de Dulbecco (Gibco-Brl, Cergy-Pontoise, France) complété avec 10% de sérum foetal de veau inactivé par chauffage (Gibco-Brl, Cergy-Pontoise, France), 50000 unités/l de pénicilline et 50 mg/l streptomycine (Gibco-Brl, Cergy-Pontoise, France), et 2 mM de glutamine (Gibco-Brl, Cergy-Pontoise, France) ont été ensemencées sur une plaque de 96 puits au jour 0. Les cellules ont été traitées au jour 1 pendant 96 heures avec des concentrations croissantes de chacun des composés à tester jusqu'à 10 µM. A la fin de cette période, la quantification de la prolifération cellulaire est évaluée par test colorimétrique en se basant sur le clivage du sel de tétrazolium WST1 par les déhydrogénases mitochondriales dans les cellules viables conduisant à la formation de formazan (Boehringer Mannheim, Meylan, France). Ces tests sont effectués en double avec 8 déterminations par concentration testée. Pour chaque composé à tester, les valeurs incluses dans la partie linéaire de la sigmoïde ont été retenues pour une analyse en régression linéaire et utilisées pour estimer la concentration inhibitrice CI₅₀. Les produits sont solubilisés dans le diméthylsulfoxide (DMSO) à 10⁻² M et utilisés en culture avec 0,1% DMSO en final.

### Résultats des tests

a) Les composés des exemples 1 à 98, 101 à 104 et 107 à 115 présentent une CI₅₀ inférieure ou égale à 10 µM sur l'activité phosphatase de l'enzyme recombinante Cdc25-C purifiée.
b) Les composés des exemples 1 à 9, 11, 14 à 34, 36 à 53, 55 à 58, 60 à 98 et 101 à 115 présentent une CI₅₀ inférieure ou égale à 10 µM sur la prolifération cellulaire des lignées Mia-Paca2.
c) Les composés des exemples 1 à 9, 11, 14 à 34, 36 à 53, 55 à 58, 60 à 98 et 101 à 115 présentent une CI₅₀ inférieure ou égale à 10 µM sur la prolifération cellulaire des lignées DU-145.

### EXEMPLE DE COMPOSÉ DE FORMULE GÉNÉRALE (IV):

### Préparation du tétrachlorhydrate de (1R)-1-[({(2R)-2-amino-3-[(8S)-8-(cyclohexylméthyl)-2-phényl-5,6-dihydroimidazo[1,2-a]pyrazin-7(8H)-yl]-3-oxopropyl}dithio)méthyl]-2-[(8S)-8-(cyclohexylméthyl)-2-phényl-5,6-dihydroimidazo[1,2-a]pyrazin-7(8H)-yl]-2-oxoéthylamine :

### Étape 1: dimère de la [7-(2-(R)-t-bulyloxycarbonylamino-1-oxo-3-thiopropyl)-(S)-8-(cyclohexylméthyl)-2-phényl-5,6,7,8-tétrahydroimidazo[1,2a]pyrazine] :

La condensation de 2 équivalents de (8*S*)-8-(cyclohexylméthyl)-2-phényl-5,6,7,8-tétrahydroimidazo[1,2-*a*]pyrazine (25 g ; obtenue selon le protocole décrit dans la demande PCT WO 97/30053) avec 1 équivalent de Boc-L-Cystine (16,6 g) est effectué dans du diméthylformamide (160 ml) en présence de HBTU (43 g) et de diisopropyléthylamine (30 ml). Une fois la réaction complète, le milieu réactionnel est dilué avec de l'eau (160 ml) et le produit récupéré par filtration. Une purification par chromatographie sur silice (éluant : mélange CH₂Cl₂/AcOEt) permet d'isoler le produit avec un rendement de 60%.

### Étape 2: tétrachlorhydrate de (1R)-1-[({(2R)-2-amino-3-[(8S)-8-(cyclohexylméthyl)-2-phényl-5,6-dihydroimidazo[1,2-a]pyrazin-7(8H)-yl]-3-oxopropyl}dithio)méthyl]-2-[(8S)-8-(cyclohexylméthyl)-2-phényl-5,6-dihydroimidazo[1,2-a]pyrazin-7(8H)-yl]-2-oxoéthylamine:

Le produit de l'étape 1 (26 g) est dissous dans de l'isopropanol (260 ml). Après refroidissement à 0 °C, un excès d'une solution de HCl dans de l'isopropanol (5 à 6*N* ; 17 ml) est ajouté goutte à goutte pour surveiller la vitesse de libération du gaz formé par la déprotection du groups Boc. Après agitation une nuit à température ambiante, la fin de la réaction provoque une cristallisation *in situ* du tétrachlorhydrate. Le milieu réactionnel est ensuite refroidi à 0 °C pour rendre complète la cristallisation. La filtration, le lavage des cristaux avec de l'isopropanol et leur séchage sous vide permettent d'isoler le produit attendu avec un rendement de 75%.

### EXEMPLES D'ASSOCIATIONS SELON L'INVENTION

### A) TEST DE PROLIFÉRATION CELLULAIRE SUR DES CELLULES HT-29

Les associations présentées à titre d'exemples d'associations selon l'invention peuvent être testées quant à leur activité biologique et les résultats de l'association comparés aux résultats obtenus pour cahcun des composés de l'association utilisé séparément. Le protocole pour le test employé pour obtenir les résultats indiqués est décit ci-après :

### Lignée cellulaire

La lignée cellulaire HT-29 (cellules humaines de cancer du colon) a été acquise auprès de American Tissue Culture Collection (Rockville, Maryland, USA).

### Mesure de la prolifération cellulaire in vitro

Les cellules HT-29 ( 2000 cellules/puits) sont cultivées en plaques 96 puits.

Au jour 0, ces cellules sont ensemencées dans 90 µl de milieu de Eagle modifié par Dulbecco (Gibco-Brl, Cergy-Pontoise, France) complété avec 10% de sérum foetal de veau inactivé par chauffage (Gibco-Brl, Cergy-Pontoise, France ), 50000 unités/l de pénicilline et 50 mg/l streptomycine (Gibco-Brl, Cergy-Pontoise, France), et 2 mM de glutamine (Gibco-Brl, Cergy-Pontoise, France).

Les cellules ont été traitées simultanément avec des concentrations de deux produits seuls ou en association au Jour 1 et cela pendant 120 heures.

A la fin de cette période (J6), la quantification de la prolifération cellulaire est évaluée par test colorimétrique en se basant sur le clivage du sel de tétrazolium WST1 par les déhydrogénases mitochondriales dans les cellules vivantes conduisant à la formation de formazan (Boehringer Mannheim, Meylan, France). Ces tests sont effectués au moins en double avec 4 déterminations pour chaque produit seul et pour chaque association testée. Ceci permet de déterminer le nombre de cellules vivantes à la fin de chaque traitement.

### B) ASSOCIATIONS SELON L'INVENTION

### Association 1 :

Inhibiteur de phosphatase Cdc25 = chlorhydrate de 5-{[2-(diméthylamino)éthyl]amino}-2-méthyl-1,3-benzothiazole-4,7-dione **(A1)**
Agent anti-cancéreux associé = 5-fluorouracyle **(B1)**

Les résultats au test de prolifération cellulaire décrit ci-dessus (exprimés en % de cellules survivantes) pour les composés susmentionnés seuls ou en association sont reportés dans le tableau ci-après.

| Composé A1 (5.10⁻⁷ M) seul | Composé A1 (5.10⁻⁷ M) + composé B1 (2,5. 10⁻⁶ M) | Composé B1 (2,5. 10⁻⁶ M) seul |
|---|---|---|
| 35 | 13 | 42 |

### Association 2 :

Inhibiteur de phosphatase Cdc25 = chlorhydrate de 5-{[2-(diméthylamino)éthyl]amino}-2-méthyl-1,3-benzothiazole-4,7-dione **(A1)**
Agent anti-cancéreux associé = mitomycine C **(B2)**

Les résultats au test de prolifération cellulaire décrit ci-dessus (exprimés en % de cellules survivantes) pour les composés susmentionnés seuls ou en association sont reportés dans le tableau ci-après.

| Composé A1 (2,5.10⁻⁷ M) seul | Composé A1 (2,5.10⁻⁷ M) + composé B2 (10⁻⁷ M) | Composé B2 (10⁻⁷ M) seul |
|---|---|---|
| 91 | 36 | 49 |

### Association 3 :

Inhibiteur de phosphatase Cdc25 = chlorhydrate de 5-{[2-(diméthylamino)éthyl]amino}-2-méthyl-1,3-benzothiazole-4,7-dione **(A1)**
Agent anti-cancéreux associé = taxol **(B3)**

Les résultats au test de prolifération cellulaire décrit ci-dessus (exprimés en % de cellules survivantes) pour les composés susmentionnés seuls ou en association sont reportés dans le tableau ci-après.

| Composé A1 (5.10⁻⁷ M) seul | Composé A1 (5.10⁻⁷ M) + composé B3 (5.10⁻⁹ M) | Composé B3 (5.10⁻⁹ M) seul |
|---|---|---|
| 18 | 9 | 74 |

### Association 4 :

Inhibiteur de phosphatase Cdc25 = chlorhydrate de 5- {[2-(diméthylamino)éthyl]amino} -2-méthyl-1,3-benzothiazole-4,7-dione **(A1)**
Agent anti-cancéreux associé = cisplatine **(B4)**

Les résultats au test de prolifération cellulaire décrit ci-dessus (exprimés en % de cellules survivantes) pour les composés susmentionnés seuls ou en association sont reportés dans le tableau ci-après.

| Composé A1 (5.10⁻⁷ M) seul | Composé A1 (5.10⁻⁷ M) + composé B4 (10⁻⁵ M) | Composé B4 (10⁻⁵ M) seul |
|---|---|---|
| 31 | 11 | 33 |

### Association 5 :

Inhibiteur de phosphatase Cdc25 = chlorhydrate de 5-{[2-(diméthylamino)éthyl]amino}-2-méthyl-1,3-benzothiazole-4,7-dione **(A1)**
Agent anti-cancéreux associé = méthotrexate **(B5)**

Les résultats au test de prolifération cellulaire décrit ci-dessus (exprimés en % de cellules survivantes) pour les composés susmentionnés seuls ou en association sont reportés dans le tableau ci-après.

| Composé A1 (5.10⁻⁷ M) seul | Composé A1 (5.10⁻⁷ M) + composé B5 (5.10⁻⁸ M) | Composé B5 (5.10⁻⁸ M) seul |
|---|---|---|
| 27 | 17 | 83 |

### Association 6 :

Inhibiteur de phosphatase Cdc25 = chlorhydrate de 5-{[2-(diméthylamino)éthyl]amino}-2-méthyl-1,3-benzothiazole-4,7-dione **(A1)**
Agent anti-cancéreux associé = doxorubicine **(B6)**

Les résultats au test de prolifération cellulaire décrit ci-dessus (exprimés en % de cellules survivantes) pour les composés susmentionnés seuls ou en association sont reportés dans le tableau ci-après.

| Composé A1 (5.10⁻⁷ M) seul | Composé A1 (5.10⁻⁷ M) + composé B6 (5.10⁻⁷ M) | Composé B6 (5.10⁻⁷ M) seul |
|---|---|---|
| 25 | 9 | 38 |

### Association 7 :

Inhibiteur de phosphatase Cdc25 = chlorhydrate de 5-{[2-(diméthylamino)éthyl]amino}-2-méthyl-1,3-benzothiazole-4,7-dione **(A1)**
Agent anti-cancéreux associé = 4-(2-bromophényl)-1-(2-(1-((4-cyano-3-méthoxy)phénylméthyl)imidazo-5-yl)-1-oxoéthyl)-1,2-dihydro-8-fluoroimidazo[1,2a][1,4]-benzodiazépine **(B7)**

Les résultats au test de prolifération cellulaire décrit ci-dessus (exprimés en % de cellules survivantes) pour les composés susmentionnés seuls ou en association sont reportés dans le tableau ci-après.

| Composé A1 (5.10⁻⁷ M) seul | Composé A1 (5.10⁻⁷ M) + composé B7 (10⁻⁵ M) | Composé B7 (10⁻⁵ M) seul |
|---|---|---|
| 51 | 14 | 92 |

### Association 8 :

Inhibiteur de phosphatase Cdc25 = chlorhydrate de 5-{[2-(diméthylamino)éthyl]amino}-2-méthyl-1,3-benzothiazole-4,7-dione **(A1)**
Agent anti-cancéreux associé = 8-bromo-2-(1R-isopropyl-2-hydroxyéthylamino)-4-(3-fluorophénylméthylamino)-pyrazolo[1,5-a]-1,3,5-triazine **(B8)**

Les résultats au test de prolifération cellulaire décrit ci-dessus (exprimés en % de cellules survivantes) pour les composés susmentionnés seuls ou en association sont reportés dans le tableau ci-après.

| Composé A1 (5.10⁻⁷ M) seul | Composé A1 (5.10⁻⁷ M) + composé B8 (10⁻⁶ M) | Composé B8 (10⁻⁶ M) seul |
|---|---|---|
| 47 | 6 | 69 |

### Association 9 :

Inhibiteur de phosphatase Cdc25 = chlorhydrate de 5-{[2-(diméthylamino)éthyl]amino}-2-méthyl-1,3-benzothiazole-4,7-dione **(A1)**
Agent anti-cancéreux associé = tétrachlorhydrate de (1*R*)-1-[({(2*R*)-2-amino-3-[(8*S*)-8-(cyclohexylméthyl)- 2-phényl-5,6-dihydroimidazo[1,2-*a*]pyrazin-7(8*H*)-yl]-3-oxopropyl}dithio)méthyl]-2-[(8*S*)-8-(cyclohexylméthyl)-2-phényl-5,6-dihydroimidazo[1,2-*a*]pyrazin-7(8*H*)-yl]-2-oxoéthylamine **(B9)**

Les résultats au test de prolifération cellulaire décrit ci-dessus (exprimés en % de cellules survivantes) pour les composés susmentionnés seuls ou en association sont reportés dans le tableau ci-après.

| Composé A1 (5.10⁻⁷ M) seul | Composé A1 (5.10⁻⁷ M) + composé B9 (10⁻⁵ M) | Composé B9 (10⁻⁵ M) seul |
|---|---|---|
| 36 | 9 | 38 |

### Association 10 :

Inhibiteur de phosphatase Cdc25 = chlorhydrate de 5-{[2-(diméthylamino)éthyl]amino}-2-méthyl-1,3-benzothiazole-4,7-dione **(A1)**
Agent anti-cancéreux associé = amsacrine **(B10)**

Les résultats au test de prolifération cellulaire décrit ci-dessus (exprimés en % de cellules survivantes) pour les composés susmentionnés seuls ou en association sont reportés dans le tableau ci-après.

| Composé A1 (5.10⁻⁷ M) seul | Composé A1 (5.10⁻⁷ M) + composé B10 (10⁻⁷ M) | Composé B10 (10⁻⁷ M) seul |
|---|---|---|
| 42 | 39 | 89 |

### Association 11 :

Inhibiteur de phosphatase Cdc25 = chlorhydrate de 5-{[2-(diméthylamino)éthyl]amino}-2-méthyl-1,3-benzothiazole-4,7-dione **(A1)**
Agent anti-cancéreux associé = SN-38 **(B11)**

Les résultats au test de prolifération cellulaire décrit ci-dessus (exprimés en % de cellules survivantes) pour les composés susmentionnés seuls ou en association sont reportés dans le tableau ci-après.

| Composé A1 (5.10⁻⁷ M) seul | Composé A1 (5.10⁻⁷ M) + composé B11 (5.10⁻⁹ M) | Composé B11 (5.10⁻⁹ M) seul |
|---|---|---|
| 44 | 7 | 98 |

### Association 12 :

Inhibiteur de phosphatase Cdc25 = chlorhydrate de 5-{[2-(diméthylamino)éthyl]amino}-2-méthyl-1,3-benzothiazole-4,7-dione **(A1)**
Agent anti-cancéreux associé = diflomotécan **(B12)**

Les résultats au test de prolifération cellulaire décrit ci-dessus (exprimés en % de cellules survivantes) pour les composés susmentionnés seuls ou en association sont reportés dans le tableau ci-après.

| Composé A1 (5.10⁻⁷ M) seul | Composé A1 (5.10⁻⁷ M) + composé B 12 (10⁻⁹ M) | Composé B12 (10⁻⁹ M) seul |
|---|---|---|
| 33 | 22 | 83 |

### Association 13 :

Inhibiteur de phosphatase Cdc25 = chlorhydrate de 5-{[2-(diméthylamino)éthyl]amino}-2-méthyl-1,3-benzothiazole-4,7-dione **(A1)**
Agent anti-cancéreux associé = BN-80927 ou chlorhydrate de (+)-9-chloro-5-éthyl-5-hydroxy-10-méthyl-12-(4-méthylpipéridinométhyl)-4,5,13,15-tétrahydro-1H,3H-oxépino[3',4':6,7]indolizino[1,2-c]quinoline-3,15-dione **(B13)**

Les résultats au test de prolifération cellulaire décrit ci-dessus (exprimés en % de cellules survivantes) pour les composés susmentionnés seuls ou en association sont reportés dans le tableau ci-après.

| Composé A1 (5.10⁻⁷ M) seul | Composé A1 (5.10⁻⁷ M) + composé B 13 (10⁻⁹ M) | Composé B13 (10⁻⁹ M) seul |
|---|---|---|
| 43 | 32 | 91 |

### Association 14 :

Inhibiteur de phosphatase Cdc25 = chlorhydrate de 5-{[2-(diméthylamino)éthyl]amino}-2-méthyl-1,3-benzothiazole-4,7-dione **(A1)**
Agent anti-cancéreux associé = roscovitine **(B14)**

Les résultats au test de prolifération cellulaire décrit ci-dessus (exprimés en % de cellules survivantes) pour les composés susmentionnés seuls ou en association sont reportés dans le tableau ci-après.

| Composé A1 (10⁻⁶ M) seul | Composé A1 (10⁻⁶ M) + composé B 14 (5.10⁻⁵ M) | Composé B14 (5.10⁻⁵ M) seul |
|---|---|---|
| 36 | 0 | 44 |

### Association 15 :

Inhibiteur de phosphatase Cdc25 = ménadione **(A2)**
Agent anti-cancéreux associé = roscovitine **(B14)**

Les résultats au test de prolifération cellulaire décrit ci-dessus (exprimés en % de cellules survivantes) pour les composés susmentionnés seuls ou en association sont reportés dans le tableau ci-après.

| Composé A2 (4.10⁻⁵ M) seul | Composé A2 (4.10⁻⁵ M) + composé B 14 (5.10⁻⁵ M) | Composé B 14 (5.10⁻⁵ M) seul |
|---|---|---|
| 7 | 0 | 47 |

### Association 16 :

Inhibiteur de phosphatase Cdc25 = ménadione (A2)
Agent anti-cancéreux associé = 8-bromo-2-(1R-isopropyl-2-hydroxyéthylamino)-4-(3-fluorophénylméthylamino)-pyrazolo[1,5-a]-1,3,5-triazine **(B8)**

Les résultats au test de prolifération cellulaire décrit ci-dessus (exprimés en % de cellules survivantes) pour les composés susmentionnés seuls ou en association sont reportés dans le tableau ci-après.

| Composé A2 (4.10⁻⁵ M) seul | Composé A2 (4.10⁻⁵ M) + composé B8 (10⁻⁶ M) | Composé B8 (10⁻⁶ M) seul |
|---|---|---|
| 33 | 4 | 17 |

### Les associations 17 à 19 donnent des résultats similaires à ceux observés pour les associations 11 à 13.

### Association 17 :

Inhibiteur de phosphatase Cdc25 = benzoate de 2-méthyl-5-[(2-pyrrolidin-1-yléthyl)amino]-1,3-benzothiazole-4,7-dione **(A3)**
Agent anti-cancéreux associé = diflomotécan **(B12)**

### Association 18 :

Inhibiteur de phosphatase Cdc25 = benzoate de 2-méthyl-5-[(2-pipéridin-1-yléthyl)amino]-1,3-benzothiazole-4,7-dione **(A4)**
Agent anti-cancéreux associé = BN-80927 ou chlorhydrate de (+)-9-chloro-5-éthyl-5-hydroxy-10-méthyl-12-(4-méthylpipéridinométhyl)-4,5,13,15-tétrahydro-1H,3H-oxépino[3',4':6,7]indolizino[1,2-c]quinoline-3,15-dione **(B13)**

### Association 19 :

Inhibiteur de phosphatase Cdc25 = 2-(2-chloro-6-fluorophényl)-5-{[2-(diméthylamino)éthyl]amino}-1,3-benzothiazole-4,7-dione **(A5)**
Agent anti-cancéreux associé = diflomotécan **(B12)**

### Les associations 20 à 22 donnent des résultats similaires à ceux observés pour l'association 8.

### Association 20 :

Inhibiteur de phosphatase Cdc25 = benzoate de 2-méthyl-5-[(2-pyrrolidin-1-yléthyl)amino]-1,3-benzothiazole-4,7-dione **(A3)**
Agent anti-cancéreux associé = 8-bromo-4-[(3-pyridyl)méthylamino]-2-méthylthiopyrazolo[1,5-a]-1,3,5-triazine **(B15)**

### Association 21 :

Inhibiteur de phosphatase Cdc25 = benzoate de 2-méthyl-5-[(2-pipéridin-1-yléthyl)amino]-1,3-benzothiazole-4,7-dione **(A4)**
Agent anti-cancéreux associé = 8-bromo-2-(1R-isopropyl-2-hydroxyéthylamino)-4-(3-fluorophénylméthylamino)-pyrazolo[1,5-a]-1,3,5-triazine **(B8)**

### Association 22 :

Inhibiteur de phosphatase Cdc25 = 2-(2-chloro-6-fluorophényl)-5-{[2-(diméthylamino)éthyl]amino}-1,3-benzothiazole-4,7-dione **(A5)**
Agent anti-cancéreux associé = 8-bromo-2-(1*R*-isopropyl-2-hydroxyéthylamino)-4-(3-pyridylméthylamino)pyrazolo[1,5-a]-1,3,5-triazine **(B16)**

### Association 23:

Inhibiteur de phosphatase Cdc25 = benzoate de 2-méthyl-5-[(2-pyrrolidin-1-yléthyl)amino]-1,3-benzothiazole-4,7-dione **(A3)**
Agent anti-cancéreux associé = taxol **(B3)**

Les résultats au test de prolifération cellulaire décrit ci-dessus (exprimés en % de cellules survivantes) pour les composés susmentionnés seuls ou en association sont reportés dans le tableau ci-après.

| Composé A3 (1,25.10⁻⁷ M) seul | Composé A3 (1,25.10⁻⁷ M) + composé B3 (5.10⁻⁹ M) | Composé B3 (5.10⁻⁹ M) seul |
|---|---|---|
| 91 | 32 | 54 |

### Association 24:

Inhibiteur de phosphatase Cdc25 = benzoate de 2-méthyl-5-[(2-pyrrolidin-1-yléthyl)amino]-1,3-benzothiazole-4,7-dione **(A3)**

Agent anti-cancéreux associé = SN-38 **(B11)**

Les résultats au test de prolifération cellulaire décrit ci-dessus (exprimés en % de cellules survivantes) pour les composés susmentionnés seuls ou en association sont reportés dans le tableau ci-après.

| Composé A3 (2,5.10⁻⁷ M) seul | Composé A3 (2,5.10⁻⁷ M) + composé B 11 (10⁻⁸ M) | Composé B11 (10⁻⁸ M) seul |
|---|---|---|
| 75 | 40 | 73 |

### Association 25:

Inhibiteur de phosphatase Cdc25 = benzoate de 2-méthyl-5-[(2-pyrrolidin-1-yléthyl)amino]-1,3-benzothiazole-4,7-dione **(A3)**
Agent anti-cancéreux associé = mitomycine C **(B2)**

Les résultats au test de prolifération cellulaire décrit ci-dessus (exprimés en % de cellules survivantes) pour les composés susmentionnés seuls ou en association sont reportés dans le tableau ci-après.

| Composé A3 (2,5.10⁻⁷ M) seul | Composé A3 (2,5.10⁻⁷ M) + composé B2 (5.10⁻⁸ M) | Composé B2 (5.10⁻⁸ M) seul |
|---|---|---|
| 84 | 55 | 82 |

### Association 26:

Inhibiteur de phosphatase Cdc25 = benzoate de 2-méthyl-5-[(2-pyrrolidin-1-yléthyl)amino]-1,3-benzothiazole-4,7-dione **(A3)**
Agent anti-cancéreux associé = doxorubicine **(B6)**

Les résultats au test de prolifération cellulaire décrit ci-dessus (exprimés en % de cellules survivantes) pour les composés susmentionnés seuls ou en association sont reportés dans le tableau ci-après.

| Composé A3 (2,5.10⁻⁷ M) seul | Composé A3 (2,5.10⁻⁷ M) + composé B6 (10⁻⁷ M) | Composé B6 (10⁻⁷ M) seul |
|---|---|---|
| 73 | 58 | 75 |

### Association 27:

Inhibiteur de phosphatase Cdc25 = benzoate de 2-méthyl-5-[(2-pyrrolidin-1-yléthyl)amino]-1,3-benzothiazole-4,7-dione **(A3)**
Agent anti-cancéreux associé = 8-bromo-2-(1R-isopropyl-2-hydroxyethylamino)-4-(3-fluorophenylmethylamino)-pyrazolo[1,5-a]-1,3,5-triazine **(B8)**

Les résultats au test de prolifération cellulaire décrit ci-dessus (exprimés en % de cellules survivantes) pour les composés susmentionnés seuls ou en association sont reportés dans le tableau ci-après.

| Composé A3 (1,25.10⁻⁷ M) seul | Composé A3 (1,25.10⁻⁷ M) + composé B8 (10⁻⁶ M) | Composé B8 (10⁻⁶ M) seul |
|---|---|---|
| 89 | 12 | 21 |

### Association 28:

Inhibiteur de phosphatase Cdc25 = benzoate de 2-méthyl-5-[(2-pyrrolidin-1-yléthyl)amino]-1,3-benzothiazole-4,7-dione **(A3)**
Agent anti-cancéreux associé = cisplatine **(B4)**

Les résultats au test de prolifération cellulaire décrit ci-dessus (exprimés en % de cellules survivantes) pour les composés susmentionnés seuls ou en association sont reportés dans le tableau ci-après.

| Composé A3 (2,5.10⁻⁷ M) seul | Composé A3 (2,5.10⁻⁷ M) + composé B4 (10⁻⁵ M) | Composé B4 (10⁻⁵ M) seul |
|---|---|---|
| 78 | 36 | 75 |

### Association 29:

Inhibiteur de phosphatase Cdc25 = benzoate de 2-méthyl-5-[(2-pipéridin-1-yléthyl)amino]-1,3-benzothiazole-4,7-dione **(A4)**
Agent anti-cancéreux associé = taxol **(B3)**

Les résultats au test de prolifération cellulaire décrit ci-dessus (exprimés en % de cellules survivantes) pour les composés susmentionnés seuls ou en association sont reportés dans le tableau ci-après.

| Composé A4 (2,5.10⁻⁷ M) seul | Composé A4 (2,5.10⁻⁷ M) + composé B3 (5.10⁻⁹ M) | Composé B3 (5.10⁻⁹ M) seul |
|---|---|---|
| 94 | 33 | 51 |

### Association 30:

Inhibiteur de phosphatase Cdc25 = benzoate de 2-méthyl-5-[(2-pipéridin-1-yléthyl)amino]-1,3-benzothiazole-4,7-dione **(A4)**
Agent anti-cancéreux associé = SN-38 **(B11)**

Les résultats au test de prolifération cellulaire décrit ci-dessus (exprimés en % de cellules survivantes) pour les composés susmentionnés seuls ou en association sont reportés dans le tableau ci-après.

| Composé A4 (5.10⁻⁷ M) seul | Composé A4 (5.10⁻⁷ M) + composé B11 (10⁻⁸ M) | Composé B11 (10⁻⁸ M) seul |
|---|---|---|
| 78 | 42 | 67 |

### Association 31:

Inhibiteur de phosphatase Cdc25 = benzoate de 2-méthyl-5-[(2-pipéridin-1-yléthyl)amino]-1,3-benzothiazole-4,7-dione **(A4)**
Agent anti-cancéreux associé = diflomotécan **(B12)**

Les résultats au test de prolifération cellulaire décrit ci-dessus (exprimés en % de cellules survivantes) pour les composés susmentionnés seuls ou en association sont reportés dans le tableau ci-après.

| Composé A4 (5.10⁻⁷ M) seul | Composé A4 (5.10⁻⁷ M) + composé B 12 (10⁻⁹ M) | Composé B12 (10⁻⁹ M) seul |
|---|---|---|
| 79 | 58 | 89 |

### Association 32:

Inhibiteur de phosphatase Cdc25 = benzoate de 2-méthyl-5-[(2-pipéridin-1-yléthyl)amino]-1,3-benzothiazole-4,7-dione **(A4)**
Agent anti-cancéreux associé = mitomycine C **(B2)**

Les résultats au test de prolifération cellulaire décrit ci-dessus (exprimés en % de cellules survivantes) pour les composés susmentionnés seuls ou en association sont reportés dans le tableau ci-après.

| Composé A4 (5.10⁻⁷ M) seul | Composé A4 (5.10⁻⁷ M) + composé B2 (5.10⁻⁸ M) | Composé B2 (5.10⁻⁸ M) seul |
|---|---|---|
| 74 | 83 | 88 |

### Association 33:

Inhibiteur de phosphatase Cdc25 = benzoate de 2-méthyl-5-[(2-pipéridin-1-yléthyl)amino]-1,3-benzothiazole-4,7-dione **(A4)**
Agent anti-cancéreux associé = mitomycin C **(B2)**

Les résultats au test de prolifération cellulaire décrit ci-dessus (exprimés en % de cellules survivantes) pour les composés susmentionnés seuls ou en association sont reportés dans le tableau ci-après.

| Composé A4 (5.10⁻⁷ M) seul | Composé A4 (5.10⁻⁷ M) + composé B2 (5.10⁻⁸ M) | Composé B2 (5.10⁻⁸ M) seul |
|---|---|---|
| 74 | 83 | 88 |

### Association 34:

Inhibiteur de phosphatase Cdc25 = benzoate de 2-méthyl-5-[(2-pipéridin-1-yléthyl)amino]-1,3-benzothiazole-4,7-dione **(A4)**
Agent anti-cancéreux associé = 5-fluorouracile **(B1)**

Les résultats au test de prolifération cellulaire décrit ci-dessus (exprimés en % de cellules survivantes) pour les composés susmentionnés seuls ou en association sont reportés dans le tableau ci-après.

| Composé A4 (5.10⁻⁷ M) seul | Composé A4 (5.10⁻⁷ M) + composé B1 (2,5.10⁻⁶ M) | Composé B1 (2,5.10⁻⁶ M) seul |
|---|---|---|
| 75 | 34 | 51 |

### Association 35:

Inhibiteur de phosphatase Cdc25 = benzoate de 2-méthyl-5-[(2-pipéridin-1-yléthyl)amino]-1,3-benzothiazole-4,7-dione **(A4)**
Agent anti-cancéreux associé = cisplatine **(B4)**

Les résultats au test de prolifération cellulaire décrit ci-dessus (exprimés en % de cellules survivantes) pour les composés susmentionnés seuls ou en association sont reportés dans le tableau ci-après.

| Composé A4 (5.10⁻⁷ M) seul | Composé A4 (5.10⁻⁷ M) + composé B4 (10⁻⁵ M) | Composé B4 (10⁻⁵ M) seul |
|---|---|---|
| 71 | 43 | 85 |

### Association 36:

Inhibiteur de phosphatase Cdc25 = benzoate de 2-méthyl-5-[(2-pipéridin-1-yléthyl)amino]-1,3-benzothiazole-4,7-dione **(A4)**
Agent anti-cancéreux associé = tétrachlorhydrate de (1*R*)-1-[({(2*R*)-2-amino-3-[(8*S*)-8-(cyclohexylméthyl)-2-phényl-5,6-dihydroimidazo[1,2-*a*]pyrazin-7(8*H*)-yl]-3-oxopropyl}dithio)méthyl]-2-[(8*S*)-8-(cyclohexylméthyl)-2-phényl-5,6-dihydroimidazo[1,2-*a*]pyrazin-7(8*H*)-yl]-2-oxoéthylamine **(B9)**

Les résultats au test de prolifération cellulaire décrit ci-dessus (exprimés en % de cellules survivantes) pour les composés susmentionnés seuls ou en association sont reportés dans le tableau ci-après.

| Composé A4 (5.10⁻⁷ M) seul | Composé A4 (5.10⁻⁷ M) + composé B9 (5.10⁻⁶ M) | Composé B9 (5.10⁻⁶ M) seul |
|---|---|---|
| 78 | 51 | 80 |

## Revendications

1. Produit comprenant au moins un inhibiteur de phosphatase Cdc25 en association avec au moins un autre agent anti-cancéreux pour une utilisation thérapeutique simultanée, séparée ou étalée dans le temps dans le traitement du cancer **caractérisé en ce que** l'agent anti-cancéreux associé à l'inhibiteur de phosphatase Cdc25 est un inhibiteur des kinases dépendantes des cyclines (CDKs).

2. Produit selon la revendication 1, **caractérisé en ce que** l'inhibiteur de phosphatase Cdc25 associé à l'autre agent anti-cancéreux est un composé de formule générale **(I)** 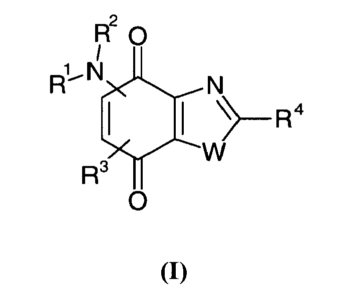 dans laquelle :
R¹ représente un atome d'hydrogène ou un radical alkyle, alkoxyalkyle, alkylthioalkyle, cycloalkyle, -(CH₂)-X-Y, -(CH₂)-Z-NR⁵R⁶ ou un radical -CHR³⁵R³⁶ dans lequel R³⁵ et
R³⁶ forment ensemble avec l'atome de carbone qui les porte un radical indanyle ou tétralinyle, ou encore R³⁵ et R³⁶ forment ensemble avec l'atome de carbone qui les porte un hétérocycle saturé comptant de 5 à 7 chaînons et de 1 à 2 hétéroatomes choisis parmi O, N et S, les atomes d'azote dudit hétérocycle étant éventuellement substitués par des radicaux choisis parmi les radicaux alkyle et le radical benzyle,
R¹ pouvant aussi, lorsque W représente O, représenter en outre un radical aryle carbocyclique éventuellement substitué de 1 à 3 fois par des substituants choisis indépendamment parmi un atome halogène et un radical alkyle, haloalkyle ou alkoxy,
X représentant une liaison ou un radical alkylène linéaire ou ramifié comptant de 1 à 5 atomes de carbone,
Y représentant un système cyclique carboné saturé comptant de 1 à 3 cycles condensés choisis indépendamment parmi des cycles de 3 à 7 chaînons, ou bien Y représentant un hétérocycle saturé comptant de 1 à 2 hétéroatomes choisis indépendamment parmi O, N et S et attaché au radical X par un chaînon N ou CH, ledit hétérocycle saturé comptant par ailleurs de 2 à 6 chaînons supplémentaires choisis indépendamment parmi -CHR⁷-, -CO-, -NR⁸-, -O- et -S-, R⁷ représentant un atome d'hydrogène ou un radical alkyle et R⁸ représentant un atome d'hydrogène ou un radical alkyle ou aralkyle, ou encore Y représentant un radical aryle carbocyclique ou hétérocyclique éventuellement substitué de 1 à 3 fois par des substituants choisis indépendamment parmi le groupe constitué par un atome halogène, un radical alkyle, un radical haloalkyle, un radical alkoxy, un radical haloalkoxy, un radical hydroxy, un radical nitro, un radical cyano, le radical phényle, un radical SO₂NHR⁹ et un radical NR¹⁰R¹¹, R⁹ représentant un atome d'hydrogène ou un radical alkyle ou phényle, et R¹⁰ et R¹¹ représentant indépendamment des radicaux alkyle,
Z représentant une liaison ou un radical alkylène linéaire ou ramifié comptant de 1 à 5 atomes de carbone,
R⁵ et R⁶ étant choisis indépendamment parmi un atome d'hydrogène, un radical alkyle, aralkyle ou -(CH₂)ₙ-OH dans lequel n représente un entier de 1 à 6, ou R⁵ représentant un radical alkoxycarbonyle, haloalkoxycarbonyle ou
aralkoxycarbonyle et R⁶ représentant un atome d'hydrogène ou un radical méthyle,
ou encore R⁵ et R⁶ formant ensemble avec l'atome d'azote un hétérocycle de 4 à 7 chaînons comportant de 1 à 2 hétéroatomes, les chaînons nécessaires pour compléter l'hétérocycle étant choisis indépendamment parmi les radicaux -CR¹²R¹³, -O-, -S- et -NR¹⁴- R¹² et R¹³ représentant indépendamment à chaque fois qu'ils interviennent un atome d'hydrogène ou un radical alkyle, et R¹⁴ représentant un atome d'hydrogène ou un radical alkyle ou aralkyle, ou encore R¹⁴ représentant un radical phényle éventuellement substitué de 1 à 3 fois par des substituants choisis indépendamment parmi un atome halogène et un radical alkyle ou alkoxy,
R² représentant un atome d'hydrogène ou un radical alkyle ou aralkyle ;
ou encore R¹ et R² formant ensemble avec l'atome d'azote un hétérocycle de 4 à 8 chaînons comportant de 1 à 2 hétéroatomes, les chaînons nécessaires pour compléter l'hétérocycle étant choisis indépendamment parmi les radicaux -CR¹⁵R¹⁶-, -O-, -S- et -NR¹⁷-, R¹⁵ et R¹⁶ représentant indépendamment à chaque fois qu'ils interviennent un atome d'hydrogène ou un radical alkyle, et R¹⁷ représentant un atome d'hydrogène ou un radical alkyle ou aralkyle ;
R³ représente un atome d'hydrogène, un atome halogène, ou un radical alkyle, haloalkyle, alkoxy ou alkylthio ;
R⁴ représente un radical alkyle, cycloalkyle, cycloalkylalkyle, cyano, amino, -CH₂-COOR¹⁸, -CH2-CO-NR¹⁹R²⁰ ou -CH₂-NR²¹R²², ou bien R⁴ représente un radical aryle carbocyclique ou hétérocyclique éventuellement substitué de 1 à 4 fois par des substituants choisis indépendamment parmi un atome halogène et un radical alkyle, haloalkyle, alkoxy, haloalkoxy ou NR³⁷R³⁸ ou encore R⁴ représente un radical phényle possédant deux substituants qui forment ensemble un radical méthylènedioxy ou éthylènedioxy,
R¹⁸ représentant un atome d'hydrogène ou un radical alkyle,
R¹⁹ représentant un atome d'hydrogène, un radical alkyle ou un radical aralkyle dont le groupe aryle est éventuellement substitué de 1 à 3 fois par des substituants choisis indépendamment parmi le groupe constitué par un atome halogène, un radical alkyle, un radical haloalkyle, un radical alkoxy, un radical haloalkoxy, un radical hydroxy, un radical nitro, un radical cyano, le radical phényle, un radical SO₂NHR²³ et un radical NR²⁴R²¹, R²³ représentant un atome d'hydrogène ou un radical alkyle ou phényle, et R²⁴ et R²⁵ représentant indépendamment des radicaux alkyle,
R²⁰ représentant un atome d'hydrogène ou un radical alkyle,
ou encore R¹⁹ et R²⁰ formant ensemble avec l'atome d'azote un hétérocycle de 4 à 7 chaînons comportant de 1 à 2 hétéroatomes, les chaînons nécessaires pour compléter l'hétérocycle étant choisis indépendamment parmi les radicaux -CR²⁶R²⁷-, -O-, -S- et -NR²⁸-, R²⁶ et R²⁷ représentant indépendamment à chaque fois qu'ils interviennent un atome d'hydrogène ou un radical alkyle, et R²⁸ représentant un atome d'hydrogène ou un radical alkyle ou aralkyle, ou encore R²⁸ représentant un radical phényle éventuellement substitué de 1 à 3 fois par des substituants choisis indépendamment parmi un atome halogène et un radical alkyle ou alkoxy,
R²¹ représentant un atome d'hydrogène, un radical alkyle ou un radical aralkyle dont le groupe aryle est éventuellement substitué de 1 à 3 fois par des substituants choisis indépendamment parmi le groupe constitué par un atome halogène, un radical alkyle, un radical haloalkyle, un radical alkoxy, un radical haloalkoxy, un radical hydroxy, un radical nitro, un radical cyano, le radical phényle, un radical SO₂HR²⁹ et un radical NR³⁰R³¹, R²⁹ représentant un atome d'hydrogène ou un radical alkyle ou phényle, et R³⁰ et R³¹ représentant indépendamment des radicaux alkyle,
R²² représentant un atome d'hydrogène ou un radical alkyle,
ou encore R²¹ et R²² formant ensemble avec l'atome d'azote un hétérocycle de 4 à 7 chaînons comportant de 1 à 2 hétéroatomes, les chaînons nécessaires pour compléter l'hétérocycle étant choisis indépendamment parmi les radicaux -CR³²R³³-, -O-, -S- et -NR³⁴-, R³² et R³³ représentant indépendamment à chaque fois qu'ils interviennent un atome d'hydrogène ou un radical alkyle, et R³⁴ représentant un atome d'hydrogène, un radical alkyle ou aralkyle, ou encore R³⁴ représentant un radical phényle éventuellement substitué de 1 à 3 fois par des substituants choisis indépendamment parmi un atome halogène et un radical alkyle ou alkoxy,
R³⁷ et R³⁸ étant choisis indépendamment parmi un atome d'hydrogène et un radical alkyle ou R³⁷ et R³⁸ formant ensemble avec l'atome d'azote un hétérocycle de 4 à 7 chaînons comportant de 1 à 2 hétéroatomes, les chaînons nécessaires pour compléter l'hétérocycle étant choisis indépendamment parmi les radicaux -CR³⁹R⁴⁰-, -O-, -S- et -NR⁴¹-, R³⁹ et R⁴⁰ représentant indépendamment à chaque fois qu'ils interviennent un atome d'hydrogène ou un radical alkyle, et R⁴¹ représentant un atome d'hydrogène ou un radical alkyle ; et
W représente O ou S ;
ou un sel pharmaceutiquement acceptable d'un composé de formule générale **(I).**

3. Produit selon la revendication 2, **caractérisé en ce que** le composé de formule générale **(I)** ou son sel pharmaceutiquement acceptable est choisi parmi les composés suivants :
- 5-{[2-(diméthylammo)éthyl]amino}-2-méthyl-1,3-benzothiazole-4,7-dione;
- 2-méthyl-5-[(2-pyrrolidin-1-yléthyl)amino]-1,3-benzothiazole-4,7-dione ;
- 2-méthyl-5-[(2-pipéridin-1-yléthyl)amino]-1,3-benzothiazole-4,7-dione ;
- 2-(2-chloro-6-fluorophényl)-5-{[2-(diméthylamino)éthyl]amino}-1,3-benzothiazole-4,7-dione ;
et les sels pharmaceutiquement acceptables de ces derniers.

4. Produit selon la revendication 1, **caractérisé en ce que** l'inhibiteur de phosphatase Cdc25 associé à l'autre agent anti-cancéreux est choisi parmi la ménadione et ses analogues.

5. Produit selon l'une des revendications 1 à 4, **caractérisé en ce que** l'inhibiteur des CDKs est choisi parmi les composés de formule générale **(VII)** 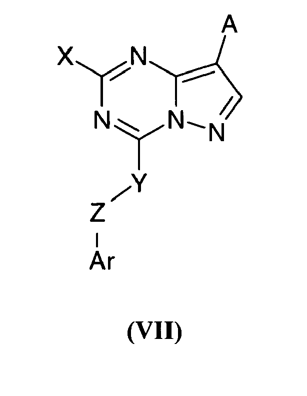 sous forme racémique, d'énantiomère ou toute combinaison de ces formes, dans laquelle
A représente un atome d'hydrogène, un atome halogène, un radical formyle, cyano, nitro, guanidinoaminométhylènyle, (1,3-dihydro-2-oxoindol)-3-ylidèneméthyle, alkylcarbonyle, aralkylcarbonyle ou hétéroaralkylcarbonyle, ou encore un radical -L-NR¹R² dans lequel L représente un radical alkylène et R¹ et R² sont choisis indépendamment parmi un atome d'hydrogène et un radical alkyle ou R¹ et R² pris ensemble avec l'atome d'azote qui les porte forment un hétérocycle de 5 à 7 chaînons, les chaînons complémentaires étant choisis indépendamment parmi le groupe composé de -CH₂-, -NR³-, -S- et -O-, R³ représentant indépendamment à chaque fois qu'il intervient un atome d'hydrogène ou un radical alkyle ;
X représente un atome d'hydrogène, un radical alkylthio, aralkylthio, alkylthioxo ou aralkylthioxo, ou encore un radical NR⁴R⁵ dans lequel R⁴ représente un radical alkyle, un radical hydroxyalkyle, un radical cycloalkyle éventuellement substitué par un ou des radicaux choisis parmi les radicaux alkyle, hydroxy et amino, un radical aralkyle dont le radical aryle est éventuellement substitué par un ou des radicaux choisis parmi un atome halogène, le radical cyano, le radical nitro et les radicaux alkyle ou alkoxy, ou encore R⁴ représente un radical hétéroaryle ou hétéroarylalkyle, le radical hétéroaryle des radicaux hétéroaryle ou hétéroarylalkyle étant éventuellement substitué par un ou des radicaux alkyle et R⁵ représente un atome d'hydrogène, ou alors R⁴ et R⁵ pris ensemble avec l'atome d'azote qui les porte forment un hétérocycle de 5 à 7 chaînons, les chaînons complémentaires étant choisis indépendamment parmi le groupe composé de -CH₂-, -NR⁶-, -S- et -O-, R⁶ représentant indépendamment à chaque fois qu'il intervient un atome d'hydrogène ou un radical alkyle ou \hydroxyalkyle ;
Y représente NH ou un atome d'oxygène ;
Z représente une liaison ou un radical alkyle ou alkylthioalkyle ; et
Ar représente un radical aryle carbocyclique éventuellement substitué de 1 à 3 fois par des radicaux choisis indépendamment parmi un atome halogène, le radical cyano, le radical nitro, un radical alkyle ou alkoxy et un radical NR⁷R⁸ dans lequel R⁷ et R⁸ représentent indépendamment un atome d'hydrogène ou un radical alkyle ou R⁷ et R⁸ pris ensemble avec l'atome d'azote qui les porte forment un hétérocycle de 5 à 7 chaînons, les chaînons complémentaires étant choisis indépendamment parmi le groupe composé de -CH₂-, -NR⁹-, -S- et -O-, R⁹ représentant indépendamment à chaque fois qu'il intervient un atome d'hydrogène ou un radical alkyle,
ou encore Ar représente un radical aryle hétérocyclique comptant 5 ou 6 chaînons et dont le ou les hétéroatomes sont choisis parmi des atomes d'azote, d'oxygène ou de soufre, lesdits hétéroatomes pouvant éventuellement être oxydés (Ar peut représenter par exemple le radical oxidopyridyle) et ledit radical aryle hétérocyclique pouvant être éventuellement substitué par un ou des radicaux choisis indépendamment parmi les radicaux alkyle, aminoalkyle, alkylaminoalkyle et dialkylaminoalkyle ;
et les sels pharmaceutiquement acceptables de ces composés.

6. Produit selon l'une des revendications 1 à 4, **caractérisé en ce que** l'inhibiteur des CDKs est choisi parmi la roscovitine et ses analogues.
